(19) Europäisches Patentamt
European Patent Office
Office européen des brevets

(11) Publication number: **0 098 252 B1**

(12) # EUROPEAN PATENT SPECIFICATION

(45) Date of publication of patent specification: **07.06.89**

(51) Int. Cl.$^4$: **C 07 H 15/04**, C 07 H 3/06, C 07 H 3/04, C 07 K 9/00, C 07 G 17/00

(21) Application number: **83850176.5**

(22) Date of filing: **21.06.83**

(54) **New and novel glycosides, glycoconjugates and processes for their preparation.**

(30) Priority: **23.06.82 SE 8203925**

(43) Date of publication of application:
**11.01.84 Bulletin 84/02**

(45) Publication of the grant of the patent:
**07.06.89 Bulletin 89/23**

(84) Designated Contracting States:
**AT BE CH DE FR GB IT LI NL SE**

(56) References cited:
**CHEMICAL ABSTRACTS, vol. 82, no. 1, January 6, 1975, p. 396, abstract no. 4529v, COLUMBUS OHIO (US), R.T. LEE et al.: "Synthesis of 3-(2-aminoethylthio)propyl glycosides"**

**CARBOHYDRATE RESEARCH, vol. 111, no. 1, December 1982, Elsevier Scientific Publishing Company AMSTERDAM (NL), J. DAHMEN et al.: "Preparation and applications of 2-bromoethyl glycosides: synthesis of spacer-arm glycosides and agglutination inhibitors", pages C1-C4.**

(73) Proprietor: **BIOCARB AB**
**S-223 70 Lund (SE)**

(72) Inventor: **Dahmén, Jan**
**Lärkv. 5**
**S-232 02 Akarp (SE)**
Inventor: **Frejd, Torbjörn**
**Vattug. 9C**
**S240 17 Södra Sandby (SE)**
Inventor: **Magnusson, Göran**
**Dalins Väg 1**
**S-223 60 Lund (SE)**
Inventor: **Noori, Ghazi**
**Uardav. 2F**
**S-223 71 Lund (SE)**

(74) Representative: **Henningsson, Gunnar et al**
**Bergling & Sundbergh AB Box 7645**
**S-103 94 Stockholm (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 098 252 B1

## Description

The present invention relates to novel O-glycosides and glycoconjugates, as well as to a process for the preparation of the glycosides.

A growing appreciation of the role of glycoconjugates in biological receptor interactions has recently directed much interest into the chemistry and molecular biology of these compounds. A vast array of biological observations have been described in the recent literature and several excellent reviews have appeared. Blood group antigens are the classical examples of carbohydrate receptors. During the last few years it has been shown that these receptors are ubiquitous and modern studies now include such diverse fields as lectines and antibodies, cell-surface receptors on bacteria, viruses, mycoplasma and protozoans; bacterial toxins; hormone receptors; cell-growth regulation and oncogenesis; sperm-egg interaction and fertilization; recognition phenomena in plants, targeting of drugs.

Chemical synthesis and modification of glycoconjugates has led to for example artificial immunogens for the preparation of carbohydrate-specific antibodies, artificial glycolipids for the incorporation into liposomes and functionalized glycosides have been used for the preparation of solid phases for affinity chromatography. In all these cases there is a need for stereospecific methods that permit the preparation of pure α- or β-glycosides having spacer-arm aglycons that at a later stage can be covalently coupled to a carrier molecule or particle, preferably under physiological conditions. In addition, simple-aglycon (e.g. methyl and ethyl) glycosides are needed for specificity studies of agglutination systems like erythrocyte-antibody and -bacteria interactions.

Spacer-arm glycosides of oligosaccharides can be prepared by two principally different routes. Total synthesis, using protected monosaccharides as starting materials, usually leads to anomerically pure compounds but the total yield of the reaction sequence is low. In the second approach, the spacer-arm is connected (by glycoside synthesis) to the reducing end of the complete oligosaccharide (obtained from biological material or by synthesis). The latter method is preferred when the oligosaccharide can be obtained in large quantity from an inexpensive starting material (e.g. by partial hydrolysis of polysaccharides). However, it can be difficult to separate α- and β-glycosides especially when long spacer-arms and/or higher oligosaccharides are used.

The general problem of glycoside synthesis has been reviewed in a most recommendable way by Bochkov and Zaikow[1], whereas the methods for preparation of spacer-arm glycosides are scattered in the chemical literature[2].

Certain general criteria can be defined for the preparation of spacer-arm glycosides and their coupling to carrier molecules (or particles):

i) It should be possible to connect the spacer-arm and the carbohydrate moiety (by glycoside synthesis) in a stereospecific way, so that pure α- or β-glycosides can be obtained.

ii) The spacer-arm should be compatible with standard reagents and reaction conditions used in synthesis and modification of carbohydrates.

iii) It should be possible to vary the length, hydrophobicity and end-group functionality of the spacer-arm.

iv) It should be possible to activate the spacer-arm terminal group at physiological conditions for the following coupling to proteins and other sensitive materials.

v) The spacer-arm should couple covalently and selectively to the carrier.

vi) The surface charge and conformation of the carrier (for example protein) should not be changed drastically by the coupled carbohydrate moiety.

In the plethora of methods described in the literature[16], there can be found some individual cases where most of the above-mentioned criteria are met. However, there still exists a need for general methods that allows for a flexible choice of spacer-arm without the necessity of carrying out a new glycoside synthesis for every variation.

There have been a few reports in the literature on the stepwise construction of spacer-arm glycosides[3] where for example the standard allyl protecting group has been used as the initial aglycon, ω-Haloalkyl glycosides have not been used for this purpose (although a synthesis of 2-bromoethyl-β-D-glucopyranoside (*3*) and its further coupling to certain aromatic amines has been reported[5]).

Based on the present invention there is now introduced a new and improved sequential synthesis of spacer-arm glycosides based on the initial preparation of an anomerically pure ω-haloalkyl glycoside (in the following text, reference is usually made to 2-bromoethyl glycosides) of the biologically active oligosaccharide in question followed by the substitution of bromine by a suitably functionalized thiol that allows for the final coupling to the carrier. Furthermore, the bromine atom can be easily replaced by hydrogen (or tritium), using standard hydrogenation conditions, resulting in alkyl glycosides that can be used as inhibitors for the evaluation of agglutination reactions. The 2-bromoethyl group seems to be compatible with most of the standard reactions that are used in synthetic carbohydrate chemistry thus allowing for involved multistep preparations of oligosaccharide 2-bromoethyl glycosides (*vide infra*).

Although in the present disclosure reference is mostly had to the 2-bromoethyl group as a part of the glycosides prepared, it should be noted that the corresponding chloro and iodo derivatives are equally useful. The chloro and iodo varieties thus function in much the same manner as the corresponding 2-bromo derivatives and the invention covers all three halides. Furthermore, the invention covers as starting

material, glycosides, wherein the ω-haloalkyl moiety contains 2 carbon atoms.

Such starting materials are prepared by reacting an activated sugar selected from:

a 1-acyl derivative of a sugar,
a 1,2-orthoacyl ester derivative of a sugar,
a 1-bromo derivative of a sugar,
a 1,2-oxazoline derivative of a sugar,
a 1-acyl 2-deoxy-2-phthalimido derivative of a sugar and
a 1-bromo-2-deoxy-2-phthalimido derivative of a sugar;

with an alcohol having the formula: Hal-$(CH_2)_m$—OH wherein Hal equals chlorine, bromine or iodine and m is an integer of from 2—20, inclusive, to form the O-glycoside, and recovering the glycoside formed.

A preferred embodiment of the said process involves glycosidating a 1,2-sugar acetate, orthoester, oxazoline or 1-acyl-2-phthalimido derivative in the presence of a Lewis acid. Another embodiment of the process of the invention involves glycosidation of a glycosyl bromide with the aid of a heavy-metal salt, optionally protecting the 2-hydroxyl group. The reaction may be performed in an organic solvent, for example methylene chloride.

For the purpose of attaching the glycoside prepared to a spacer arm the process of the invention comprises nucleophilic addition of a 2-functional spacer arm compound, the nucleophilic functionality of which comprises sulphur. The compound hereby obtained may via its other functionality be then covalently linked to a carrier. Such carrier may comprise a protein or a modified polysaccharide, in which case the linking reaction is preferably performed in aqueous solution. The said other functionality of the spacer arm compound may be selected from CHO, COOH, $CON_3$ and $NH_2$, H, $CH(OR'')_2$, $NO_2$, OH, SH, $COOCH_3$, $COOCH_2CH_3$, $CONHNH_2$, wherein R'' is $C_{1-4}$-alkyl.

The said process finds particular utility when a mixture of α- and β-2-bromoethyl glycosides is formed, in that the isomers may be easily separated, the desired isomer being recovered.

Some applications of the inventive concept are diagrammatically shown in Fig. 1 as appended.

The invention provides for new and novel O-glycosides having the general formula:

$$(sugar)_n\text{-O—}(CH_2)_m\text{—S—R—R'}$$

wherein n is an integer of from 1—10, inclusive, m is 2, R is selected from the group consisting of alkylene having at most 25 carbon atoms and arylene and R' is selected from the group consisting of H, CHO, $CH(OR'')_2$, $NO_2$, $NH_2$, OH, SH, COOH, $COOCH_3$, $COOCH_2CH_3$, $CONHNH_2$ and $CON_3$, wherein R'' is $C_{1-4}$-alkyl. In the said formula the sugar moiety is preferably selected from the group of aldoses defined below in this disclosure. Moreover, in the above formula R' is suitably a carboxylic acid derivative, preferably an ester. R' may also preferably be sulfhydryl group, hydrogen, an amino group or a nitro group. In the two last instances it is also preferred that R is arylene.

In the O-glycoside of the invention the sugar is preferably an aldose selected from a D-glucose, D-galactose, D-mannose, D-xylose, L-fucose, 2-acetamido-2-deoxy-D-glucose, 2-deoxy-2-phthalimido glucose, 2-acetamido-2-deoxy-D-galactose, 2-azido-2-deoxy-D-glucose, 2-azido-2-deoxy-D-galactose, D-glucuronic acid, D-galacturonic acid, 2-deoxy-2-phthalimido glucose and 2-deoxy-2-phthalimido galactose. In particular, the aldose is galactose and n is preferably an integer from 1—4, inclusive. In another preferred embodiment the sugar moiety of the O-glycoside of the invention contains both galactose and 2-acetamido-2-deoxcy-D-glucose or 2-acetamido-2-deoxy-D-galactose, in which case n is preferably an integer from 2—5, inclusive.

Examples of neo-glycolipids for use as hydrophobic coating materials are:

D-Galβ1→4-D-GlcNAc β$OCH_2CH_2S$—$(CH_2)_1CH_3$,
L-Fucα1-2-D-Gal β$OCH_2CH_2S$—$(CH_2)_1CH_3$,
D-Galα1→4-D-Galβ1→4-D-Glcβ$OCH_2CH_2S$—$(CH_2)_1CH_3$,
D-Galα1→4-D-Galβ1→4-D-GlcNAcβ$OCH_2CH_2S$—$(CH_2)_1CH_3$,
D-Glcα1→6-D-Glcα1→4-D-Glcα1→4-D-Glcβ$OCH_2CH_2S$—$(CH_2)_1CH_3$, and
D-Galβ1→4GlcNAcβ$OCH_2CH_2S$—$(CH_2)_1CH_3$,
wherein 1 is ≤ 24, e.g. 7 or 17.

According to another aspect of the invention there is provided a process for preparing an O-glycoside of the general formula:

$$(sugar)_n\text{-O—}(CH_2)_m\text{—S—R—R'}$$

wherein the different symbols are as previously defined, comprising reacting an O-glycoside having the general formula:

$$(sugar)_n\text{-O—}(CH_2)_m\text{-Hal}$$

wherein Hal is chlorine, bromine or iodine, m is an integer of 2—20, inclusive, and n is an integer of 1—10, inclusive, with a thiol of the general formula:

$$\text{HS—R—R'}$$

wherein R and R′ have the above meaning.

The invention also provides for glycoconjugates having the general formula:

$$(sugar)_n\text{-}O—(CH_2)_m—S—R—R''$$

wherein n is an integer of from 1—10, inclusive, m is 2, R is selected from the group consisting of alkylene having at most 25 carbon atoms and arylene and R″ constitutes a carrier. Such glycoconjugates may have the formula

$$D\text{-}Gal\alpha1\rightarrow4\text{-}D\text{-}Gal\beta OCH_2CH_2—S—CH_2CH_2CONH—R'''$$

wherein R‴ is selected from the group consisting of residues of proteins, polysaccharides, plastic materials and inorganic materials. Among suitable materials forming R‴ there may be mentioned bovine serum albumin (BSA), key-hole limpet haemocyanine (KLH), aminated sepharoses, silica gels, etc.

Particularly preferred glycoconjugates are:
D-Galβ1→4-D-GlcNAcβ$OCH_2CH_2SCH_2CH_2CONH$—R‴,
L-Fucα1→2-D-Galβ$OCH_2CH_2SCH_2CH_2CONH$—R‴,
D-Galα1→4-D-Galβ1→4-D-Glcβ$OCH_2CH_2$—S—$CH_2CH_2CONHR'''$,
D-Galα1→4-D-Galβ1→4-D-GlcNAcβ$OCH_2CH_2$—S—$CH_2CH_2CON$—R‴,
D-Glcα1→6-D-Glcα1→4-D-Glcα1→4-D-Glcβ$OCH_2CH_2$—S—$CH_2CH_2$—CONH—R‴ and
D-Galβ1→4GlcNAcβNAcβ$OCH_2CH_2$—S—$CH_2CH_2CONH$—R‴,
wherein R‴ is selected from the group consisting of residues of proteins, polysaccharides, plastic materials and inorganic materials.

The above-identified glycoconjugates are suitably prepared by reacting a glycoside of the general formula:

$$(sugar)_n\text{-}O—(CH_2)_m—S—R—R'$$

with an appropriate carrier chosen from those mentioned above.

Within its scope the invention provides for a bi-dentate O-glycoside having the general formula:

$$[(sugar)_n\text{-}O—(CH_2)_m—S]_2\text{-}R$$

wherein m, n and R have the above meaning. Such bi-dentate O-glycosides may be prepared by reacting a glycoside of the general formula:

$$(sugar)_n\text{-}O—(CH_2)_m\text{-}Hal$$

with a dithiol of the general formula:

$$HS—(CH_2)_m—SH$$

wherein m is an integer of 2—20, inclusive.

Preferred ethyl glycosides are:
D-Galα1→4-D-Galβ$OCH_2CH_3$,
L-Fucα1→2-D-Galβ$OCH_2CH_3$,
D-Galα1→4-D-Galβ1→4-D-Glcβ$OCH_2CH_3$,
D-Galα1→4-D-Galβ1→4-D-GlcNAcβ$OCH_2CH_3$,
D-Glcα1→6-D-Glcα1→4-D-Glcα1→4-D-Glcβ$OCH_2CH_3$ and
D-Galβ1→4GlcNAcβ$OCH_2CH_3$.
These are particularly useful as agglutination inhibitors.

Furthermore, the number of sugar entities in the O-glycosides described can be increased by transformation to higher glycosides. This is provided for by reacting an activated sugar with a glycoside of the formula:

$$(sugar)_{n-x}\text{-}O—(CH_2)_m\text{-}Hal$$

where x is an integer of from 1 to n-1, inclusive, the resulting higher glycoside being recovered. This process may preferably be used to prepare 2-bromoethyl glycosides of the formula:
L-Fucα1→-D-Galβ$OCH_2CH_2Br$,
D-Galα1→4-D-Galβ1→4-D-Glcβ$OCH_2CH_2Br$, and
D-Galα1→4-D-Galβ1→4-D-GlcNPhthβO—$CH_2CH_2Br$.

In the processes described herein it is sometimes desirable to protect certain groups of the sugar reactants to prevent undesired side reactions to take place. This is conventional in the art and for details regarding such protection of reactive groups *vide* references 55 and 56.

In regard to the glycosides and glycoconjugates prepared according to this invention it is to be noted that the invention covers α- and β-configurations as well as mixtures thereof.

Preparation and characterization of 2-bromoethyl glycosides

The compounds can be prepared by standard methods for glycoside synthesis (cf ref. 1) starting from acetylated or benzylated 1-bromo sugars. For simple sugars (monosaccharides and a few disaccharides) it turns out that a direct glycosidation of the per-O-acetylated sugar with 2-bromoethanol, using boron-trifluoride etherate in methylene chloride, is a very expedient process that normally permits the isolation of the pure 1,2-*trans* glycoside in crystalline form, directly from the crude reaction mixture (see Examples and Table 1). We recently reported this use of $BF_3$-etherate for the synthesis of 2,2,2-trichloroethyl glycosides[6]. The same Lewis acid has been used for anomerization purposes[7] and for the preparation of a variety of thioglycosides[8].

Because of difficulty of separating anomeric pairs of higher oligosaccharides, it is in this case normally advisable to use more stereo selective methods of glycoside synthesis[1] than the $BF_3$-etherate method mentioned above. We have for example been able to synthesize 1,2-*trans* 2-bromoethyl glycosides using the silver trifluoromethane-sulphonate method[9] and 1,2-*cis* glycosides by a variation[10] of the quaternary ammonium bromide method devised by Lemieux[11], all in good yield and stereoselectivity (cf Table 1). In addition, 2-bromoethyl 3,4,6-tri-*O*-acetyl-2-acetamido-2-deoxy-β-D-glucopyranoside (*8*) was obtained by the oxazoline method[25] (Table 1). Figure 2 as appended shows a summary of the different methods.

Table 1. Product data and yields for the preparation of 2-bromoethyl glycosides

| Product | | Method[a] | Yield (%)[b] | M.p. (°C) | $[\alpha]_D^{24}$ (°) |
|---|---|---|---|---|---|
| 1 | 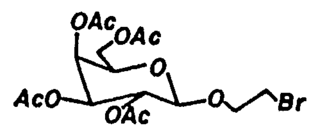 | A | 46 | 114-116 | -5.2 (*c* 1,4, $CDCl_3$) |
| 2 | 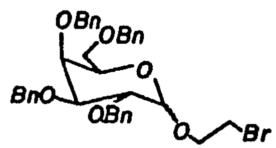 | D | 18[c] *(2α)*<br>30[c] *(2α+2β)* | sirup | +30.5 (*c* 1,4, $CHCl_3$) |
| 3 | 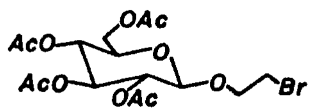 | A | 42 | 119-120 | -10.9 (*c* 1.2, $CDCl_3$) |
| 4 | 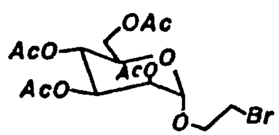 | A | 58 | 118-119 | +44.7 (*c* 0.6, $CDCl_3$) |
| 5 | 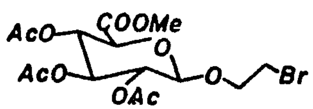 | A | 36 | 142-143 | -21.4 (*c* 0.8, $CDCl_3$) |
| 6 | 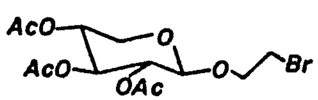 | A | 85 | 144-145 | -47.9 (*c* 1.7, $CDCl_3$) |

Table 1 (contd.)

| Product | | Method[a] | Yield (%)[b] | M.p. (°C) | $\|\alpha\|_D^{24}$ (°) |
|---|---|---|---|---|---|
| 7 | 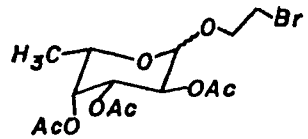 | A | 53[c] (7β)<br>16[c] (7α) | sirup<br>sirup | - 24.7 (*c* 2.2, $CDCl_3$)<br>- 22.4 (*c* 0.8, $CDCl_3$) |
| 8 | 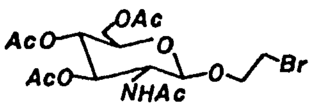 | C | 39 | 174-175 | -6.6 (*c* 1.1, $CDCl_3$) |
| 9 | 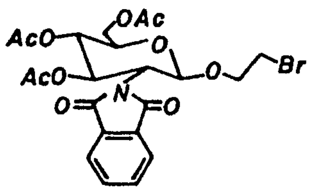 | A | 50[c] | 111-114 | +21.4 (*c* 1.0, $CHCl_3$) |
| 9b | 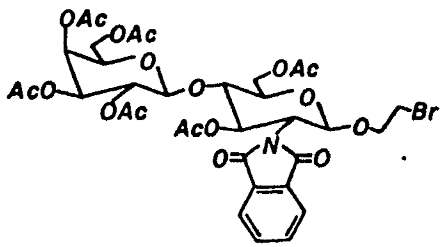 | A | 55[c] | 224-225 | +9.3 (*c* 1.0, $CHCl_3$) |
| 10 | 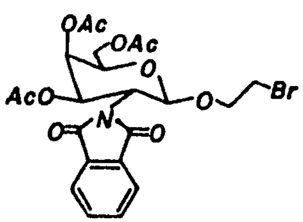 | A | 80[c] | sirup | -11.0 (*c* 0.7, $CHCl_3$) |

Table 1 (contd.)

| Product | | Method[a] | Yield (%)[b] | M.p. (°C) | $[\alpha]_D^{24}$ (°) |
|---|---|---|---|---|---|
| 11 | 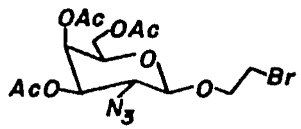 | E | 8[c] | sirup | +127 (c 1.0, $CHCl_3$) |
| 12 | 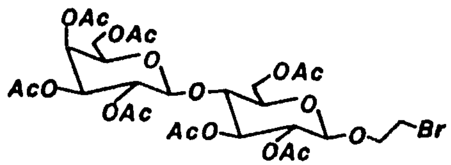 | A | 55[c] | amorph. | -10.9 (c 1.3, $CHCl_3$) |
| 13 | 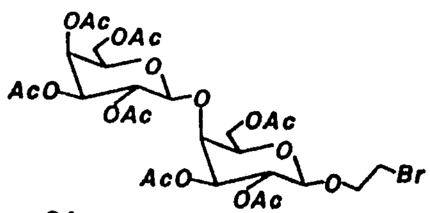 | A | 67[c] | amorph. | + 5.8 (c 2.4, $CDCl_3$) |
| 14 | 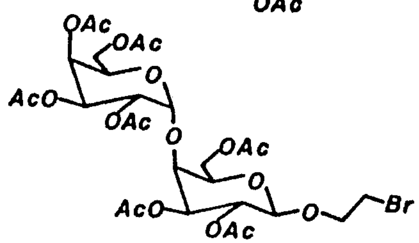 | B | 77[c] | 174-177 | +75.2 (c 2.0, $CHCl_3$) |

a) See EXAMPLES b) Isolated by spontaneous crystallisation of the crude reaction mixture.
c) Isolated by chromatography on silica gel.

The physical and chemical properties of 2-bromoethyl glycosides, as well as their simple preparation, make these compounds attractive intermediates for the preparation of neo-glycoconjugates. The monosaccharide glycosides crystallize very well, which makes the preparation of anomerically pure compounds simple; the chemical reactivity of these glycosides will be dealt with later in this report.

The $^{13}C$—NMR spectra of the different 2-bromoethyl glycosides show just a slight variation in the chemical shift of the bromoethyl group carbon signals (cf Table 2). The $^{1}H$—NMR spectra (Table 3) are on the other hand sometimes difficult to analyze because the complex spin-pattern of the bromoethyl group can obscure some of the sugar ring proton signals. Figure 3 is appended depicts a typical spectrum (bromoethyl group part) together with a computer simulation. In this special case, there was no interference from the sugar ring protons.

Table 2. $^{13}$C-NMR chemical shifts for per-O-acetylated 2-bromoethyl glycosides

Numbering: 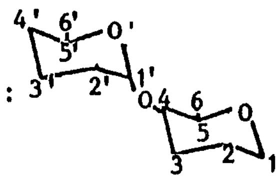

| Nr | C1 | C2 | C3 | C4 | C5 | C6 | C7[c] | C8[c] | C1' | C2' | C3' | C4' | C5' | C6' | $CH_3$ | CO (acetyl) |
|---|---|---|---|---|---|---|---|---|---|---|---|---|---|---|---|---|
| | Chemical shift[a] | | | | | | | | | | | | | | | |
| 1 | 102.0 | 71.3[b] | 71.2 | 70.2 | 67.4 | 61.7 | 69.0 | 30.4 | | | | | | | 21.3 21.1<br>21.1 21.0 | 170.8 170.7<br>170.6 170.0 |
| 3 | 101.5 | 73.1 | 72.4 | 71.5 | 70.2 | 62.3 | 68.8 | 30.3 | | | | | | | 21.1 21.2<br>21.0 21.0 | 171.1 170.7<br>169.8 169.8 |
| 4 | 98.2 | 69.9 | 69.5 | 69.4 | 66.5 | 62.9 | 69.0 | 30.1 | | | | | | | 21.3 21.2<br>21.2 21.1 | 171.1 170.5<br>170.3 170.2 |
| 5 | 101.4 | 73.1 | 72.4 | 71.5 | 70.4 | 167.6 | 69.9 | 30.3 | | | | | | | 21.2 21.1<br>21.0 53.5 | 170.6 169.9<br>169.8 |
| 6 | 100.7 | 71.0 | 70.4 | 68.7 | 62.0 | | 69.2 | 30.0 | | | | | | | 20.8 20.8<br>20.8 | 170.0 169.8<br>169.5 |
| 7α | 96.4 | 71.1 | 68.1 | 67.9 | 64.8 | 15.9 | 68.4 | 30.2 | | | | | | | 20.9 20.7<br>20.7 | 170.6 170.6<br>170.0 |
| 7β | 101.3 | 71.2 | 70.1 | 69.3 | 68.7 | 16.1 | 69.5 | 30.1 | | | | | | | 20.7 20.7<br>20.6 | 170.6 170.2<br>169.6 |
| 8 | 100.7 | 54.5 | 72.2 | 71.7 | 69.0 | 62.2 | 69.5 | 30.4 | | | | | | | 23.3 20.7<br>20.7 20.6 | 170.9 170.7<br>170.6 169.4 |
| 9 | 98.3 | 54.4 | 72.0 | 70.6 | 68.9 | 61.9 | 69.8 | 29.8 | | | | | | | 20.8 20.6<br>20.5 | 170.7 170.1<br>169.4 |
| 9b | 98.0 | 54.7 | 72.7 | 75.4 | 66.5 | 62.0 | 69.7 | 29.8 | 101.0 | 71.0 | 70.9 | 70.6 | 69.0 | 60.6 | 20.8 20.6<br>20.5 20.5 | 170.4 170.3<br>170.1 170.0<br>169.7 169.0 |

Table 2. (contd.)

| Nr | C1 | C2 | C3 | C4 | C5 | C6 | C7[c] | C8[c] | C1' | C2' | C3' | C4' | C5' | C6' | $CH_3$ | | CO (acetyl) | |
|---|---|---|---|---|---|---|---|---|---|---|---|---|---|---|---|---|---|---|
| 10 | 98.7 | 51.2 | 71.0 | 66.7 | 67.9 | 61.4 | 69.8 | 29.9 | | | | | | | 20.7<br>20.5 | 20.7 | 170.4<br>169.8 | 170.3 |
| 11 | 98.4 | 57.3 | 67.2 | 67.6 | 68.1 | 61.7 | 68.9 | 29.5 | | | | | | | 20.71<br>20.63 | 20.67 | 170.4<br>169.8 | 170.0 |
| 12 | 101.1 | 72.8 | 71.4 | 76.2 | 70.7 | 61.9 | 69.8 | 29.8 | 100.8 | 72.6 | 71.0 | 69.1 | 66.6 | 60.8 | 20.8<br>20.8<br>20.6<br>20.6 | 20.8<br>20.8<br>20.6 | 170.4<br>170.3<br>170.0<br>169.0 | 170.4<br>170.1<br>169.7 |
| 13 | 101.9 | 73.1 | 70.1 | 74.2 | 68.7 | 63.3 | 69.0 | 30.2 | 100.9 | 72.0 | 70.1 | 68.5 | 66.9 | 61.3 | 20.9<br>20.8<br>20.6<br>20.6 | 20.8<br>20.8<br>20.6 | 170.6<br>170.2<br>170.2<br>169.4 | 170.4<br>170.2<br>169.5 |
| 14 | 101.3 | 72.5 | 68.5 | 76.9 | 67.8 | 60.5 | 69.5 | 30.0 | 99.3 | 72.0 | 68.4 | 67.3 | 67.1 | 91.9 | 20.9<br>20.7<br>20.6<br>20.6 | 20.7<br>20.7<br>20.6 | 170.6<br>170.4<br>170.1<br>169.2 | 170.5<br>170.4<br>169.8 |

a) ppm, $CDCl_3$ internal TMS

b) underlined values can be interchanged

c) identified by the INEPT[12] technique.

Table 3. $^{1}$H-NMR chemical shifts[a] and coupling constants[b] for per-O-acylated 2-bromoethyl glycopyranosides[c]

| Nr | H1 | H2 | H3 | H4 | H5ax | H5eq | H6 | H6' | $O-CH_2CH_2$ | $CH_2-CH_2Br$ | $CH_3-CO$ |
|---|---|---|---|---|---|---|---|---|---|---|---|
| 1 | 4.55[a] | 5.24 | 5.03 | 5.41 | 3.75-3.87 | | 4.10---4.25 | | 3.87-4.20 | 3.43-3.53 | 2.16 2.09 |
| | ($J_{1,2}$ 7.8)[b] | ($J_{2,3}$ 10.5) | ($J_{3,4}$ 3.5) | ($J_{4,5}$ 1,0) | (m[d]) | | (---m---) | | (---m---) | (---m---) | 2.06 1.99 |
| 3 | 4.58 | 5.03 | 5.23 | 5.09 | 3.66-3.78 | | 4.10---4.34 | | 3.78-4.20 | 3.42-3.51 | 2.10 2.08 |
| | ($J_{1.2}$ 7.8) | ($J_{2,3}$ 9.5) | ($J_{3,4}$ 9.5) | ($J_{4,5}$ 9.5) | ( m ) | | (---m---) | | (---m---) | (---m---) | 2.03 2.02 |
| 4 | 4.88 | 5.20-------------------5.40 | | | 4.09-4.19 | | 4.10---4.34 | | 3.83-4.50 | 3.53 | 2.16 2.11 |
| | ($J_{1,2}$ 1.5) | (--------------m--------------) | | | (-- m --) | | (---m---) | | (-- m --) | (t, J 5.8) | 2.06 2.00 |
| 5 | 4.63 | 5.04 | 5.29 | 5.22 | 4.06 | 3.77($COOCH_3$) | | | 3.75-4.27 | 3.43-3.50 | 2.06 2.02 |
| | ($J_{1,2}$ 7.6) | ($J_{2,3}$ 9.5) | ($J_{3,4}$ 9.5) | ($J_{4,5}$ 9.4) | | | | | (-- m --) | (-- m --) | 2.02 |
| 6 | 4.56 | 5.03-4.90 | 5.17 | 5.03-4.90 | 4.15 | 3.39 | | | 3.75-4.15 | 3.47 | 2.08 2.06 |
| | ($J_{1,2}$ 6.7) | ($J_{2,3}$ 8.3) | ($J_{3,4}$ 8.3) | ($J_{4,5ax}$ 5.0; $J_{4,5eq}$ 8.5; $J_{5ax,5eq}$ 12.0) | | | | | (-- m --) | (t, J 6.0) | 2.05 |
| 7α | 5.14 | 5.05-------------------5.43 | | | 4.26 | | 1.12 | | 3.81 3.84 | 3.50 | 2.17 2.10 |
| | ($J_{1,2}$ 0) | (--------------m-------------) | | | ($J_{5,6}$ 6.4) | | | | ($J_{AB}$ -12.0) c.f. Fig 3 | (t, J 5.8) | 2.00 |
| 7β | 4.49 | 5.17-5.26 | 5.02 | 5.17-5.26 | 3.73-3.88 | | 1.20 | | 3.73-4.32 | 3.43-3.50 | 2.17 2.07 |
| | ($J_{1,2}$ 7.9) | ($J_{2,3}$ 10.5) | ($J_{3,4}$ 3.5) | (-- m --) | ($J_{5,6}$ 6.4) | | | | (-- m --) | (-- m --) | 1.98 |
| 8 | 4.77 | 3.78-3.93 | 5.32 | 5.07 | 3.66-3.78 | | 4.08---4.31 | | 3.78-4.19 | 3.48 | 2.08 2.02 |
| | ($J_{1,2}$ 8.0) | ($J_{2,3}$ 10.0) | ($J_{3,4}$ 10.0) | ($J_{4,5}$ 10.0) | (-- m --) | | (--- m ---) | | (-- m --) | (t, J 6.0) | 2.01 1.96 |

Table 3. (contd.)

| Nr | H1 | H2 | H3 | H4 | H5ax | H5eq | H6 | H6' | O–$CH_2CH_2$ | $CH_2$–$CH_2Br$ | $CH_3$–CO |
|---|---|---|---|---|---|---|---|---|---|---|---|
| 9 | 5.43 | 4.35 | 5.82 | 5.19 | 3.80-------------4.40 | | | | 3.70-4.20 | 3.30-3.40 | 2.13 2.04 |
| | ($J_{1,2}$ 8.4) | ($J_{2,3}$ 10.7) | ($J_{3,4}$ 9.6) | ($J_{4,5}$ 9.6) | (----------m----------) | | | | (---m---) | (---m---) | 1.88 |
| 10 | 5.37 | 4.57 | 5.83 | 5.50 | 4.10------------------4.25 | | | | 3.76 | 3.34 | 2.21 2.02 |
| | ($J_{1,2}$ 8.5) | ($J_{2,3}$ 11.5) | ($J_{3,4}$ 3.4) | | (-----------m-------------) | | | | ( m ) | ( m ) | 1.87 |
| 11 | 5.07 | 3.68 | 5.39 | 5.48 | 4.37 | | 4.12 | 4.09 | 3.88-4.05 | 3.55 | 2.15 2.06 |
| | ($J_{1,2}$ 3.7) | ($J_{2,3}$ 11.2 | ($J_{3,4}$ 3.4) | ($J_{4,5}$ <1) | ($J_{5,6}$ ~6) | | (---m---) | | (---m---) | (t, J 6.0) | 2.06 |

Table 3. (contd.)

| Nr | H1 | H2 | H3 | H1' | H2' | H3' | H4' | $CH_2Br$ | $CH_3CO$ |
|---|---|---|---|---|---|---|---|---|---|
| 12 | 4.54<br>($J_{1,2}$ 8.0) | 5.21<br>($J_{2,3}$ 9.5) | 4.92<br>($J_{3,4}$ 8,0) | 4.49<br>($J_{1',2'}$ 8.0) | 5.12<br>($J_{2',3'}$ 10.5) | 4.96<br>($J_{3',4'}$ 3.5) | 5.35<br>($J_{4',5'}$ 1.0) | 3.44<br>(t, J 6.0) | 2.15 2.13<br>2.06 2.05<br>2.04 1.97 |
| 13 | 4.49<br>($J_{1,2}$ 7.8) | 5.10<br>($J_{2,3}$ 10.0) | 4.89<br>($J_{3,4}$ 3.0) | 4.42<br>($J_{1',2'}$ 7.9) | 5.25<br>($J_{2',3'}$ 10.5) | 5.01<br>($J_{3',4'}$ 3.5) | 5.36<br>($J_{4'5'}$ 1.0) | 3.47<br>(t, J 6.0) | 2.17 2.15<br>2.11 2.08<br>2.06 2.04<br>1.99 |
| 14 | 4.54<br>($J_{1,2}$ 8.0) | 5.22<br>($J_{2,3}$ 11.0) | 4.83<br>($J_{3,4}$ 3.0) | 5.01<br>($J_{1',2'}$ 3.0) | 5.39<br>($J_{2',3'}$ 11.0) | 5.21<br>($J_{3',4'}$ 3.0) | 5.57<br>($J_{4',5'}$ 1.0) | 3.49<br>(t, J 5.0) | 2.15 2.12<br>2.11 2.09<br>2.06 2.00 |
| 9b | 5.42<br>($J_{1,2}$ 8.5 | | 5.77<br>($J_{3,4}$ 7.9) | 4.55<br>($J_{1',2'}$ 7.9) | 5.13<br>($J_{2',3'}$ 7.7) | 4.97<br>($J_{3',4'}$ 3.3) | 5.34<br>($J_{4',5'}$ <1.0) | | |

a) ppm, $CDCl_3$, internal TMS.

b) observed spacing

c) For numbering, see Table 2

d) complex pattern, "multiplet".

Preparation of complete spacer-arm glycosides

The 2-bromoethyl glycosides can conceivably be elaborated into a vast array of compounds that are suited for coupling to proteins and other macromolecular entities. It is, however, evident from the literature[2], that two different functional groups can master virtually all cases of covalent attachment of the glycoside to the carrier molecule. Accordingly, we decided to use terminal amino- (in the form of a para-substituted aniline) and carboxyl group derivatives for coupling (by amide bond formation) to carrier carboxyl and amino groups respectively. In addition, non-covalent bonding (so called hydrophobic bonding) is known to occur between for example long aliphatic chains and lipid cellular membranes. Spacer-arms of this type thus makes coating of biological membranes by well-defined oligosaccharides possible.

Table 4 shows the model spacer-arm glycosides (see Examples for preparation). The thiols are all commercially available compounds. Aliphatic thiol esters with other chain-length can be prepared by standard synthetic methods using ω-hydroxy esters (easily available from alicyclic ketones by the Baeyer-Villiger reaction followed by alcoholysis), thus allowing great variability in the length of the spacer-arm. Variation of the fatty alkyl chain length and branching can lead to synthetic glycolipids that bind into cellular membranes or form liposomes and other aggregates in many different ways.

Primary alkyl bromides are well-known alylating agents and thiols (especially thiolates) are nucleophiles of high repute. It is also possible to perform the reaction with protected sugars, such as per-O-acetates, since this adds flexibility in the choice of chromatographic methods for final purification. In the "anhydrous" method (G in Table 4) deacetylation before work-up and purification of the reaction gives good yields of the desired products.

The "aqueous" method (F in Table 4) is selective and gives the desired sulphides in high yield without any deacetylation or other unwanted side reactions. Table 4 shows spacer-arm glycosides that are ready for deprotection and/or chemical activation that finally makes them suited for coupling to carrier molecules.

Table 4. Product yields in the reaction: 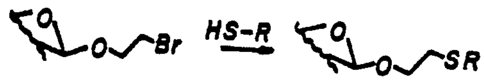


| Product | | Method[a] | Yield (%)[b] |
|---|---|---|---|
| 15 | 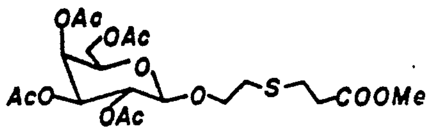 | F | 73 |
| 16 | 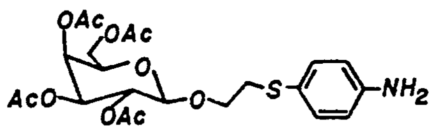 | F | 71 |
| 17 | 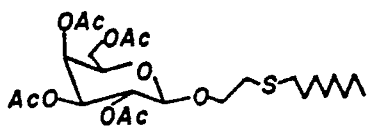 | F | 70 |
| 18 | 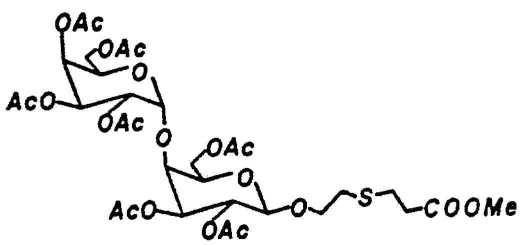 | F | 80 |
| 19 | 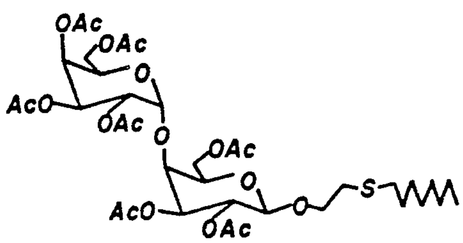 | F | 71 |
| 20 | 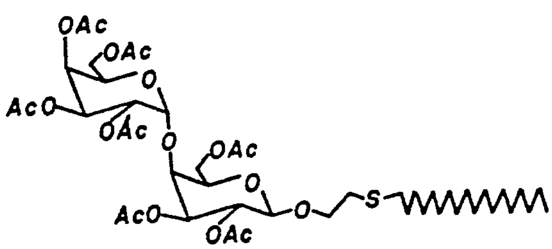 | G | 69 |

Table 4. (contd.)

| Product | | Method[a] | Yield (%)[b] |
|---|---|---|---|
| 21 | 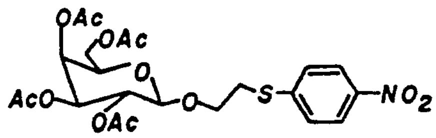 | G | 62 |
| 22 | 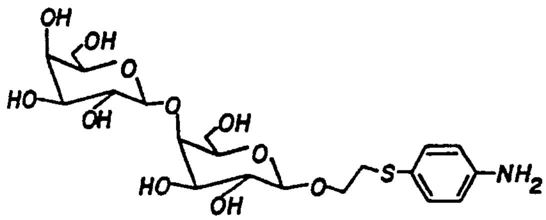 | G | 99 |
| 23 | 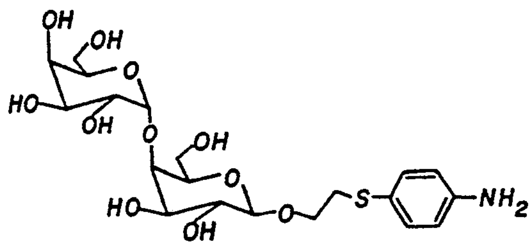 | G | 97 |

a) Method F: Phase transfer catalysis; benzene/$H_2O$/$Cs_2CO_3$ or NaOH/$(C_8H_{17})_3NCH_3Br$.
Method G: DMF/NaH or $Cs_2CO_3$.
For details, see EXAMPLES.

b) Pure compound by chromatographic isolation.

Preparation of complete neo-glycoconjugates

The present invention makes it possible to produce neo-glycoconjugates where the carbohydrate part is well characterized both regio- and stereospecifically. Furthermore, by employing the same intermediate 2-bromoethyl glycosides, it is possible to prepare oligosaccharide glycosides that are well suited for the study of inhibition of agglutination reactions between for example erythrocytes and antibodies raised against the neo-glycoconjugates mentioned above. Preparation of these inhibitors is described below.

Much ingenuity has been spent on the problem of attaching spacer-arm glycosides to macromolecules under semi-physiological conditions[2], [14]. We have applied well-established reaction conditions for the attachment of two model compounds to proteins (see Figure 4). The simple spacer-arm galactoside *24* was coupled to bovine serum albumine (BSA) by the method developed by Inman[15] and Lemieux[14] and coworkers in order to investigate the degree of binding (number of hapten molecules per molecule of BSA). This was done by the wellknown phenol/sulphuric acid method[16] but also with a more unconventional method. The spacer-arm used in this work contains a sulphur atom which makes it possible to determine the amount of covalently bound glycoside by a differential elemental (combustion) analysis of the amount of sulphur added to the protein. Both methods reveal that 35 galactose units were bound to each protein molecule. The highest possible degree of binding is 57 (the number of terminal amino groups in lysin residues).

The "aniline" spacer-arm was used to couple the di-galactoside *23* by the thio-phosgene method[17] to the proteins BSA and key-hole limpet haemocyanine (KLH). The degree of coupling was determined by the antrone method[18] which indicated a binding of 37 and 437 hapten molecules per protein molecule. The neo-glycoproteins thus produced were used as antigens in order to raise monoclonal anti-bodies against the di-galactose structure.

In addition to the preparation of neo-glycoproteins, the present spacer-arm-glycosides are well suited for coupling to particles (e.g. glass, lates, silica gel and cross-linked polysaccharides) which could be used

for affinity chromatographic purification of lectin-like materials and other molecules with carbohydrate specificity. Furthermore, small, non-sedimenting, particles with co-valently bound oligosaccharides should find applications as stable and well+defined substitutes for erythrocytes in agglutination studies with antibodies and bacteria.

Preparation of simple glycosides, suitable as agglutination inhibitors

Evaluation of carbohydrate-specific agglutination reactions is performed by the use of well-defined inhibitors that in a more or less specific way can prevent the formation of the agglutination precipitate or even dissolve it after its formation. The inhibitors normally used are either free mono- or oligosaccharides or simple (e.g. methyl and ethyl) glycosides. The latter have the added advantage of a well-defined anomeric configuration.

The 2-bromoethyl glycosides of this invention make it possible to prepare ethyl glycosides (by a simple hydrogenolysis reaction) from the same intermediates as were used for the preparation of the complete spacer-arm-glycosides. In this way one avoids otherwise necessary doubling of the total synthetic effort. Radioactively labeled glycosides can be prepared by simply exchanging the hydrogen gas by tritium.

Replacement of the functionalized thiols that were used for spacer-arm synthesis by di-thiols makes it possible to prepare bi-dentate inhibitor glycosides, and tri-, tetra- etc. thiols should give tri-, tetra- etc. dentate inhibitors by analogous procedures. Variation of the di-thiol chainlength makes it possible to prepare a series of inhibitors where the distance between the sugar residues can be varied almost at will. For the preparation of inhibitors with very long interconnecting chains, it is possible to change the ratio between the 2-bromoethyl glycoside and the di-thiol so that mainly the intermediate, thiol-terminated spacer-arm glycoside is formed in the reaction (see Examples). Standard oxidative di-sulphide formation[19] can then be used for the formation of the bi-dentate inhibitor compound. The different types of inhibitor glycosides, that have been prepared from 2-bromoethyl glycosides, are shown in Figure 5.

The 2-bromoethyl group in standard carbohydrate synthetic chemistry.

As shown above, the 2-bromoethyl glycosides are very versatile compounds that can be used for the preparation of many kinds of biologically interesting carbohydrate derivatives. The 2-bromoethyl group is also compatible with most of the synthetic reactions that are used in carbohydrate chemistry. As an illustration, we include here a total synthesis (Figure 6) of the H blood-group specific disaccharide in the 2-bromoethyl β-glycoside form (*39*) and its further coupling to BSA (*44*). All these reactions can be performed without any unwanted interference from the bromoethyl aglycon. However, the hydrogenolytic cleavage of the benzyl and benzylidene groups deserves some additional comments: In the preparation of the ethyl glycosides (Figure 5), the bromine atom was cleaved off by hydrogen under *basic* conditions whereas the hydrogenolysis reaction shown in Figure 6 formed in acetic acid; *acidic* hydrogenolysis conditions obviously leaves the 2-bromoethyl group intact.

The 2-bromoethyl glycoside *40* was reacted with methyl 3-mercaptopropionate (cf Table 4 and Examples) to give the spacer-arm glycoside *41*.

This compound was finally coupled (*via* the hydrazide *43*) to bovine serum albumin to give the complete semi-synthetic antigen *44*.

The invention will be further illustrated by specific examples of a non-limiting character. In these examples melting points were recorded on a calibrated Kofler hot stage or on a Reichert melting point microscope. Optical rotations were recorded on a Perkin Elmer 241 Polarimeter. NMR spectra were recorded on a Varian XL—200 spectrometer. Solvent removal was done on a rotary evaporator followed by oil-pump evaporation below 13.3 Pa.

Examples 1—14

Preparation of 2-bromoethyl glycosides (cf. Figure 2 and Table 1)

Reactions were monitored by thin layer chromatography (TLC) using the technique for selective visualization[6] of sugar per-O-acetates and glycosides. Yields, melting points and optical rotations for the 2-bromoethyl glycosides (*1—14*) are shown in Table 1 and $^{13}C$— and $^{1}H$—HMR spectra are shown in Table 2 and 3, respectively.

*Method A* (cf. ref 19)

The sugar per-O-acetate (5 mmol) and 2-bromoethanol (6 mmol) were dissolved in dry methylene chloride (10 ml) and cooled (ice bath). Borontrifluoride etherate (25 mmol) was added dropwise (ca 15 min). The ice bath was removed after 1 h and the reaction was continued at room temperature until the starting material had been consumed (TLC; 2—20 h).

Glycosides with the 1,2-*trans* configuration are formed first. At the end of the reaction period, a small amount of the 1,2-*cis* glycoside is usually formed, probably by an anomerisation reaction[7]. In addition, a long reaction time normally gives minor amounts of deacetylated compounds. The formation of these side products can be minimized by a careful monitoring of the reaction.

The reaction mixture was poured into ice water (10 ml) and the phases were separated. The water phase was extracted with methylene chloride (5 ml) and the combined methylene chloride solutions were washed (water, sodium hydrogen carbonate solution, water) and dried (sodium sulfate). Filtration and

evaporation gave a syrup that was dissolved in warm ether or ethyl acetate. Addition of petroleum ether or iso-octane gave the crystalline (where applicable; see Table 1) per-O-acetylated 2-bromoethyl glycoside. Yield: see Table 1. Preparative chromatography of the mother liquor, raised the yield considerably.

*Method B*

2-Bromoethyl 2,3,6-tri-O-acetyl-4-O-(2,3,4,6,-tetra-O-acetyl-α-D-galactopyranosyl)-β-D-galactopyranoside (14)

A solution of hydrogen bromide in acetic acid (45%, 30 ml) was added dropwise (ca 25 min) to a solution of 1,2,3,6-tetra-*O*-acetyl-4-O-(2,3,4,6-tetra-*O*-acetyl-α-D-galactopyranosyl)-α-D-galactopyranose (20.0 g; 29.5 mmol) in acetic acid/acetic anhydride (2/1; 20 ml) at 0°C. The reaction mixture was kept at 0°C for 4 h and then at room temperature for 1.5 h and finally it was diluted with methylene chloride (400 ml) and poured into an ice cold, saturated sodium hydrogen carbonate solution (400 ml). The aqueous phase was extracted with methylene chloride (50 ml) and the combined extracts were washed with another portion of bicarbonate solution (300 ml) and dried (sodium sulfate). Filtration and evaporation gave *1-bromo-2,3,6-tri-O-acetyl-4-O-(2,3,4,6-tetra-O-acetyl-α-D-galactopyranosyl)-α-D-galactopyranose* (20.8 g; pure by TLC) as a crystalline mass. Recrystallization from ethyl acetate-ether gave an analytical sample; Mg 97—99°; $[\alpha]_D^{21}$+219° (c 0.8; $CHCl_3$); $^1$H—NMR ($CDCl_3$; TMS) δ 6.73 (d, 1H, $J_{1,2}$=4Hz; H1), 5.55 (dd, 1H, J=1.5Hz, J=3Hz, H4′), 5.36 (dd, 1H, $J_{2',3'}$=11Hz, $J_{3',4'}$=3Hz; H3′), 5.29 (dd, 1H, $J_{2,3}$=11Hz, $J_{3,4}$=2.5Hz; H3), 5.25 (dd, 1H, $J_{1',2'}$=3.5Hz, $3_{2',3',}$=11Hz; H2′) 5.10 (dd, 1H, $J_{1,2}$=4Hz, $J_{2,3}$=11Hz, H2), 5.01 (d, 1H, $J_{1',2'}$=3.5 Hz; H1′), 4.49 (dt, 1H, $J_{4',5'}$=1.5Hz, $J_{5',6'}$=7Hz, H5′), 4.40—4.05 (6H), 2.15, 2.13, 2.12, 2.12, 2.09, 2.03, 2.00 (s, 3H each, $CH_3CO$) ppm. $^{13}$C—NMR ($CDCl_3$, TMS) δ 170.4, 170.3, 170.2, 170.1, 169.9, 169.7 (CO), 99.1 (C1′), 88.7 (C1), 76.3, 72.7, 69.9, 68.0, 67.7, 67.6, 67.3, 67.2 (*C*HOAc), 61.5, 60.7 (C6, C6′), 20.9, 20.7, 20.6 (*C*$H_3$—CO) ppm.

A solution of the crude acetobromo sugar (20.5 g; 29.3 mmol) in dry methylene chloride (100 ml) was added dropwise (ca 20 min) to a cold (−78°) solution of silver trifluoromethanesulfonate (nonpurified, commercial material; 10.3 g; 40 mmol), 2,4,6-trimethylpyridine (3.6 g; 30 mmol) and 2-bromoethanol (17.5 g; 140 mmol) in methylene chloride (250 ml) under nitrogen. The reaction mixture was stirred in the dark for 23 h during which time it had reached room temperature. The yellowish-white precipitate, that had formed, was removed and the resulting colorless solution was washed consecutively with 1M hydrochloric acid, water, saturated sodium hydrogen carbonate solution and water. Drying (sodium sulfate), filtration and evaporation gave a residue which crystallized spontaneously. Recrystallization from ethyl acetate, followed by evaporation of the mother liquor and recrystallization from methanol, gave a total of 16.6 g (77%) of the title compound *14*. Melting point and optical rotation are shown in Table 1 and the $^{13}$C— and $^1$H—NMR data are shown in Table 2 and 3 respectively.

*Method C*

2-Bromoethyl 3,4,6-tri-O-acetyl-2-acetamido-2-deoxy-β-D-glucopyranoside (*8*)

Per-O-acetylated glucose oxazoline[37] (see Figure 2; 4.62 g; 14.0 mmol), 2-bromoethanol (8.49 g; 67.9 mmol) and p-toluenesulfonic acid (81 mg) were dissolved in toluene and nitromethane (1/1; 80 ml). The solution was heated (110°) for ca 10 minutes and cooled. A TLC-analysis ($SiO_2$, toluene/ether/methanol 7/7/1) showed that all of the starting oxazoline had been consumed. Pyridine (20 drops) was added and the reaction mixture was evaporated to dryness. Preparative chromatography of the residue gave the title compound (*8*). Yield, melting point and optical rotation are shown in Table 1 and $^{13}$C— and $^1$H—NMR data are shown in Table 2 and 3 respectively.

*Method D*

2-Bromoethyl 2,3,4,6-tetra-O-benzyl-α-D-galactopyranoside (2)

Trimethylsilyl bromide (7.96 g, 6.78 ml, 52 mmol) was added with a syringe to a mixture of 2,3,4,6-tetra-*O*-benzyl-D-galactopyranose (28 g, 52 mmol), 2-bromoethanol (4.46 ml, 40.0 mmol), cobalt(II)bromide (11.4 g, 52.0 mmol), tetraethylammonium bromide (10.9 g, 52.0 mmol) and molecular seives (4Å, 41 g) in 138 ml of methylene chloride under nitrogen with protection from light. (The cobalt(II)chloride had been dried at 180°, 5×$10^{-3}$ mm Hg over night.) The reaction mixture was stirred at room temperature over night and filtered. The filtrum was washed with 100 ml of methylene chloride. The filtrate was evaporated at reduced measure to give 25.8 g of the crude product, which was chromatographed ($SiO_2$, iso-octane:ethylacetate 3:2). Fraction 1 was the pure title compound (6.0 g) and fraction 2 was a mixture of the anomers (α/β 10.0 g). Total yield of glycosides: 16 g (48%). (This procedure is a slight modification of that of Koto et al[23]. $[\alpha]_D^{23}$= +30.5° (c 1.4, $CHCl_3$). $^1$H—NMR ($CDCl_3$, TMS) δ 7.30 (m, 20H, Ph), 4.97—4.42 (m, 9H), 4.0—3.8 (m, 6H), 3.52 (t, 4H, *inter alia* $CH_2$Br).

$^{13}$C—NMR($CDCl_3$, TMS) δ 103.3 (d, C1, $J_{C-H}$=168Hz); 84.2, 81.6, 80.2, 75.0 (C2—C5); 80.0, 78.7, 78.6, 78.5, 74.2, 73.5 (C6, benzylic $CH_2$ and aglycon O—$CH_2$), 35.5 ($CH_2$Br).

*Method E*

2-Bromoethyl 3,4,6-tri-O-acetyl 2-azido-2-deoxy-α-D-galactopyranoside (11)

From a mixture of benzene (30 ml) and nitromethane (30 ml) 30 ml of the solvent mixture was distilled off. The temperature of the remaining solvent mixture was adjusted to 60°C, whereafter 2-bromoethanol (0.355 ml, 5.00 mmol) was added followed by mercuric cyanide (1.26 g, 5.00 mmol) and 1-chloro-3,4,6-tri-O-acetyl-2-azido-2-deoxy-β-D-galactopyranose[21] (1.74 g, 4.98 mmol) dissolved in a few ml of benzene. The reaction mixture was stirred at 60°C for 3 days, 100 ml of methylene chloride was then added and the organic solution was washed with water (3×25 ml). After drying ($Na_2SO_4$) and removal of the solvent *in vacuo* the residue, 1.90 g, was chromatographed ($SiO_2$, isooctane: ethylacetate 2:1) to give 1-chloro-3,4,6-tri-*O*-acetyl-2-azido-2-deoxy-α-D-galactopyranose (350 mg) and a second fraction (340 mg) containing both anomers of *11*. This fraction was chromatographed ($SiO_2$, 1,1,2-trichlorotrifluoroethane:ether 55:15) to give the pure title compound *11* (165 mg, 8%) as a syrup. Optical rotation is shown in Table 1 and the $^1H$— and $^{13}C$—NMR data are shown in Table 3 and 2 respectively.

Examples 15—23

Preparation of complete spacer-arm glycosides (cf. Table 4)

Compounds *15, 16, 17, 18* and *19* were prepared by method F and compounds *20, 21, 22* and *23* by method G; *vide infra.*

*Method F*

2-(2-Methoxycarbonylethylthio)ethyl 2,3,4,6-tetra-O-acetyl-β-D-galactopyranoside (15)

2-Bromoethyl 2,3,4,6-tetra-*O*-acetyl-β-D-galactopyranoside (*1*) (1.76 g; 3.90 mmol), methyl 3-mercaptopropionate (1.0 ml; 9.2 mmol), methyltrioctylammonium chloride (ca 10 mg) and sodium hydroxide (0.36 g; 9.0 mmol) were dissolved in benzene (5 ml) and water (5 ml). The reaction mixture was stirred vigorously at room temperature. The reaction was half-way after ca 40 minutes (TLC: $SiO_2$, ethyl acetate/isooctane 2/1) but after this time it slowed down considerably. The reaction was continued over night which gave an almost complete consumption of the starting material. It should be noted that no deacetylation occurred under these reaction conditions. The aqueous phase was extracted with benzene and the combined benzene solutions were washed once with water. Drying, evaporation and chromatography ($SiO_2$, ethyl acetate/isooctane 1/1) gave the title compound (*15*) as a syrup that crystallized slowly. Yield: 1.41 g (73%). Recrystallization from abs. ethanol gave an analytical sample: mp 81—82°; $[\alpha]_D^{24}-10.5°$ (c 1.0; $CDCl_3$); $^1H$—NMR ($CDCl_3$, TMS) δ 5.42 (d, 1H, $J_{3,4}$=3.5Hz, H4), 5.24 (dd, 1H, $J_{1,2}$=7.8 Hz, $J_{2,3}$=10.5Hz, H2), 5.05 (dd, 1H, H3), 4.54 (d, 1H, H1), 3.65—4.26 (m, 5H, H5, H6, O—$CH_2$—$CH_2$), 3.72 (s, 3H, $CH_3$), 2.59—2.88 (m, 6H, S—$CH_2$, $CH_2$—CO), 2.17, 2.09, 2.06, 2.00 (s, 3H each, $CH_3CO$) ppm.

$^{13}C$—NMR ($CDCl_3$, TMS) δ 172.2, 170.4, 170.2, 170.1, 169.5 (CO), 101.3 (C1), 70.9, 70.8, 69.6, 68.7, 67.0 (C2—C5, O—$CH_2$), 61.3 (C6), 51.8 ($OCH_3$), 34.7, 31.5, 27.4 ($CH_2$—S, $CH_2CO$), 20.8, 20.7, 20.6 ($CH_3CO$) ppm.

2-(2-Methoxycarbonylethylthio)ethyl 2,3,6-tri-O-acetyl-4-O-(2,3,4,6-tetra-O-acetyl-α-D-galactopyranosyl)-β-D-galactopyranoside (18)

2-Bromoethyl 2,3,6-tri-*O*-acetyl-4-*O*-(2,3,4,6-tetra-*O*-acetyl-α-D-galactopyranosyl)-β-D-galactopyranoside (*14*) (251 mg; 0.338 mmol), methyl 3-mercaptopropionate (39 µl; 0.35 mmol) methyltrioctylammonium chloride (ca 10 mg) and cesium carbonate (114 mg; 0.35 mmol) were dissolved in benzene (0.5 ml) and water (0.5 ml). The reaction mixture was stirred (magnet) over night and analyzed by TLC ($SiO_2$, ethyl acetate/isoctane 4/1; reaction 2/3 completed). Methyl 3-mercaptopropionate (ca 20 µl) was added and the reaction mixture was stirred for 24 h (TLC showed complete reaction). The phases were separated, the water phase was extracted twice with methylene chloride and the combined organic phases were dried (sodium sulfate) which gave a yellow to green solution. Evaporation gave a syrup which was chromatographed ($SiO_2$, ethyl acetate/isooctane 2/1) to give the title compound (*18*) as a syrup. Yield 211 mg (80%). $[\alpha]_D^{23}+70.0°$ (c 0.6; $CHCl_3$); $^1H$—NMR ($CDCl_3$, TMS) δ 5.57 (d, 1H, $J_{3',4'}$=3.0Hz, H4'), 5.39 (dd, 1H, $J_{2',3'}$=11.0Hz, H2'), 5.19 (dd, 1H, H3'), 5.17 (dd, 1H, $J_{2,3}$=11Hz, H2), 5.00 (d, 1H, $J_{1',2'}$=4.0Hz, H1'), 4.80 (dd, 1H, $J_{3,4}$=3.0Hz, H3), 4.51 (d, 1H, $J_{1,2}$=8.0Hz, H1), 3.70 (S, 3H, $CH_3$—O), 2.82, 2.75, 2.63 (t, 2H, each, J=7Hz, $CH_2$—S, $CH_2$—CO), 2.13, 2.11, 2.08, 2.08, 2.06, 2.04, 1.98 (s, 3H, each, $CH_3CO$) ppm.

$^{13}C$—NMR ($CDCl_3$, TMS) δ 172.3 ($COOCH_3$), 170.7, 170.6, 170.5, 170.4, 170.1, 169.8, 169.2 ($CH_3CO$), 101.1 (C1), 99.4 (C1'), 77.1, 72.7, 72.0, 68.6, 68.5, 67.9, 67.4, 67.1 (C2—C5, C2'—C5'), 69.5 ($OCH_2$), 62.0, 60.5 (C6, C6'), 51.8 ($COOCH_3$), 34.7, 31.4, 27.4 ($CH_2S$, $CH_2$—CO), 21.0, 20.7, ($CH_3CO$) ppm.

2-(p-Aminophenylthio)ethyl 2,3,4,6-tetra-O-acetyl-β-D-galactopyranoside (16)

[i]2-Bromoethyl 2,3,4,6-tetra-*O*-acetyl-β-D-galactopyranoside (*1*) (2.05 g; 4.50 mmol), p-aminothiophenol (1.2 g; 9.6 mmol), methyltrioctylammonium chloride (20 mg) and sodium hydroxide (0.3 g; 7.5 mmol) were dissolved in benzene (5 ml) and water (5 ml). The reaction mixture was stirred vigorously for 9h (TLC: $SiO_2$, ethyl actate/isooctane 3/1) and worked up as above. Chromatography ($SiO_2$, ethyl acetate/isooctane 2/1) gave the title compound as an amorphous solid. yield: 1.6 g (71%). $[\alpha]_D^{21}+7.7°$ (c 1.0; $CHCl_3$); $^1H$—NMR ($CDCl_3$, TMS) δ 7.25, 6.63 (Abq, 4H, $J_{AB}$=8.6Hz, aromatic H), 5.39 (d, 1H, $J_{3,4}$=3.5Hz, H4), 5.22 (dd, 1H, $J_{1,2}$=7.9Hz, $J_{2,3}$=10.4Hz, H2), 5.01 (dd, 1H, H3), 4.45 (d, 1H, H1), 2.92 (t, 1H, J=6.8Hz, S—$CH_2$), 2.15, 2.08, 2.05, 2.00 (s, 3H each, $CH_3CO$) ppm.

$^{13}C$—NMR($CDCl_3$, TMS) δ 170.4, 170.3, 170.2, 169.5 (CO), 146.4, 134.5, 122.0, 115.6 (aromatic C), 101.5 (C1), 70.9, 70.6, 68.8, 68.7, 67.0 (C2—C5, 0—$CH_2$), 61.2 (C6), 35.7 (S—$CH_2$), 20.9, 20.7, 20.6 ($CH_3$—CO) ppm.

[ii]2-(p-Nitrophenylthio)ethyl 2,3,4,6-tetra-*O*-acetyl-β-D-galactopyranoside (*21*) (90 mg; 0.18 mmol) was hydrogenated over night (4 atm $H_2$; Pd/C 10%, 90 mg) in 75 ml of methanol which gave the title compound (*16*) in 58% yield after chromatography as above.

2-(Octylthio)ethyl 2,3,4,6-tetra-O-acetyl-β-D-galactopyranoside (17)

2-Bromoethyl 2,3,4,6-tetra-*O*-acetyl-β-D-galactopyranoside (*1*) (2.0 g; 4.4 mmol), octanethiol (0.7 g; 4.8 mmol), methyltrioctylammonium chloride (20 mg) and sodium hydroxide (0.3 g; 7.5 mmol) were dissolved in benzene (9 ml) and water (8 ml). The reaction mixture was stirred vigorously for 3 h (TLC: $SiO_2$, ethyl acetate/isooctane 3/1) and worked up as for compound *15* above. Chromatography ($SiO_2$, ethyl acetate/isooctane 2/1) gave the title compound (*17*) as a colourless oil. Yield: 1.6 g (70%). $[\alpha]_D^{21}-8.6°$ (c 1.2; $CHCl_3$); $^1H$—NMR ($CDCl_3$, TMS) δ 5.40 (dd, 1H, $J_{4,5}$=1.0Hz, H4), 5.23 (dd, 1H, $J_{2,3}$=10.5Hz, H2), 5.03 (dd, 1H, $J_{3,4}$=3.5Hz, H3), 4.52 (d, 1H, $J_{1,2}$=8.0Hz, H1), 2.71, 2.53 (t, 2H, each, J=7.0Hz, S—$CH_2$), 2.16, 2.08, 2.06, 1.99 (s, 3H, each, $CH_3CO$), 0.88 (t, 3H, J=7.0Hz, $CH_3$—$CH_2$) ppm.

$^{13}C$—NMR ($CDCl_3$, TMS) δ 170.4, 170.2, 170.1, 169.4 (CO), 101.4 (Cl), 70.9, 70.7, 68.7, 67.0 (C2—C5), 69.8 ($CH_2$—O), 61.3 (C6), 32.6, 31.8, 31.4, 29.8, 29.2, 28.8, 22.7 ($CH_2$), 20.8, 20.7, 20.6 ($CH_3CO$) 14.1 ($CH_3$—$CH_2$) ppm.

2-(Octylthio)ethyl 2,3,6-tri-O-acetyl-4-O-(2,3,4,6-tetra-O-acetyl-α-D-galactopyranosyl)-β-D-galactopyranoside (19)

Compound *14* (484 mg; 0.65 mmol), octanethiol (110 mg; 0.75 mmol; 130 µl), methyltrioctylammonium chloride (ca 10 mg) and cesium carbonate (245 mg; 0.75 mmol) were dissolved in benzene (1 ml) and water (1 ml). The reaction mixture was treated as for compound *17*. Chromatography gave the title compound (*19*) (250 mg) and starting compound *14* (160 mg). Yield (based on consumed *14*): 71%; $[\alpha]_D^{23}+65.3°$ (c=1.4; $CHCl_3$); $^1N$—NMR($CDCl_3$, TMS) δ 5.57 (dd, 1H, $J_{4',5'}$= 1Hz; H4', 5.39 (dd, 1H, $J_{2',3'}$=11Hz, $J_{3',4'}$=3.5Hz; H3'), 5.20 (dd, 1H, $J_{1',2'}$=3.5Hz; H2'), 5.19 (dd, 1H, $J_{2,3}$=11Hz; $J_{1,2}$=7.5Hz; H2), 5.01 (d, 1H, $J_{1',2'}$=3.5Hz; H1'), 4.81 (dd, 1H, $J_{3,4}$=3Hz; H3), 4.51 (d, 1H, $J_{1,2}$=7.5Hz; H1), 2.73, 2.53 (t, 2H, each, J=7Hz; $CH_2$—S), 2.13, 2.11, 2.08, 2.08, 2.07, 2.04, 1.99 (s, 3H each; $CH_3$ —CO) ppm.

$^{13}C$—NMR($CDCl_3$, TMS) δ 170.7, 170.6, 170.5, 170.4, 170.1, 169.8, 169.1 (CO), 101.2 (C1), 99.4 (C1'), 77.1, 72.7, 71.9, 68.6, 68.6, 67.8, 67.4, 67.1 (C2—C5, C2'—C5'), 69.6 (O—$CH_2$—$CH_2$—S), 62.0, 60.5 (C6, C6'), 32.6, 31.8, 31.3, 29.8, 29.2, 28.8, 22.7 (aglycon—$CH_2$), 21.0, 20.8, 20.7 ($CH_3$—CO), 14.1 ($CH_3$—$CH_2$) ppm.

*Method G*

2-(Octadecylthio)ethyl 2,3,6-tri-O-acetyl-4-O-(2,3,4,6-tetra-O-acetyl-α-D-galactopyranosyl)-β-D-galactopyranoside (20)

Compound *14* (743 mg; 1 mmol), octadecylthiol (315 mg; 1.1 mmol) and cesium carbonate (391 mg; 1.2 mmol) were added to dry dimethylformamide (8 ml) at room temperature under nitrogen. The reaction was followed by TLC. After 24 h, the reaction mixture was distributed between water and ether. The ether phase was dried ($Na_2SO_4$) and evaporated. The residue was chromatographed (ethyl acetate/isooctane 1/1) to give the title compound (*20*) (300 mg) and the starting compound (*14*) (400 mg). Yield (based on consumed *14*): 69% $[\alpha]_D^{23}+54.7°$ (c 0.5, $CHCl_3$); $^1H$—NMR ($CDCl_3$, TMS): the main part of the spectrum (1.9—5.6 ppm) was practically identical with that of compound 19.

$^{13}C$—NMR($CDCl_3$, TMs) δ 170.6, 170.5, 170.4, 170.4, 170.1, 169,7, 169.1 (CO), 101.2 (C1), 99.4 (C1'), 77.1, 72.6, 71.9, 68.5, 68.5, 67.8, 67.8, 67.3, 67.0 (C2—C5, C2'—C5'), 69.5 (O—$CH_2$—$CH_2$—S), 62.0, 60.4 (C6, C6'), 32.6, 31.3, 29.8, 29.6, 29.5, 29.3, 29.2, 28.8, 22.6 (aglycon—$CH_2$), 20.9, 20.7, 20.7, 20.6, 20.6, ($CH_3$—CO), 14.1 ($CH_3$—$CH_2$) ppm.

2-(p-Nitrophenylthio)ethyl 2,3,4,6-tetra-O-acetyl-β-D-galactopyranoside (21)

p-Nitrothiophenol (50 mg; 0.32 mmol) was added to a suspension of sodium hydride (oil-free; 11 mg; 0.46 mmol) in dimethylformamide (3 ml) at room temperature and with stirring. 2-Bromoethyl 2,3,4,6-tetra-*O*-acetyl-β-D-galactopyranoside (1) (110 mg; 0.24 mmol) in dimethylformamide (3 ml) was added during 10 min to the thiophenol solution and the reaction mixture was stirred for another 75 min (TLC: $SiO_2$, ethyl acetate/isooctane 1/1). The reaction mixture was poured into ice-cold water (20 ml) and ether (40 ml). The aqueous phase was extracted further with ether (40 ml) and the combined organic phases were washed with cold water (20 ml) and dried (sodium sulfate). Evaporation and chromatography ($SiO_2$, ethyl acetate/isooctane 1/1) gave the title compound as a light yellow oil. Yield: 80 mg (62%). $[\alpha]_D^{23}-15.2$ (c 1.1; $CDCl_3$); $^1H$—NMR ($CDCl_3$, TMS) δ 8.14, 7.36 (ABq, 4H, $J_{AB}$=9.2Hz, aromatic H), 5.40 (dd, 1H, $J_{4,5}$=1.0Hz,H4), 5.22 (dd, 1H, $J_{2,3}$=10.5Hz, H2), 5.01 (dd, 1H, $J_{3,4}$=3.4Hz, H3), 4.52 (d, 1H, $J_{1,2}$=7,8Hz, H1), 3.25/t, 2H, J=6.5Hz, S—$CH_2$), 2.17, 2.05, 2.04, 1.99 (S, 3H, each, $CH_3CO$) ppm,

$^{13}C$—NMR ($CDCl_3$, TMS) δ 170.4, 170.2, 170.1, 169.4 (CO), 146.6, 145.3, 126.5, 124.1 (aromatic C), 101.4 (C1), 70.9, 70.8, 68.5, 66.9 (C2—C5), 67.9 ($CH_2$—O), 61.2 (C6), 31.8 (S—$CH_2$), 20.8, 20.7, 20.6 ($CH_3CO$) ppm.

2-(p-Aminophenylthio)ethyl 4-O-(β-D-galactopyranosyl)-β-D-galactopyranoside (22)

p-Aminothiophenyl (205 mg; 1.64 mmol) was dissolved in dimethylformamide (4 ml). Sodium hydride (70 mg; 2.9 mmol) was added and the mixture was stirred under nitrogen for 20 Min at room temperature. 2-Bromoethyl 2,3,6-tri-*O*-acetyl-4-*O*-(2,3,4,6-tetra-*O*-acetyl-β-D-galactopyranosyl)-β-D-galactopyranoside *14* (640 mg; 0.86 mmol) in dimethylformamide (7 ml) was added and the reaction mixture was stirred under nitrogen for 2 h (TLC:$SiO_2$, ethyl acetate/isooctane 3/1) and poured into cold water (40 ml). The water solution was extracted with ether (4×20 ml) and the combined ether solutions were washed with water (10 ml) and dried (sodium sulfate). Evaporation gave a residue that contained a series of partly de-

acetylated compounds. Evaportion of the water solution gave an additional amount of partly deacetylated material. The combined residues were de-acetylated (sodium methoxide/methanol) and the product (after evaporation) was filtered through a column ($SiO_2$, ethyl acetate/methanol 2/3) which gave a crystalline (needles) residue). Yield: 425 mg (99%). Recrystallization from methanol gave an analytical sample: mp 104—106°; $[\alpha]_D^{23}$−0.8 (c 1.1; $H_2O$); $^1H$—NMR (DMSO—D6 plus $D_2O$, 50°C, TMS) δ 7.13, 6.58 (d, 2H each, J=8.5Hz, aromatic H), 4.28 (d, 1H, J=7.0Hz, H1'), 4.28 (d, 1H, J=7.5Hz, H1), 2.90 (t, 2H, J=7.0Hz, $CH_2$—S) ppm.

$^{13}C$—NMR(DMSO—D6 plus $D_2O$) δ 149.2, 136.6, 124.7, 119.7 (aromatic C), 107.0, 105.5 (C1, C1'). 79.7, 77.8, 76.9, 75.9, 75.5, 74.2, 73.9, 71.4, 70.7 (C2—C5, C2'—C5', $CH_2O$), 63.8, 63.1 (C6, C6'), 37.6 ($CH_2$—S) ppm.

2-(p-Aminophenylthio)ethyl 4-O-(α-D-galactopyranosyl)-β-D-galactopyranoside (23)

2-Bromoethyl 2,3,6-tri-*O*-acetyl-4-*O*-(2,3,4,6-tetra-*O*-acetyl-α-D-galactopyranosyl)-β-D-galactopyranoside (*14*) (170 mg; 0.23 mmol) was treated as above (compound *22*). The crude product, that was obtained after the de-acetylation reaction, was filtered through a column ($SiO_2$, ethyl acetate/methanol 2/3) which gave the title compound (*23*) as an amorphous solid. Yield: 110 mg (97%). Chromatography ($SiO_2$, chloroform/methanol/water 65/35/10; lower phase) gave an analytical sample: $[\alpha]_D^{23}$+70.6° (c 0.58; $H_2O$); $^1H$—NMR (DMSO—D6 plus $D_2O$ 50°) δ 7.13, 6.56 (d, 2H, each, J=8.4Hz, aromatic H), 4.83 (d, 1H, J=3.3Hz, H1'), 4.14 (d, 1H, J=7.6Hz, H1), 2.89 (t, 2H, J=7.5Hz, $CH_2$—S) ppm.

$^{13}C$—NMR(DMSO—D6 plus $D_2O$) δ 149.1, 136.5, 124.8, 119.7 (aromatic C), 105.7 (C1), 102.9 (C1'), 79.8, 77.7, 75.1, 73.6, 73.5, 71.9, 71.7, 71.4, 70.8 (C2—C5, C2'—C5', $CH_2O$), 63.3, 62.8 (C6, C6'), 37.6 ($CH_2Br$) ppm.

## Examples 24—27

### Preparation of complete neo-glycoconjugates (cf. Figure 4)

The two spacer-arm glycosides *24* and *23*, having terminal ester and amino groups respectively, were coupled to protein by well established procedures 14, 15, 17.

2-(2-Methoxycarbonylethylthio)ethyl β-D-galactopyranoside (24)

2-(2-Methoxycarbonyl-ethylthio)ethyl 2,3,4,6-tetra-*O*-acetyl-β-D-galactopyranoside (15) (1.00 g; 2.32 mmol) was dissolved in 25 ml of dry methanol and added to a solution of sodium methoxide in methanol, prepared from sodium (~0.05 g) and 10 ml of methanol. Stirring was continued for 4 h after which the reaction mixture was neutralized with Duolite®—$H^+$ (methanol washed and dried). Filtration and evaporation of the solvent gave a quantitative yield of the title compound (590 mg) which was pure according to TLC ($SiO_2$, chloroform/methanol/water 65/35/10) and NMR ($D_2O$, TMS as an external standard): δ 4.44 (d, 1H, H1, $J_{1,2}$ 7.62Hz) 4.08 (m, 1H, $OCH_2$—$CH_2$), 3.72 (s, 3H, $OCH_3$), 3.52 (dd, 1H, H2), 2.92—2.70 (m, 6H, $CH_2S$, $CH_2CO$).

$^{13}C$ NMR ($D_2O$, TMS as an external standard) δ 178.2 (CO), 105.8 (C—1), 78.1, 75.6, 73.6, 71.5 (C2—C5), 71.7 (C—6), 63.8 (O—$CH_2$ of aglycon), 55.2 ($OCH_3$), 37.1, 33.7, 29.3 (—$CH_2$ of aglycon).

2-(2-Hydrazinocarbonylethylthio)ethyl β-D-Galactopyranoside (25)

2-2(-Methoxycarbonyl-ethylthio)ethyl β-D-galactopyranoside (*24* (500 mg) was dissolved in ethanol (99.5%; 2.5 ml) and hydrazine hydrate (85%; 1 ml) was added. The reaction mixture was left at room temperature over night (TLC: $SiO_2$, chloroform/methanol/water 65/35/10, lower phase). Evaporation gave the title compound (*25*) as a syrup (pure by TLC and $^1H$—NMR) in quantitative yield. Recrystallization from ethanol gave the pure compound; mp 178—179°; $[\alpha]_D^{23}$ −2.2° (c 0.5; $H_2O$); $^1H$—NMR ($D_2O$ TMS as an external standard) δ 4.43 (d, 1H, H1, $J_{1,2}$ 7.7Hz), 4.07 (m, 1H, O—$CH_2$—$CH_2$), 3.92—3.60 (m, 7H), 3.50 (dd, 1H, H2, $J_{2,3}$ 11.6Hz), 2.90, 2.86 (t, 2H each, —$CH_2S$, J≅7Hz), 2.54 (t, 2H, —$CH_2CO$). $^{13}C$—NMR ($D_2O$, TMS as external standard) δ 176.1 (C=O), 105.6 (C—1), 77.9, 75.4, 73.4, 71.6, 71.3 (C—2——C—6), 63.7 (O—$CH_2$—$CH_2$), 36.5, 33.5, 29.9 ($CH_2S$, $CH_2CO$).

Bovine serium albumin conjugate 26

The mono saccharide *25* was coupled to BSA using the acyl azide method as described for the preparation of the blood group H-disaccharide-BSA conjugate (*44*). The incorporation of the hapten was 36 mol per mol of BSA as determined by both differential sulfur elemental analysis and by the phenol-sulphuric acid method[16].

KLH conjugate 27

The disaccharide *23* was coupled to KLH using the procedure described in Ref. 17b (the thiophosgene method). The incorporation of the hapten was 490 mol per mol of KLH as determined by the phenol-sulphuric acid method[16].

## Examples 28—33

### Preparation of agglutination inhibitors (cf Figure 5)

Ethyl glycosides were prepared from the corresponding 2-bromoethyl glycoside by a hydrogenolytic cleavage of the carbon-bromine bond. The bidentate glycoside inhibitors were prepared by alkylation of di-thiols by a method analogous to the one used for the preparation of the complete spacer-arm glycosides

(Method F, *vide supra*).

Ethyl 2,3,4,6-tetra-O-acetyl-β-D-galactopyranoside (28)

2-Bromoethyl 2,3,4,6-tetra-*O*-acetyl-β-D-galactopyranoside (*1*) (98 mg; 0.22 mmol) was dissolved in methanol (5 ml) and aqueous potassium hydroxide (0.3 M, 5 ml) and hydrogenated (Pd/C 10%; 47 mg) at atmospheric pressure for 3 h. The reaction mixture (pH 6) was filtered and evaporated to give a residue that was pure by TLC ($SiO_2$, chloroform/methanol/water 65/35/10, lower phase). The residue was acetylated (acetic anhydride/pyridine 1/1; 60°; 1.5 h) and the reaction mixture was cooled, filtered and evaporated with toluene and ethanol several times and finally dried (0.1 torr). Crystallization from ether/isooctane gave the title compound (*28*). Yield: 55 mg (68%). Mp 92—93°; $[\alpha]_D^{22}$ −31.0° (c 1.2; benzene); $[\alpha]_D^{24}$ −14.4° (c 1.2; $CDCl_3$) [Litt[39] mp 88°; $[\alpha]_D^{20}$ −29.8° (c 10; benzene)]; $^1$H—NMR ($CDCl_3$, TMS) δ 5.40 (dd, 1H, $J_{4,5}$=1.0Hz, $J_{4,3}$=3.5Hz, H4), 5.22 (dd, 1H, $J_{2,3}$=10.5Hz, $J_{1,2}$=8.0Hz, H2), 5.02 (dd, 1H, H3), 4.48 (d, 1H, H1), 4.16 (m, 2H, H6), 3.93 (oct, 1H, $J_{gem}$=10.0Hz, $J_{vic}$=7.0Hz, $OCH_2CH_3$), 3.89 (dd, 1H, $J_{5,6}$≅4Hz, H5), 3.60 (oct, 1H, $OCH_2CH_3$), 2.17, 2.07, 2.06, 1.99 (s, 3H each, $CH_3CO$), 1.22 (t, 3H, $OCH_2CH_3$).

$^{13}$C—NMR ($CDCl_3$, TMS) δ 170.4, 170.3, 170.2, 169.4 (CO), 101.1 (Cl), 71.0, 70.6, 68.9, 67.1 (C2—C5), 65.7 ($CH_2CH_3$), 61.3 (C6), 20.8, 20.7, 20.7, 20.6 ($CH_3CO$), 15.1 ($CH_2CH_3$).

Ethyl 2,3,4-tri-O-acetyl-4-O-(2,3,4,6-tetra-O-acetyl-α-D-galactopyranosyl)-β-D-galactopyranoside (29)

2-Bromoethyl 2,3,6-tri-*O*-acetyl-4-*O*-(2,3,4,6-tetra-*O*-acetyl-α-D-galactopyranosyl)-β-D-galactopyranoside (*14*) (740 mg; 1 mmol) was dissolved in methanol (14 ml) by heating. Sodium methoxide in methanol (0.1 M; 2 ml) was added and the reaction mixture was stirred at room temperature for 2 h. Sodium hydroxide (87 mg; 2.2 mmol) in water (2 ml) was added together with the catalyst (10% Pd/C; 120 mg) and the reaction mixture was hydrogenated (15 h) at atmospheric pressure. Filtration (Celite®) and evaporation (<13.3 Pa) gave a colourless, amorphous residue that was suspended in a mixture of acetic anhydride and pyridine (1/1; 10 ml). The reaction mixture was stirred (75°; 3 h), cooled and poured into ether (50 ml). The ether solution was washed (water, sat. sodium hydrogen carbonate solution, water), dried (sodium sulfate) and evaporated (<0.1 torr) which gave *29* as a crystalline residue that was pure by TLC ($SiO_2$, ethyl acetate/isooctane 3/1). Yield: 650 mg (98%). Recrystallization from methanol-water gave an analytical sample (needles): mp 133—135°; $[\alpha]_D^{22}$ +81.4° (c 0.9; $CHCl_3$); $^1$H—NMR ($CDCl_3$, TMS) δ 5.57 (d, 1H, H4'), 5.39 (dd, 1H, $J_{3',4'}$=3.2Hz, H3'), 5.20 (dd, 1H, $J_{2',3'}$=11.0Hz, H2'), 5.18 (dd, 1H, $J_{2,3}$=10.8Hz, H2), 5.01 (d, 1H, $J_{1',2'}$=3.5Hz, H1'), 4.82 (dd, 1H, $J_{3,4}$=2.6Hz, H3), 4.48 (d, 1H, $J_{1,2}$=7.9Hz, H1), 4.06 (d, 1H, H4), 3.92, 3.59 (dq, 1H each, J=9.7 and 7.1Hz, $CH_3$—$CH_2$—O), 2.13, 2.11, 2.08, 2.075, 2.06, 2.04, 1.99 (S, 3H each, $CH_3$—CO), 1.22 (t, 3H, J=7.1Hz, $CH_3$—$CH_2$—O) ppm.

$^{13}$C—NMR ($CDCl_3$, TMS) δ 170.7, 170.6, 170.5, 170.4, 170.1, 169.7, 169.1 ($CH_3$—CO), 100.9 (C1), 99.4 (C1'), 77.2, 72.8, 71.9, 68.8, 68.6, 67.9, 67.4, 67.1 (C2—C5, C2'—C5'), 65.4 ($CH_2$—$CH_3$), 62.1, 60.5 (C6, C6'), 21.0, 20.8, 20.7, 20.6 ($CH_3$—CO), 15.1 ($CH_2$—$CH_3$) ppm.

Bis-galactopyranoside 30 and galactopyranoside thiol 31

2-Bromoethyl 2,3,4,6-tetra-*O*-acetyl-β-D-galactopyranoside (*1*) (915 mg; 2.0 mmol) and hexane-1,6-dithiol (150 mg; 1.0 mmol; 153 μl) were dissolved in benzene (3 ml) and aqueous potassium hydroxide (112 mg; 2 mmol; 3 ml) was added together with methyltrioctylammonium chloride (ca 10 mg). The reaction mixture was stirred under nitrogen for 24 h and the phases were separated. The aqueous phase was extracted with methylene chloride and the combined organic phases were washed with water. Drying and evaporation gave a residue that was chromatographed ($SiO_2$, ethyl acetate/isooctane 1/1) to give the title compounds *30* and *31*.

Compound *30*: Yield: 190 mg (21%); viscous syrup; $[\alpha]_D^{23}$ −10.3° (c 1.4; $CDCl_3$); $^1$H—NMR ($CDCl_3$, TMS) δ 5.40 (d, 2H, H4), 5.22 (dd, 2H, $J_{2,3}$=10.5Hz, H2), 5.03 (dd, 2H, $J_{3,4}$=3.5Hz, H3), 4.51 (d, 2H, $J_{1,2}$=8.0Hz, H1), 2.70, 2.53 (t, 4H each, J=7.0, S—$CH_2$). 2.16, 2.09, 2.06, 1.99 (s, 6H each, $CH_3CO$) ppm.

$^{13}$C—NMR ($CDCl_3$, TMS) δ 170.8, 170.7, 170.6, 169.9 (CO), 101.8 (C1), 71.3, 71.1, 69.1, 67.4 (C2—C5), 70.2 ($CH_2$—O), 61.7 (C6), 32.9, 31.8, 30.0, 28.8 ($CH_2$), 21.3, 21.1, 21.0 ($CH_3CO$) ppm.

Compound *31*: Yield: 280 mg (27%); viscous oil; $[\alpha]_D^{23}$ −8.9° (c 1.3; $CDCl_3$); $^1$H—NMR ($CDCl_3$, TMS) δ 5.41 (d, 1H, H4), 5.24 (dd, 1H, $J_{2,3}$=10.5Hz, $H_2$), 5.04 (dd, 1H, $J_{3,4}$=3.5Hz, H3), 4.53 (d, 1H, $J_{1,2}$=8.0Hz, H1), 2.72 (t, 2H, J=7.0Hz, S—$CH_2$), 2.50—2.60 (m, 4H, S—$CH_2$), 2.16, 2.08, 2.06, 1.99 (s, 3H each, $CH_3CO$) ppm.

$^{13}$C—NMR ($CDCl_3$, TMS) δ 170.8, 170.7, 170.6, 169.9 (CO), 101.9 (C1), 71.4, 71.2, 69.2, 67.5 (C2—C5), 70.3 ($CH_2$—O), 61.7 (C6), 34.3, 33.0, 31.9, 30.1, 28.7, 28.4, 25.0 ($CH_2$), 21.3, 21.2, 21.1 ($CH_3CO$) ppm.

Bis-di-galactopyranoside 32 and di-galactopyranoside thiol 33

2-Bromoethyl 2,3,6-tri-*O*-acetyl-4-*O*-(2,3,4,6-tetra-*O*-acetyl-α-D-galactopyranosyl)-β-D-galactopyranoside (*14*) (1486 mg; 2.0 mmol) was treated as above (compounds *30* and *31*). Chromatography ($SiO_2$, ethyl acetate/isooctane 4/1) gave the title compounds *32* and *33* together with the starting compound *14* (457 mg; 31%). Compound *32*: Yield: 312 mg (21%); viscous syrup; $[\alpha]_D^{23}$ +62.2° (c 0.9; $CHCl_3$); $^1$H—NMR ($CDCl_3$, TMS) δ 5.56 (d, 2H, $J_{3',4'}$=3.0Hz, H4'), 5.39 (dd, 2H, $J_{2',3'}$=11.0Hz, H2'), 5.20 (dd, 2H, $J_{2,3}$=11.0Hz, H2), 5.18 (dd, 2H, H3'), 5.00 (d, 2H, $J_{1',2'}$=4.0Hz, H1'), 4.81 (dd, 2H, $J_{3,4}$=10.5Hz, H3), 4.50 (d, 2H, $J_{1,2}$=8.0Hz, H1), 2.53, 2.72 (t, 4H each, J=7.0Hz, S—$CH_2$), 2.13, 2.10, 2.07, 2.06, 2.04, 1.98 (s, $CH_3$—CO) ppm.

$^{13}$C—NMR ($CDCl_3$, TMS) δ 170.7, 170.6, 170.5, 170.4, 170.1, 169.8, 169.1 ($CH_3CO$), 101.2 (C1), 99.4 (C1'),

77.1, 72.7, 72.0, 68.6, 68.6, 67.9, 67.4, 67.1 (C2—C5, C2'—C5'), 69.5 (O—$CH_2$), 62.0, 60.5 (C6, C6'), 32.6, 31.4, 29.8, 29.2, 28.8 ($CH_2$), 29.4 (C5 of nonane-dithiol-portion), 21.0, 20.8, 20.7, 20.6 ($CH_3$CO) ppm.

Compound *33*: Yield: 335 mg (20%); viscous syrup; $[\alpha]_D^{23}$ +60.3° (c 1.9; $CHCl_3$); $^1$H—NMR ($CDCl_3$, TMS) δ 5.58 (d, 1H, $J_{3',4'}$=3.0Hz, H4'), 5.40 (dd, 1H, $J_{2',3'}$=11.0Hz, H2'), 5.21 (dd, 1H, H3'), 5.19 (dd, 1H, $J_{2,3}$=11.0Hz, H2), 5.01 (d, 1H, $J_{1',2'}$=4.0Hz, H1'), 4.82 (dd, 1H, $J_{3,4}$=3.0Hz, H3), 4.52 (d, 1H, $J_{1,2}$=8.0Hz, H1), 2.74, 2.55, 2.52 (t, 2H each, J=7Hz, $CH_2$—S), 2.14, 2.11, 2.08, 2.07, 2.04, 1.99 (s, $CH_3$CO) ppm.

$^{13}$C—NMR ($CDCl_3$, TMS) δ 170.7, 170.5, 170.4, 170.3, 170.1, 169.8, 169.1 ($CH_3$—CO), 101.2 (C1), 99.4 (C1'), 77.1, 72.7, 71.9, 68.6, 68.6, 67.8, 67.4, 67.1 (C2—C5, C2'—C5'), 69.5 (O—$CH_2$), 62.0, 60.5 (C6, C6'), 34.0, 32.6, 31.3, 29.8, 29.4, 29.2, 29.0, 28.8, 28.3, 24.6 ($CH_2$), 20.9, 20.8, 20.7, 20.6 ($CH_3$—CO) ppm.

## Examples 34—44

The 2-bromoethyl group in standard carbohydrate synthetic chemistry

The H-blood group specific disaccharide[22] has been synthesized in the form of the 2-bromoethyl glycoside, as an illustration of the compatibility of the 2-bromoethyl group with synthetic chemical reactions.

2-Bromoethyl 4,6-O-benzylidene-β-D-galactopyranoside (35)

2-Bromoethyl 2,3,4,6-tetra-*O*-acetyl-β-D-galactopyranoside (*1*) (30.0 g; 76 mmol) was dissolved in methanol (100 ml) and added to a solution of sodium (0.2 g) in methanol (500 ml) at room temperature. The de-acetylation was completed within a few minutes (TLC: $SiO_2$, ethyl acetate/acetic acid/water 2/1/1). The reaction mixture was neutralized with ion-exchange resin (Duolite $H^+$, methanol-washed), the resin was filtered off and the filtrate was evaporated (<40°) to give the glycoside *34* (18 g; unstable syrup). This was used immediately in the following step.

Dry zink (II) chloride[23] (14.4 g; 106 mmol) was dissolved in tetrahydrofuran (80 ml) and benzaldehyde (11.2 ml; 106 mmol; freshly distilled and kept under nitrogen in the dark) was added. The solution was stirred for 15 min. at room temperature and 2-bromoethyl β-D-galactopyranoside *34* (18 g; crude product; *vide supra*), dissolved in tetrahydrofuran (90 ml), was added. The reaction mixture was stirred over night at room temperature while protected from light. Evaporation gave a residue that was dissolved in methylene chloride (200 ml) and washed with water (2 × 100 ml). Drying (sodium sulfate) and evaporation gave a crude product that was dissolved in methanol (55 ml) by heating. The hot solution was diluted with ether (200 ml) and cooled which gave the title compound (*35*) by crystallization (12.5 g). The mother liquor was evaporated and chromatographed ($SiO_2$, ethyl acetate/methanol 85/15) which gave a further crop of *35* (1.8 g). Yield: 13.85 g (49%). Mp 138—139°; $[\alpha]_D^{24}$ −29.9° (c 1.2; $CDCl_3$).

$^1$H—NMR ($CDCl_3$, TMS) δ 7.4 (m) 7.5 (m) (5H, aromatic), 5.56 (s, 1H, benzylidene), 4.35 (d, 1H, H—1, $J_{1,2}$=7.1Hz), 4.32 (dd, H—6B, $J_{6A,6B}$=12.7Hz, $J_{5,6B}$=1.5Hz), 4.27 (t, O—$CH_2CH_2$), 4.21 (m, 2H, H—3, H—4), 4.08 (dd, 1H, H—6A, $J_{5,6A}$=1.8Hz), 3.88 (m, O$CH_2CH_2$), 3.79 (m, H—2), 3.51—3.58 (m, 2H, $CH_2$Br), 3.47 (m, 1H, 5—H).

$^{13}$C—NMR ($CDCl_3$, TMS) δ 129.3, 128.3, 126.5 (aromatic), 103.1, 101.5 (C—1, benzylidene), 75.3, 72.6, 71.6, 66.9 (C—5——C—2), 69.5, 69.2 (C—6, O—$CH_2CH_2$), 30.4 ($CH_2$Br).

2-Bromoethyl 3-O-benzoyl-4,6-O-benzylidene-β-D-galactopyranoside (36)

2-Bromoethyl 4,6-*O*-benzylidene-β-D-galactopyranoside (*35*) (10.0 g; 26.7 mmol) was dissolved in methylene chloride (50 ml) and pyridine (20 ml) and cooled to −30°. A solution of benzoyl chloride (5.2 g; 37 mmol) in methylene chloride (60 ml) was added dropwise and the temperature of the reaction mixture was allowed to rise to 0° during 1 h (TLC: $SiO_2$, ethyl acetate/isooctane 2/1). The reaction mixture was washed with cold water and the water phase was extracted several times with methylene chloride. The combined organic phases were dried ($Na_2SO_4$) and evaporated twice with toluene. The residue was chromatographed ($SiO_2$, ethyl acetate/isooctane 2/1) and crystallized from isopropanol. The mother liquor was re-chromatographed and crystallized. The total yield of crystalline material (*36*) was 7.6 g (59%). Mp 141°C. $[\alpha]_D^{23}$ = +100° (C = 0.78, $CHCl_3$) $^1$H—NMR ($CDCl_3$, TMS) δ 8.13, 8.10, 7.43—7.35 (m, 10H, aromatic), 5.53 (s, 1H, benzylidene), 5.17 (dd, 1H, H—3, $J_{2,3}$=10.2Hz), 4.51 (d, H—1, $J_{1,2}$=7.7Hz), 4.50 (bd, H—4), 4.36 (dd, H—6B), 4.29 (m, O$CH_2CH_2$), 4.24 (dd, H—2, $J_{2,3}$=10.2Hz), 4.10 (dd, 1H, H—6A, $J_{6A,6B}$=12.7Hz, $J_{5,6}$=1.8Hz), 3.88 (m, 1H, O$CH_2CH_2$), 3.47 (m, H—5), 3.55 (m, $CH_2$Br).

2-Bromoethyl 3-O-benzoyl-4,6-O-benzylidene-2-O-(2,3,4-tri-O-benzyl-α-L-fucopyranosyl)-β-D-galactopyranoside (37)

A methylene chloride solution of 1-bromo-2,3,4-tri-*O*-benzyl-α-L-fucopyranose, prepared from 1-O-(p-nitrobenzoyl)-2,3,4-tri-*O*-benzyl-β-L-fucopyranose (4.00 g; 6.86 mmol) with hydrogen bromide in methylene chloride (total volume 30 ml) was added to a mixture of 2-bromoethyl 3-*O*-benzoyl-4,6-*O*-benzylidene-β-D-galactopyranoside (*36*) (4.00 g; 8.35 mmol), tetraethyl ammonium bromide (1.8 g; 8.5 mmol) and 5 g of 4Å molecular sieves in 20 ml of methylene chloride at room temperature in a dry nitrogen atmosphere. The proceeding of the reaction was followed by TLC (toluene:ethyl acetate 5:1). Two additional batches of the fucosyl bromide (from 2.00 g; 3.46 mmol of the precursor) were added after 2 and 5 days, respectively. Totally 8.00 g (13.7 mmol) of 1-O-(p-nitrobenzoyl)-2,3,4-*O*-benzyl-β-L-fucopyranose was used. After 8 days, *31* was almost completely consumed. The reaction mixture was filtered through

Celite, which was washed with 2 × 25 ml of methylene chloride. The filtrate was washed with 2 × 50 ml of aqueous sodium bicarbonate and 50 ml of water. After drying ($Na_2SO_4$) and evaporation of the solvent, the residue was chromatographed ($SiO_2$, toluene:ethyl acetate 5:1) to give 3.0 g (40%) of the pure title compound (*37*) as a foam, which was recrystallized from isooctane-ethyl acetate; mp 172°C. $[\alpha]_D^{24}$ +26° (c = 0.72, $CHCl_3$).

$^1H$—NMR ($CDCl_3$, TMS) δ 8.06—6.98 (m, 25H, aromatic), 5.50 (s, benzylidene), 5.46 (d, H—1', $J_{1',2'}$=3.0Hz), 5.40 (dd, H—3, $J_{2,3}$=9.7Hz, $J_{3,4}$=3.8Hz), 4.95 (d, 1H, benzyl), 4.77 (d, 1H, benzyl), 4.68 (d, H—1, $J_{1,2}$=7.7Hz), 4.56 (H—4), 4.56 (H—5'), 4.47 (H—2), 4.34 (m, $OCH_2CH_2$), 4.32 (s, benzyl), 4.10 (bd, 1H, H—6A, $J_{6A,6B}$=13.6Hz), 4.02 (dd, 1H, H—3'), 3.94 (dd, 1H, H—2'), 3.73 (m, $OCH_2CH_2$), 3.68 (bs, 1H, H—4'), 3.59 (bs, 1H, H—5), 3.49 (m, 2H, $CH_2Br$), 1.14 (d, 3H, H—6', $J_{5',6'}$=6.6Hz).

2-Bromoethyl 4,6-O-benzylidene-2-O-(2,3,4-tri-O-benzyl-α-L-fucopyranosyl)-β-D-galactopyranoside (38)

Methanolic sodium methoxide was prepared from 250 ml of methanol and sodium (0.1 g; 4 mmol). Compound, *37* (4.00 g; 4.46 mmol) in 25 ml of methanol was added and the reaction mixture was stirred for 4 days at room temperature. TLC (toluene/ethyl acetate 5/1) showed complete conversion of *37*. The reaction manner was neutralized with methanol-washed Duolite—$H^+$ and filtered. The solvent was removed to give 3.45 g of crude *33*, which was chromatographed ($SiO_2$, toluene:ethyl acetate 5:1). Thus 2.40 g (68%) of *38* was obtained as a foam. $[\alpha]_D^{23}$ −49° (c = 0.78, $CHCl_3$).

$^1H$—NMR ($CDCl_3$, TMS) δ 7.54—7.22 (m, 20H, aromatic), 5.55 (s, 1H, benzylidene), 5.30 (d, 1H, H—1', $J_{1',2'}$=3.2Hz), 4.97 (d, 1H, benzylic), 4.64 (d, benzylic), 4.45 (d, H—1, J~8Hz), 4.32 (H—5'), 4.03 (H—2'), 1.13 (d, 3H, H—6', $J_{5',6'}$=6.4Hz).

$^{13}H$—NMR ($CDCl_3$, TMS) δ 101.8, 101.4 (C—1 and benzylidene —C), 99.1 (C—1'), 30.1 ($CH_2Br$), 16.7 (C—6').

2-Bromoethyl 3,4,6-tri-O-acetyl-2-O-(2,3,4-tri-O-acetyl-α-L-fucopyranosyl-β-D-galactopyranoside (40)

A solution of *38* (0.50 g; 0.63 mmol) in 20 ml of acetic acid was hydrogenated with 0.50 g 10% palladium on charcoal at atmospheric pressure over night. The reaction mixture was filtered and the solvent was removed by repeated evaporations with toluene to give 0.27 g (100%) of 2-bromoethyl 2-*O*-(α-L fucopyranosyl)-β-D-galactopyranoside (*39*), which was pure by TLC ($SiO_2$; chloroform; methanol; water 65:35:10). This product was acetylated with 5 ml of pyridine and 2.5 ml of acetic anhydride at room temperature over night. Evaporation of the solvents several times with toluene followed by drying at $10^{-2}$ mm Hg gave 230 mg (53%) of pure 2-bromoethyl 3,4,6-tri-*O*-acetyl-2-*O*-(2,3,4-tri-*O*-acetyl-α-L-fucopyranosyl)-β-D-galactopyranoside (*40*) (TLC, $SiO_2$; iso-octane:ethyl acetate 1:2). $[\alpha]_D^{23}$ −77° (c 0.95, $CHCl_3$).

$^1H$—NMR ($CDCl_3$, TMS) δ 5.43 (d, 1H, H—1', $J_{1',2'}$=3.7Hz), 5.34 (dd, H—3'), 5.3 (bs, H—4, H—4'), 5.04 (dd, 1H, H—3, $J_{3,4}$=3.4Hz), 4.99 (dd, 1H, H—2'), $J_{2',3'}$=10.9Hz), 4.74 (q, 1H, H—5', $J_{5',6'}$=6.7Hz), 4.55 (d, 1H, H—1, $J_{1,2}$=7.9Hz), 4.30 (dt, O—$CH_2$ $CH_2$), 4.1 (H—6), 4.00 (dd, H—2, $J_{2,3}$=10Hz), 3.90 (m, $OCH_2$ $CH_2$ and H—5), 3.52 (m, 2H, $CH_2Br$), 1.15 (d, 3H, H—6'), 2.15, 2.14, 2.05, 2.00, 1.99, 1.98 (18H, OAc).

2-(2-Methoxycarbonylethylthio)ethyl 2-O-(α-L-fucopyranosyl)-β-D-galactopyranoside (42)

Without further purification, *40* (230 mg; 0.335 mmol) was dissolved in 0.7 ml of benzene. To this solution, methyl 3-mercaptopropionate (94 mg; 0.787 mmol), water (0.5 ml), cesium carbonate (257 mg; 0.787 mmol) and methyl trioctyl ammonium chloride (ca 5 mg) were added and the resulting mixture stirred at room temperature for 24 hours. TLC ($SiO_2$; isooctane:ethyl acetate 1:2) showed a new more polar spot as the major product but a considerable amount of the starting material was still left. Another 2 equivalents of cesium carbonate and the thiol ester were added and stirring was continued for 7 days. At this point there was still starting material left but since deacetylation products started to appear, the reaction mixture was diluted with 50 ml of benzene, the organic phase was separated, washed with water and dried ($Na_2SO_4$). After removal of the solvent the crude product was chromatographed ($SiO_2$; isooctane:ethyl acetate 1:1) to give 150 mg (62%) of *41* as a syrup.

Sodium methoxide in methanol was prepared from 50 ml of dry methanol and 10 mg of sodium. To this solution, *41* (150 mg; 0.207 mmol) was added and the mixture stirred for 4 h at room temperature. TLC (chloroform, methanol, water: 65/35/10) showed at this point a single spot. Duolite—$H^+$ (methanol washed and carefully dried) was added to neutrality. Filtration and evaporation of the solvent gave 76 mg (78%) of the title compound (*42*) as a syrup, which was used without further purification for the next step. $[\alpha]_D^{20}$=−75° (c 0.70, $H_2O$).

$^1H$—NMR ($D_2O$, TMS as external standard) δ 5.29 (d, 1H, H—1', $J_{1',2'}$=3,6Hz), 4.54 (d, 1H, H—1, $J_{1,2}$=7.7Hz), 4.43 (q, 1H, H—5'), 4.1 (m, 1H, O—$CH_2CH_2$), 3.75 (s, $OCH_3$), 3.63 (dd, H—2), 2.88—2.75 (m, 6H, $CH_2S$, $CH_2$—CO), 1.23 (d, 3H, H—6', $J_{5',6'}$=6.4Hz).

$^{13}C$—NMR ($D_2O$, TMS as external standard) δ 178.1 (CO), 104.3, 102.0 (C—1, C—1'), 78.9, 77.9, 76.6, 74.8, 72.3, 71.8, 71.5, 71.1, 69.7, 63.8, 55.2, 37.1, 33.9, 29.3 (ring- and spacer carbons), 18.2 (C—6').

The blood group H disaccharide conjugate with BSA (44)

Compound *42* (61 mg; 0.13 mmol) was dissolved in 0.25 ml of absolute ethanol and 0.10 ml of 85% hydrazine hydrate was added. After 2 days the solvent and the excess of hydrazine hydrate were

evaporated to give a quantitative yield of hydrazide *43* (TLC; chloroform, methanol, water; 65/35/10. The hydrazide *43* (20 mg; 0.043 mmol) was dissolved in DMF (1 ml) and the solution cooled to −25°C. A 4.2N solution of hydrogen chloride in dioxane (60 µl) was added, followed by t-butylnitrite (29 mg; 0.08 mmol) in 80 µl of DMF. After 30 min, sulfamic acid (80 µl, 0.040 mmol) was added and stirring continued for 15 min. The acylazide solution was added directly to a 0°C solution of bovine serum albumine (BSA) (58 mg) dissolved in an aqueous solution, 0.08 M in $Na_2B_4O_7$ and 0.35 M in $KHCO_3$ (2 ml). The pH of the solution was adjusted to 9.05—9.30 with 1N aqueous NaOH and 1N HCl during the addition of the acylazide solution. After 20 h at +4° the reaction mixture was dialyzed against water and lyophilized to provide the antigen (33 mg) as a white powder. The degree of incorporation was found to be 8 mol of hapten per mol of BSA as determined by the phenol-sulfuric acid method[16].

Examples 9b and 45—47

Four new compounds were prepared and characterized: Compound *9b* (Table 1, 2 and 3); compounds *45, 46* and *47* (*vide infra*). These four compounds were all prepared according to Method A as defined in Table 1 and Experimental, using the appropriate ω-bromoalkyl alcohol. Product data and yields for compounds *45—47* are given in Table 5.

Table 5. Product data and yields for the preparation of ω-bromoalkyl glycosides (c.f. Method A).

| Product | Starting alcohol | Yield (%)[a] | Melting point (°C) | $[\alpha]_D$ |
|---|---|---|---|---|
| 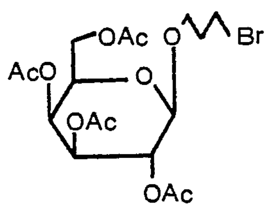 *45;* D—Gal | 3-bromopropanol | 53 | 68-70 | +7.7(c 1.0; $CHCl_3$) |
| 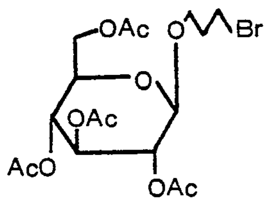 *46;* D—Glc | 3-bromopropanol | 90 | 67-68 | +0.4(c 1.1; $CHCl_3$) |
| 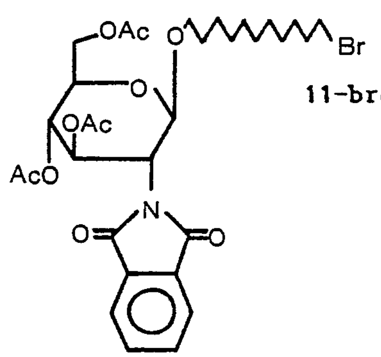 *47;* D—GlcNPhtp | 11-bromoundecanol | 35 | sirup | +15.6(c 1.0; $CHCl_3$) |

a) Isolated by chromatography on silica gel.

Examples 48—50 below are full accounts of the preparation of glycoconjugates where the carbohydrate part has a well-documented biological significance. These examples should be read independently of each other and of the preceding disclosure.

Example 48

2-Bromoethyl glycosides. Synthesis of spacer-arm, lipid and ethyl glycosides of the trisaccharide portion of the blood-group p$^k$ antigen. Preparation of neo-glycoproteins

SUMMARY OF THE EXAMPLE

The title compounds were prepared *via* the corresponding 2-bromoethyl glycoside 11 by displacement of bromide ion with methyl 3-mercaptopropionate, octadecylthiol and hydrogen, respectively. Silver triflate promoted glycosylation of 2-bromoethyl 2,3,6-tri-*O*-benzyl-β-*D*-glucopyranoside (6) with 2,3,6-tri-*O*-acetyl-4-*O*-(2,3,4,6-tetra-*O*-acetyl-α-*D*-galactopyranosyl) α-*D*-galactopyranosyl bromide (1) gave 11. A tetra-deuterated analog (26) was prepared by essentially the same route. The spacer-arm glycoside (16) was coupled to bovine serum albumine and key-hole limpet haemocyanine, thus furnishing the corresponding neo-glycoproteins.

INTRODUCTION

Trihexosylceramide[24] (the P$^k$-antigen[25]) and globotetraosylceramide[26] (the P-antigen[25]) have been suggested to function as receptors for pathogenic *E.coli* bacteria in the human urinary tract. We recently reported an improved synthesis[27] of the Galα1→4Gal unit (considered to be the important part of the receptor) of these ceramides, as well as a series of derivatives of value for the biological evaluation of the receptor phenomenon. Simple glycosides (Me and p-$NO_2C_6H_4$) of the trisaccharide (Galα1→4Galβ1→4Glc) of the p$^k$-antigen have been prepared[28] via suitably protected lactose- and galactose derivatives. We now report an alternative route, starting from the acetobromo sugar 1 and the 2-bromoethyl glucoside 6, leading to a common 2-bromoethyl glycoside precursor (11) that was used for the preparation of spacer-arm, lipid and ethyl glycosides of the P$^k$-antigen.

Specifically deuterated derivatives of carbohydrates can be used to simplify interpretations of mass- and $^1$H-n.m.r.-spectra, and as labeled compounds for investigations of dynamic properties by $^2$H-n.m.r. techniques of for example neo-glycolipids in cell membranes. We have prepared a tetra-deuterated analog to 11 (26) from 23 and 6, following the same route as for the preparation of 11 (*vide infra*). (See Fig. 7)

RESULTS AND DISCUSSION

2-Bromoethyl 2,3,4,6-tetra-*O*-acetyl-β-*D*-glucopyranoside[29] (2) was treated with methanolic sodium methoxide and the deacetylated product in *N,N*-dimethylformamide was immediately added to a mixture of zink-II-chloride and benzaldehyde[30] to give 73% of 3. Treatment with benzyl bromide under phase-transfer catalysis conditions gave crystalline 4 and 5 in 80% and 20% yield, respectively. Treatment[31] of 4 with sodium cyanoborohydride gave 2-bromoethyl 2,3,6-tri-*O*-benzyl-β-*D*-glucopyranoside (6) in 59% yield. Treatment of 3 with benzoyl chloride in pyridine gave 7 in 86% yield. Reduction of the benzylidene group as above gave 2-bromoethyl 2,3-di-*O*-benzoyl-6-*O*-benzyl-β-*D*-glucopyranoside (8) in 65% yield.

The partly protected 2-bromoethyl glycosides 6 and 8 were used as aglycons in silver triflate-promoted glycosylations with 2,3,6-tri-*O*-acetyl-4-*O*-(2,3,4,6-tetra-*O*-acetyl-α-*D*-galactopyranosyl)-α-*D*-galactopyranosyl bromide[29] (1). With 6, the β-glycoside 9 (and a small amount of α-glycoside; *vide infra*) was formed in 55% yield, whereas with 8, a ~3:1 αβ-mixture (14 and 12) was formed. Catalytic hydrogenation of 12 gave 13 in 100% yield which on treatment with methanolic sodium methoxide and finally reacetylation gave 11.

Catalytic hydrogenation of recrystallised 9 in acetic acid gave a quantitative yield of 10 (basic reaction conditions leave benzyl groups intact whereas the use of acidic conditions permits the removal of such protecting groups without affecting the 2-bromoethyl aglycon). Acetylation gave 69% yield of the 2-bromoethyl glycoside 11, which is central for the preparation of the remaining compounds (15—22). The tetradeuterated compound 26 was prepared by the same method from 23 and 6 via 24 and 25.

Nucleophilic displacement of bromide ion from 11, using methyl 3-mercaptopropionate[32] or octadecyl-thiol in *N,N*-dimethylformamide containing cesium carbonate, gave the spacer-arm glycoside 15 and the neo-glycolipid 17 both in 87% yield. Deacetylation of 15 and 17 gave 16 and 18 in 97% and 88% yield. The glycoside 16 was coupled to bovine serum albumin and keyhole limpet haemocyanin, using a modification[1] of the Inman-Lemieux procedure[33–35] with dimethylsulfoxide as solvent instead of N,N-dimethylformamide, thus furnishing the neo-glycoproteins 21 and 22, respectively. Catalytic hydrogenation of 11 under basic conditions[35], followed by acetylation of the product, gave 19 in 62% yield. Deacetylation of 19 gave a quantitative yield of 20. The use of crude 11 in the latter reaction sequence gave a small amount (<5%) of α-glycoside (t.l.c. and $^1$H—n.m.r.) that had been formed in the reaction of 6 with 1. The structure of 20 was confirmed by sugar[36] and methylation[37] analysis.

EXPERIMENTAL

General methods were as reported[38]. Tetramethylsilane ($Me_4Si$) or sodium 3-(trimethylsilyl)-propionate-$d_4$ (TSP) were used as n.m.r. references.

2-Bromoethyl 4,6-*O*-benzylidene-β-*D*-glucopyranoside (3).

A solution of 2-bromoethyl 2,3,4,6-tetra-*O*-acetyl-β-*D*-glucopyranoside[29] (2, 30 g, 66 mmol) in warm methanol (300 mL) was rapidly cooled and treated with methanolic sodium methoxide (0.1 M, 30 mL) at

room temperature for 3 h. The reaction was monitored by t.l.c. ($SiO_2$, chloroform-methanol-water, 65:35:10, lower phase). The reaction mixture was filtered through a column (5 × 4 cm) of Duolite® C 26 ($H^+$) resin and the solvent was removed. The residue (18.6 g) in *N,N*-dimethylformamide (20 mL) was added dropwise (10 min) with stirring to a mixture of zink chloride (44 g, 103 mmol) and benzaldehyde (48 g, 471 mmol). After 21 h, ether (500 mL) and ice-water (300 mL) was added and the aqeous phase was extracted with ether (2 × 100 mL). The ether extracts were washed with aqueous sodium hydrogencarbonate (2 × 100 mL) and water (50 mL). Dichloromethane was added to prevent crystallisation. The solution was dried ($Na_2SO_4$) and the solvent was removed, which gave crystalline 3 (18.1 g, 73%). Recrystallisation from ethanol gave material with m.p. 157—159°, $[\alpha]_D^{21}$ −43° (*c* 2, chloroform). N.m.r. data: $^1H$ ($CDCl_3$ + $D_2O$, $Me_4Si$) δ 7.56—7.34 (5H), 5.52 (s, 1H, $O_2CH$—Ph), 4.43 (d, 1H, *J* 8Hz, H—1), 4.32 (dd, 1H, *J* 11 and 5Hz), 4.14 (td, 1H, *J* 6 and 11Hz), 3.97—3.34 (8H). $^{13}C$ ($CDCl_3$ $Me_4Si$) δ 136.9, 129.3, 128.3 (2 C), 126.3 (2 C), 103.3, 101.9 (C—1), 80.4, 74.4, 73.0, 69.9 ($CH_2$), 68.5 ($CH_2$), 66.4, 30.1 ($CH_2$—Br).

Anal. Calc. for $C_{15}H_{19}O_6Br$: C, 48.01; H, 5.10.
Found: C, 48.18; H, 5.04.

2-Bromoethyl 2,3-di-*O*-benzyl-4,6-*O*-benzylidene-β-*D*-glucopyranoside (4)

A mixture of 3 (4.4 g, 11.7 mmol), benzyl bromide (20 mL, 77 mmol), tetrabutylammoniumhydrogen sulfate (0.5 g) and aqueous sodium hydroxide (10%, 44 mL) was stirred at room temperature for 17 h, diluted with toluene, washed with water, dried ($Na_2SO_4$) and concentrated. Column (5 × 18 cm) chromatography ($SiO_2$, toluene, then toluene-ethyl acetate 19:1 and finally ethyl acetate) gave 4 (5.2 g, 80%) and 5 (0.9 g, 20%).

Compound 4 had m.p. 97—98° (from ethanol), $[\alpha]_D^{21}$ −30° (*c* 2, chloroform). N.m.r. data: $^1H$ ($CDCl_3$, $Me_4Si$) δ 7.55—6.88 (15H), 5.58 (s, 1H, $O_2CH$—Ph), 4.98, 4.77 (ABq, 2H, $J_{AB}$ 11Hz, $CH_2$Ph), 4.92, 4.81 (ABq, 2H, $J_{AB}$ 11.5Hz, $CH_2$Ph), 4.56 (d, 1H, *J* 7.5Hz, H—1), 4.35 (dd, 1H, *J* 10.5 and 5Hz) 4.22 (dt, 1H, *J* 11 and 5.5Hz, $CH_2$—$CH_2$—Br), 4.01—3.63 (4H), 3.59—3.35 (4H), 3.52 (bt, *J* 6Hz, $CH_2$Br). $^{13}C$ ($CDCl_3$, $Me_4Si$) δ 138.4, 138.2, 137.2, 129.0, 128.32 (2C), 128.28 (2C), 128.23 (4C), 128.01 (2C), 127.7, 127.6, 126.0 (2C), 104.1, 101.1 (C—1), 81.9, 81.3, 80.7, 75.4 ($CH_2$), 75.1 ($CH_2$), 70.0 ($CH_2$), 68.7 ($CH_2$), 66.1, 30.0 ($CH_2$Br).

Anal. Calc. for $C_{29}H_{31}O_6Br$: C, 62.70; H, 5.63.
Found: C, 62.94; H, 5.62.

Compound 5 had m.p. 160.5—162.5 (from ethanol-ethyl acetate), $[\alpha]_D^{21}$ −21° (*c* 1.5, chloroform). N.m.r. data: $^1H$ ($CDCl_3$, $Me_4Si$) δ 7.52—7.18 (10H), 5.57 (s, 1H, $O_2CH$—Ph), 4.86 (s, 2H, $CH_2$—Ph), 4.42 (d, 1H, *J* 8Hz, H—1), 4.38 (dd, 1H, *J* 10 and 4.5Hz), 4.02—3.51 (8H), 3.31 (bt, 1H, *J* 7.5Hz). $^{13}C$ ($CDCl_3$, $Me_4Si$) δ 138.3, 137.1, 129.0, 128.3 (2C), 128.2 (2C), 127.8 (2C), 127.6, 126.0 (2C), 101.5 and 99.1 (C—1 and CH—Ph), 81.7, 80.5, 77.4, 74.3 ($CH_2$), 68.5 ($CH_2$), 68.2, 66.8 ($CH_2$), 66.4.

Anal. Calc. for $C_{22}H_{24}O_6$: C, 68.73; H, 6.29.
Found: C, 68.58; H, 6.33.

2-Bromoethyl 2,3,6-tri-*O*-benzyl-β-*D*-glucopyranoside (6)

A mixture of 4 (29.5 g, 53 mmol), sodium cyanoborohydride (9 g, 143 mmol), molecular sieves (3Å, 30 g) and tetrahydrofuran (175 mL) was stirred while hydrogen chloride-saturated ether (225 mL) was added dropwise[31]. Additional amounts of cyanoborohydride (1 g) and ether solution (25 mL) were added. T.l.c. ($SiO_2$, toluene-ethyl acetate 19:1) showed that 4 had been consumed. Toluene (500 mL) was added and the mixture was washed with ice-water (250 mL) and cold aqueous sodium hydrogen carbonate (250 mL), dried ($Na_2SO_4$) and concentrated to give a semi-crystalline residue (32 g). Column (10 × 18 cm) chromatography ($SiO_2$, ethyl acetate-isooctane 1:2) gave 6 (17.5 g, 59%). Recrystallisation from toluene-isooctane gave an analytical sample with m.p. 63—64°, $[\alpha]_D^{21}$ −18° (*c* 1.0, chloroform). N.m.r. data: $^1H$ ($CDCl_3$, $Me_4Si$) δ 7.45—7.27 (15H), 5.03, 4.72 (ABq, 2H, $J_{AB}$ 10.5Hz, $CH_2$—Ph), 4.95, 4.72 (ABq, 2H, $J_{AB}$ 11.5Hz, $CH_2$—Ph), 4.60, 4.57 (ABq, 2H, $J_{AB}$ 12.0Hz, $CH_2$—Ph), 4.47 (d with virtual coupling[17], 1H, $J_{1,2}$ 7.5Hz, H—1), 4.23 (dt, 1H, *J* 11 and 5.5Hz, $CH_2$—$CH_2$—Br), 3.95—3.39 (10H). $^{13}C$ ($CDCl_3$, $Me_4Si$) δ 138.5, 138.3, 137.8, 128.6 (2C), 128.4 (2C), 128.36 (2C), 128.3 (2C), 128.0 (2C), 127.9, 127.7 (2C), 127.68 (2C), 103.8 (C—1), 83.8, 81.5, 75.3 ($CH_2$), 74.8 ($CH_2$), 74.2, 73.6 ($CH_2$), 71.2, 70.0 ($CH_2$), 69.7 ($CH_2$), 30.3 ($CH_2$Br).

Anal. Calc. for $C_{29}H_{33}O_6Br$: C, 62.48; H, 5.97.
Found: C, 62.64; H, 5.97.

2-Bromoethyl 2,3-di-*O*-benzoyl-4,6-*O*-benzylidene-β-*D*-glucopyranoside (7)

Benzoyl chloride (20 mL, 240 mmol) was added (7 min) dropwise with stirring to a cooled (0°) solution of 3 (17 g, 45.3 mmol) in pyridine (85 mL). Ice-water (125 mL) was added after 1 h and the mixture was extracted with ether-dichloromethane (2:1, 750 mL). The organic phase was washed with aqueous sodium hydrogencarbonate (100 mL), and brine (100 mL), dried ($Na_2SO_4$) and concentrated to give 6 (27.7 g, 82%). Recrystallisation from toluene-ethanol gave an analytical sample with m.p. 176—178°, $[\alpha]_D^{21}$ −0.1° (*c* 1.1, chloroform). N.m.r. data: $^1H$ ($CDCl_3$, $Me_4Si$) δ 8.06—7.94 (4H), 7.60—7.20 (11H), 5.80 (t, 1H, *J* 9Hz, H—3), 5.55 (s, 1H, CH—Ph), 5.50 (dd, 1H, *J* 9 and 8Hz, H—2), 4.88 (d, 1H, *J* 8Hz, H—1), 4.44 (dd, 1H, *J* 10 and 5Hz, H—5), 4.16 (dt, 1H, *J* 11 and 6Hz, $CH_2CH_2Br$), 4.04—3.65 (4H), —3.39 (t, 2H, *J* 6Hz, $CH_2$Br). $^{13}C$ ($CDCl_3$, $Me_4Si$) δ 165.6, 165.2, 136.7, 133.2, 133.1, 129.8 (4C), 129.4, 129.3, 129.1, 128.3 (4C), 128.2 (2C), 126.1 (2C), 101.8 and 101.5 (C—1 and CH—Ph), 78.7, 72.2, 72.0, 69.8 ($CH_2$), 68.6 ($CH_2$), 66.7, 29.5 ($CH_2$Br).

Anal. Calc. for $C_{29}H_{27}O_8Br$: C, 59.70; H, 4.66.
Found: C, 60.08; H, 4.80.

2-Bromoethyl 2,3-di-*O*-benzoyl-6-*O*-benzyl-β-*D*-glucopyranoside (8)

A mixture of 7 (26.1 g, 45 mmol), sodium cyanoborohydride (7 g, 110 mmol), molecular sieves (4Å, 20 g) and tetrahydrofuran (150 mL) was treated essentially as in the preparation of 6 to give 8 (17.1 g, 65%). Recrystallisation from isooctane-toluene gave an analytical sample with m.p. 78—80°, $[\alpha]_D^{21}$ +53° (*c* 1.0, chloroform). N.m.r. data: $^1H$ ($CDCl_3$, $Me_4Si$) δ 8.04—7.92 (4H), 7.57—7.27 (11H), 5.52—5.37 (2H), 4.76 (d with virtual coupling[39], 1H, $J_{1,2}$ 8Hz, H—1), 4.65, 4.61 (ABq, 2H, $J_{AB}$ 12Hz, $CH_2$—Ph), 4.14 (dt, 1H, *J* 11 and 6Hz, $CH_2$—$CH_2$—Br), 4.02—3.62 (5H,), 3.40 (bt, 2H, *J* ~7Hz, $CH_2Br$). $^{13}C$ ($CDCl_3$, $Me_4Si$) δ 167.1, 165.3, 137.6, 133.4, 133.1, 129.9 (2C), 129.7 (2C), 129.4, 128.9, 128.5 (2C), 128.4 (2C), 128.3 (2C), 127.9, 127.7 (2C), 101.1 (C—1), 76.5, 74.9, 73.7 ($CH_2$), 71.3, 70.6, 69.7 ($CH_2$), 69.5 ($CH_2$), 29.65 ($CH_2Br$).

Anal. Calc. for $C_{29}H_{29}O_8Br$: C, 59.49; H, 4.99.
Found: C, 59.19; H, 5.11.

2-Bromoethyl 2,3,6-tri-*O*-benzyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (9)

A solution of 1[29] (21 g, 30 mmol) in dichloromethane (70 mL) was added (45 min) dropwise with stirring to a solution (−78°, $N_2$) of 6 (14 g, 25 mmol), silver trifluoromethanesulfonate (9 g, 35 mmol) and tetramethylurea (4.6 g, 39.6 mmol) in dichloromethane (130 mL). After 5 h the mixture, now at room temperature, was filtered through Celite®, diluted with dichloromethane and washed with *M* hydrochloric acid, aqueous sodium hydrogencarbonate and finally dried ($Na_2SO_4$) and concentrated. The residue (35 g) was chromatographed ($SiO_2$, ethyl acetate-isooctane gradient 1:1 → 3:2) to give 1 (2 g) and 9 (16.5 g). Rechromatography of a fraction containing impure 9 gave additional material (1.3 g). Total yield of 9 was 17.8 g (70% based on reacted 6). Recrystallisation from abs. ethanol gave an analytical sample with m.p. 155—158°, $[\alpha]_D^{21}$ +58° (*c* 1.2, chloroform). N.m.r. data: $^1H$ ($CDCl_3$, $Me_4Si$) δ 7.42—7.20 (15H), 5.58 (dd, 1H, *J* 3 and <1Hz, H—4''), 5.30 (dd, 1H, *J* 11 and 3Hz, H—3''), 5.17 (dd, 1H, J 11 and 3.5Hz, H—2''), 5.11 (dd, 1H, J 11 and 8Hz), H—2'), 5.03 (d, 1H, J 11.5Hz, $CH_2Ph$), 4.94 (d, 1H, J 3.5Hz, H—1''), 4.94 (d, 1H, J 11Hz, $CH_2Ph$), 4.83 (d, 1H, J 11.5Hz, $CH_2Ph$), 4.74 (d, 1H, J 12Hz, $CH_2Ph$), 4.69 (d, 1H, J 11Hz, $CH_2Ph$), 4.67 (d, 1H, J 8Hz, H—1'), 4.58 (dd, 1H, J 11 and 2.5Hz, H—3'), 4.50 (d, 1H, J 12Hz, $CH_2Ph$), 4.43 (d, 1H, J 8Hz, H—1), 4.59—4.41 (3H, *inter alia* H—1 and $PhCH_2$), 4.30—4.04 (4H), 3.98—3.36 (12H), 2.12, 2.07, 2.05, 1.98, 1.95, 1.93 (7s, each 3H MeCO). $^{13}C$ ($CDCl_3$, $Me_4Si$) δ 170.7, 170.6, 170.4, 170.13, 170.1, 169.5, 168.9, 139.3, 138.31, 137.9, 128.5, 128.4, 128.24, 128.16, 127.93, 127.86, 127.64, 127.58, 127.0, 126.9, 103.6 (d, *J*=158Hz, C—1), 100.6 (d, *J*=156Hz, C—1'), 99.8 (d, *J*=172Hz, C—1'') 82.6 81.5, 77.5 (2C), 75.0 ($CH_2$), 74.9 ($CH_2$), 74.6, 73.6 ($CH_2$), 73.0, 71.7, 69.7 ($CH_2$), 69.3 68.5, 67.8, 67.7 ($CH_2$), 67.3, 67.0, 61.3 ($CH_2$), 60.2 ($CH_2$), 30.2 ($CH_2Br$), 21.0, 20.73, 20.66, 20.5 (MeCO).

Anal. Calc. for $C_{55}H_{67}O_{23}Br$: C, 56.16; H, 5.74.
Found: C, 55.90; H, 5.72.

2-Bromoethyl 4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (10)

Catalytic hydrogenation (atm. pressure, 10% Pd/C, 0.2 g) of 9 (1.05 g, 0.89 mmol) in acetic acid (50 mL) for 2 h, filtration and solvent removal gave 10 (0.81 g, 100%). Recrystallisation from methanol gave an analytical sample with m.p. 178—180°, $[\alpha]_D^{21}$ +77° (*c* 1.0 chloroform). N.m.r. data: $^1H$ ($CDCl_3$, $Me_4Si$) δ 5.59 (bd, 1H, *J*~3Hz, H—4''), 5.38 (dd, 1H, *J* 11 and 3Hz, H—3''), 5.24 (dd, 1H, *J* 11 and 8Hz, H—2'), 5.22 (dd, 1H, *J* 11 and ~3Hz, H—2''), 4.97 (d, 1H, *J* 3.5Hz, H—1''), 4.81 (dd, 1H, *J* 11 and 3.5Hz, H—3'), 4.67 (d, *J* 8Hz, H1'), 4.44 (d, 1H, *J* 8Hz, H—1), 4.57—4.34 (3H, *inter alia* H—1), 4.30—3.37 (15H), 2.15, 2.11 (2s, each 6H, MeCO), 2.08, 2.05, 2.00 (3s, each 3H, MeCO). $^{13}C$ ($CDCl_3$, $Me_4Si$) δ 170.6, 170.5 (2C), 170.4, 170.1, 169.9, 169.1, 102.6, 101.8 (C—1 and C—1'), 99.6 (C—1''), 80.8, 77.1, 74.5, 74.2, 73.2, 72.6 (2C), 69.8, 68.5, 68.3, 67.7, 67.3, 67.2, 62.4, 60.7, 60.4, 30.0 ($CH_2Br$), 20.8, 20.6.

Anal. Calc. for $C_{34}H_{49}O_{23}Br$: C, 45.09; H, 5.45.
Found: C, 45.22; H, 5.22.

2-Bromoethyl 2,3,6-tri-*O*-acetyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (11)

(*a*) A solution of 10 (6.65 g, 7.3 mmol) in acetic anhydridepyridine (1:1, 132 mL) was left at room temperature for 15 h and then co-concentrated with toluene. Crystallisation of the residue from abs. ethanol gave 11 (5.22 g, 69%) which had m.p. 179—181°, $[\alpha]^{21}$ +45° (*c* 1.6, chloroform). N.m.r. data: $^1H$ ($CDCl_3$, $Me_4Si$) δ 5.59 (bdd, 1H, *J*~3 and <1Hz, H—4''), 5.40 (dd, 1H, *J* 11 and 3Hz, H—3''), 5.22 (t, 1H, *J*~9Hz, H—3), 5.18 (dd, 1H, *J* 11 and 3.5Hz, H—2''), 5.11 (dd, 1H, *J* 11 and 8Hz, H—2'), 4.99 (d, 1H, *J* 3.5Hz, H—1''), 4.92 (dd, 1H, *J* 9.5 and 8Hz, H—2), 4.74 (dd, 1H, *J* 11 and 2.5Hz, H—3'), 4.56 (d, 1H, *J* 8Hz, H—1), 4.53 (d, 1H, *J* 8Hz, H—1'), 4.50—4.39 (5H), 4.23—4.05 (5H), 4.02 (bd, 1H, *J*~2.5Hz, H—4'), 3.90—3.73 (3H), 3.65 (q, 1H, *J* 9.5 and 2Hz, H—5), 3.46 (bt, 2H, *J*~6Hz, $CH_2Br$), 2.14, 2.13, 2.09, 2.07, 2.07, 2.06, 2.05, 1.99 (8s, each 3H MeCO). $^{13}C$ ($CDCl_3$, $Me_4Si$) δ 170.7, 170.44 (3C), 170.39, 170.1, 169.8, 169.6, 169.5, 168.8, 101.1 (d, *J* 165Hz, C—1'), 100.7 (d, *J* 165Hz, C—1), 99.6 (d, *J* 172Hz, C—1''), 76.9 (C—4'), 76.4 (C—4), 72.9 (C—3), 72.8 (C—3'), 72.7

(C—5), 71.8 (C—5'), 71.5 (C—2), 69.8 (O—$CH_2$—$CH_2$), 69.0 (C—2'), 68.8 (C—2''), 67.9 (C—4''), 67.14 (C—3''), 67.07 (C—5''), 62.1 (C—6), 61.3 (C—6'), 60.3 (C—6''), 20.9, 20.86, 20.8, 20.7, 20.6, 20.5.

Anal. Calcd. for $C_{40}H_{55}O_{26}Br$: C, 46.56; H, 5.37.
Found: C, 46.79; H, 5.39.

(*b*) Catalytic hydrogenation (atm. pressure, 10% Pd/C, 53 mg) of 12 (218 mg, 0.18 mmol) in acetic acid (12 mL) for 1.5 h, filtration and solvent removal gave a quantitative yield of 2-bromoethyl 2,3-di-*O*-benzoyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (13). Recrystallisation from methanol gave an analytical sample with m.p. 218—220°, $[\alpha]_D^{21}$ +69° (*c* 0.7, chloroform). N.m.r. data: $^1H$ ($CDCl_3$, $Me_4Si$) δ 8.10—7.92 (4H), 7.60—7.28 (6H), 5.72 (t, 1H, *J* 9.5Hz, H—3), 5.59 (bd, 1H, *J*~3Hz, H—4''), 5.35 (dd, 1H, *J* 9.5 and 8Hz, H—2), 5.29 (dd, 1H, *J* 11 and 3Hz, H—3''), 5.12 (dd, 1H, *J* 11 and 8Hz, H—2'), 4.98 (dd, 1H, *J* 11 and 3.5Hz, H—2''), 4.87 (d, 1H, *J* 3.5Hz, H—1''), 4.80 (d, 1H, *J* 8Hz, H—1), 4.66 (dd, 1H, *J* 11 and 3Hz, H—3'), 4.66 (d, 1H, *J* 8Hz, H—1'), 4.45 (bt, *J*~6.5Hz, H—5''), 4.28—3.79 (8H), 3.75—3.49 (3H), 3.48—3.31 (3H), 3.39 (t, 2H, J 6.5Hz, $CH_2Br$), 2.12, 2.07, 2.044, 2.04, 2.01, 1.93, 1.91 (7s, each 3H, MeCO).

Anal. Calc. for $C_{48}H_{57}O_{25}Br$: C, 51.76; H, 5.16.
Found: C, 51.93; H, 5.14.

Deacylation with methanolic sodium methoxide (0.02 M, 20 mL) for 24 h, neutralisation with Duolite C—26 ($H^+$) resin, and concentration gave a residue that was acetylated (acetic anhydride-pyridine, 1:1, 50 mL) at room temperature for 22 h. Co-concentration with toluene gave a residue that was subjected to column (1 × 14 cm) chromatography ($SiO_2$, ethyl acetate-isooctane, 2:1) to give 11 (80 mg, 43% from 12). Recrystallisation from abs. ethanol gave pure 11, as characterised by melting point, optical rotation and $^1H$-n.m.r. data.

2-Bromoethyl 2,3-di-*O*-benzoyl-6-*O*-benzyl-4-*O*-[2,3,6-tri-*O*-acetyl-4-*O*-(2,3,4,6-tetra-*O*-acetyl-α-*D*-galactopyranosyl)-α- and β-*D*-galactopyranosyl]-β-*D*-glucopyranoside (14 and 12).

A solution of 1[29] (2.7 g, 4 mmol) in dichloromethane (10 mL) was added at −78° to a solution of 8 (5.85 g, 10 mmol), silver trifluoromethanesulfonate (3.08 g, 12 mmol), and tetramethylurea (14.0 g, 12 mmol) in dichloromethane (15 mL). The mixture was treated as for the preparation of 9. Chromatography ($SiO_2$, ethyl acetate-isooctane 2:1) gave 14 (1.59 g, 34%) and a later fraction containing 12 (0.52 g, 11%). Recrystallisation of 12 from abs. ethanol gave an analytical sample.

Compound 14 was amorphous with $[\alpha]_D^{21}$ +122° (*c* 1.3, chloroform). N.m.r. data: $^1H$ ($CDCl_3$, $Me_4Si$) δ 7.98—7.84 (4H), 7.58—7.30 (10H), 5.73 (t, 1H, *J* 9Hz, H—3), 5.51 (bd, 1H, *J*~2.5Hz, H—4''), 5.40—5.25 (3H), 5.19 (dd, 1H, *J* 11 and 3.5Hz, H—2''), 5.16 (dd, 1H, *J* 11 and 3.5Hz, H—2'), 5.02 (dd, 1H, *J* 11 and 2.5Hz, H—3'), 4.90 (d, 1H, *J* 3.5Hz, H—1''), 4.79 (d, 1H, *J* 8Hz, H—1), 4.74, 4.62 (ABq, 2H, $J_{AB}$ 12.5Hz, $CH_2$—Ph), 4.50—3.72 (13H), 3.42 (bt, 2H, *J* 7Hz, $CH_2Br$), 2.12, 2.11, 2.04, 2.02, 1.97, 1.96, 1.75 (7s, each 3H, MeCO). $^{13}C$ ($CDCl_3$, $Me_4Si$) δ 170.5, 170.4, 170.2, 170.15, 170.1, 169.9, 169.5, 165.6, 165.2, 137.8, 133.4, 133.1, 129.7 (2C), 129.6 (2C), 129.2, 129.0, 128.5 (4C), 128.2 (2C), 127.8, 127.6 (2C), 100.8 (d, *J* 164Hz, C—1), 99.5 (d, *J* 172Hz, C—1''), 97.0 (d, *J* 175Hz, C—1'), 78.1, 74.9, 74.8, 74.1, 73.3 ($CH_2$), 71.8, 69.5 ($CH_2$), 69.2, 68.8, 68.7 ($CH_2$), 68.3, 67.7, 67.3, 66.8, 66.3, 62.3 ($CH_2$), 60.3 ($CH_2$), 29.6 ($CH_2Br$), 20.9, 20.7, 20.67, 20.6 (3C), 20.2.

Compound 12 had m.p. 189—192°, $[\alpha]_D^{21}$ +65° (*c* 0.5, chloroform). N.m.r. data: $^1H$ ($CDCl_3$, $Me_4Si$) δ 8.07—7.91 (4H), 7.58—7.25 (11H), 5.65 (t, 1H, *J* 9.5Hz, H—3), 5.58 (bd, 1H, *J* 3Hz, H—4''), 5.41 (dd, 1H, *J* 9.5 and 8Hz, H—2), 5.24 (dd, 1H, *J* 11 and 3Hz, H—3''), 5.06 (dd, 1H, *J* 11 and 3.5Hz, H—2''), 5.04 (dd, 1H, *J* 11 and 8Hz, H—2'), 4.85 (d, *J* 3.5Hz, H—1''), 4.78, 4.56 (ABq, 2H, $J_{AB}$, 2H, $J_{AB}$ 12Hz, $CH_2$—Ph), 4.73 (d, 1H, *J* 8Hz, H—1), 4.52 (dd, 1H, *J* 11 and 2.5Hz, H—3'), 4.49 (d, 1H, *J* 8Hz, H—1'), 4.41 (bt, 1H, *J* 7Hz, H—5''), 4.30—4.00 (5H), 3.96—3.54 (6H), 3.50—3.30, (3H), 3.41 (bt, 2H, J 7Hz, $CH_2Br$), 2.11, 2.07, 2.04, 2.02, 1.97, 1.93, 1.88 (7s, each 3H, MeCO). $^{13}C$ ($CDCl_3$, $Me_4Si$) δ 170.7, 170.6, 170.5, 170.1, 170.0. 169.4, 168.6, 165.3, 164,9, 137.8, 133.1, 132.7, 129.8—127.9, 101.2, 100.7 (2d, *J* 161 and 160Hz, C—1 and C—1'), 99.4 (d, *J* 172Hz, C—1''), 77.2, 77.17, 76.0, 75.0, 73.8, 73.1, 72.9, 71.7, 71.6, 69.5, 68.8, 68.7, 67.8, 67.3, 66.9, 60.8, 60.3, 29.6 ($CH_2Br$), 21.0—20.5.

Anal. Calc. for $C_{55}H_{63}O_{25}Br$: C, 54.86; H, 5.27.
Found: C, 54.84; H, 5.28.

2-(2-Methoxycarbonylethylthio)ethyl 2,3,6-tri-*O*-acetyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (15).

A mixture of 11 (3.09 g, 3 mmol), methyl 3-mercaptopropionate[32] (0.72 g, 6 mmol), cesium carbonate (1.2 g, 3.7 mmol), and *N,N*-dimethylformamide (15 mL) was stirred at room temperature for 1.5 h and then partitioned between dichloromethane (100 mL) and water (25 mL). The organic phase was washed with water (20 mL), dried ($Na_2SO_4$) and concentrated. Chromatography ($SiO_2$, ethyl acetate-isooctane, 2:1) gave 15 (2.81 g, 87%) as an amorphous solid with $[\alpha]_D^{21}$ +36° (*c* 1.1 chloroform). N.m.r. data: $^1H$ ($CDCl_3$, $Me_4Si$) δ 5.59 (dd, 1H, J 3 and 1Hz, H—4''), 5.40 (dd, 1H, *J* 11 and 3Hz, H—3''), 5.21 (t, 1H, *J* 9Hz, H—3), 5.18 (dd, 1H, *J* 11 and 3.5Hz, H—2''), 5.11 (dd, 1H, *J* 11 and 8Hz, H—2'), 4.99 (d, 1H, *J* 3.5Hz, H—1''), 4.90 (dd, 1H, *J* 9.5 and 8Hz, H—2), 4.73 (dd, 1H, *J* 11 and 2.5Hz, H—3'), 4,53 (d, 2H, *J* 8Hz, H—1 and H—1'), 3.71 (s, 3H, MeO), 2.81 and 2.62 (2 bt, each 2H, *J*~7Hz, S—$CH_2$—$CH_2$CO), 2.71 (t, 2H, *J* 7Hz, $CH_2$—S), 2.14, 2.13, 2.09. 2.08, (4s, each 3H), 2.07 (s, 6H), 2.06. 2.06, 2.05, 1.99 (4s, each 3H).

$^{13}C$ ($CDCl_3$, $Me_4Si$) δ 172.1, 170.5, 170.3 (3C), 170.2, 169.9, 169.5, 169.4, 169.3, 168.7, 100.9, 100.4 (2d, *J*

164Hz, C—1 and C—1′), 99.4 (d, *J* 174Hz, C—1″), 76.8, 76.2, 72.8, 72.6, 72.4, 71.6, 71.4, 69.5 ($CH_2$), 68.8, 68.6, 67.7, 66.9, 66.88, 62.0 ($CH_2$), 61.2 ($CH_2$), 60.1 ($CH_2$), 51.6 (MeO), 34.5 ($CH_2$), 31.2 ($CH_2$), 27.1 ($CH_2$), 20.8, 20.7, 20.6, 20.54 (2C), 20.5 (2C), 20.47, 20.4, 20.3.

2-(2-Methoxycarbonylethylthio)ethyl 4-*O*-[4-*O*-(α-*D*-galactopyranosyl)-β-*D*-galactopyranosyl]-β-*D*-glucopyranoside (16)

A solution of 15 (1.07 g, 1.0 mmol) in methanolic sodium methoxide (0.003 M, 40 mL) was left at room temperature for 42 h. The reaction was monitored by t.l.c. ($SiO_2$, chloroform-methanol-water 65:35:10, lower phase). Crystalline 16 was filtered off, washed with methanol and dried to give pure 16 (412 mg). The filtrate was neutralised with Duolite® C 26 ($H^+$) resin and the solvent was removed. The residue was crystallised from methanol to give a second crop of 16 (182 mg) as needles (total yield of 16: 594 mg, 97%) with m.p. 115—118°, $[\alpha]_D^{21}$ +54° (*c* 0.8, water). N.m.r. data: $^1H$ (DMSO-$d_6$, 50°, $D_2O$ added, $Me_4Si$) δ 4.82 4.29, 4.26 (3d, each 1H, *J* 3.5, 7 and 8Hz, H—1″, H—1, H—1′), 3.62 (s, 3H, MeO). $^{13}C$ ($D_2O$, TSP) δ 178.0, 106.0, 104.8 (2d, *J* 163 and 160Hz, C—1, C—1′), 103.0 (d, *J* 170Hz, C—1″), 81.3, 80.1, 78.2, 77.6, 77.1, 75.6, 74.9, 73.6, 73.5, 71.8, 71.7 ($CH_2$), 71.66, 71.3, 63.2 ($CH_2$), 63.1 ($CH_2$), 62.8 ($CH_2$), 55.0 (MeO), 36.9 ($CH_2$), 33.5 ($CH_2$), 29.2 ($CH_2$).

| | | |
|---|---|---|
| Anal. Calc. for $C_{24}H_{42}O_{18}S$: | C, 44.30; | H, 6.50. |
| Found: | C, 43.41; | H, 6.71. |

2-(Octadecylthio)ethyl 2,3,6-tri-*O*-acetyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (17)

A mixture of 11 (650 mg, 0.63 mmol), octadecylthiol (360 mg, 1.26 mmol), cesium carbonate (250 mg, 0.77 mmol) and *N,N*-dimethylformamide (3 mL) was stirred at room temperature for 78 h. The reaction was monitored by t.l.c. ($SiO_2$, ethyl acetate-isooctane, 3:1). The mixture was diluted with dichloromethane (50 mL), washed with water (2 × 25 mL), dried ($Na_2SO_4$), and concentrated. Column (5 × 18 cm) chromatography ($SiO_2$, toluene and toluene-ethyl acetate 1:1) gave amorphous 17 (677 mg, 87%) with $[\alpha]_D^{21}$ +33° (*c* 1.0, chloroform). N.m.r. data: $^1H$ ($CDCl_3$, $Me_4Si$) δ 5.59 (dd, 1H, *J* 3 and 1Hz, H—4″), 5.40 (dd, 1H, *J* 11 and 3Hz, H—3″), 5.22 (t, *J* 9Hz, H—3), 5.18 (dd, 1H, *J* 11 and 3.5Hz, H—2″), 5.11 (dd, 1H, *J* 11 and 8Hz, H—2′), 4.99 (d, 1H, *J* 3.5Hz, H—1″), 4.90 (dd, 1H, *J* 9.5 and 8Hz, H—2), 4.73 (dd, 1H, *J* 11 and 2.5Hz, H—3′), 4.52 (d, 2H, *J* 8Hz, H—1 and H—1′), 4.58—4.39 (5H, *inter alia* H—1 and H—1′), 4.25—3.48 (10H), 2.68, 2.51 (2t, each 2H, *J* 7Hz, $CH_2$—S—$CH_2$), 2.13, 2.12, 2.08, 2.08, 2.07, 2.07, 2.06, 2.054, 2.049, 1.99 (10s, each 3H, MeCO), 1.65—1.02 (32H), 0.88 (t, 3H, *J* 6.5Hz, $CH_3$—$CH_2$). $^{13}C$ ($CDCl_3$, $Me_4Si$) δ 170.7, 170.5 (3C), 170.1, 169.7, 169.6, 169.5, 168.9, 101.1, 100.6 (2d, *J* 164 and 162 Hz, C—1, C—1′), 99.6 (d, *J* 175Hz, C—1″), 76.9, 76.5, 73.1, 72.8, 72.6, 71.8, 71.6, 69.8 ($CH_2$), 68.9, 68.8, 67.9, 67.1, 67.0, 62.2 ($CH_2$), 61.3 ($CH_2$), 60.3 ($CH_2$), 32.6—22.7 ($CH_2$), 21.0—20.5 ($CH_3CO$), 14.1 ($CH_3$).

2-(Octadecylthio)ethyl 4-*O*-[4-*O*-(α-*D*-galactopyranosyl)-β-*D*-galactopyranosyl]-β-*D*-glucopyranoside (18)

A solution of 17 (1.0 g, 0.81 mmol) in methanolic sodium methoxide (0.003 M, 100 mL) was left at room temperature for 24 h and neutralised with Duolite® C—26 ($H^+$) resin. The solvent was removed and the residue was dissolved in water (300 mL) and lyophilised to give 18 (580 mg, 88%) with $[\alpha]_D^{21}$ +42° (*c* 0.7, dimethylsulfoxide). N.m.r. data: $^1H$ (DMSO-$d_6$, 50°, $D_2O$ added, $Me_4Si$) δ 4.81 (d, 1H, *J* 3.5Hz, H—1″), 4.27, 4.24 (2d, each 1H, *J* 7 and 8Hz, H—1 and H—1′), 2.67, 2.52 (2t, each 2H, *J* 7.5Hz, $CH_2$—S—$CH_2$), 0.86 (t, 3H, *J* 6.5Hz, Me). $^{13}C$ ($CDCl_3$—$CD_3OD$ 1:5) δ 105.2, 104.1 (2d, *J* 162Hz, C—1, C—1′), 102.6 (d, *J* 172Hz, C—1″), 81.1, 80.0, 76.3 (3C), 74.6 (2C), 72.9, 72.5, 71.1, 70.9, 70.4, 70.3 ($CH_2$), 62.7 ($CH_2$), 62.0 ($CH_2$), 61.3 ($CH_2$), 33.2—29.7 ($CH_2$), 23.5 ($CH_2$), 14.4 ($CH_3$).

Ethyl 2,3,6-tri-*O*-acetyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (19)

A solution of 11 (0.52 g, 0.5 mmol) in methanolic sodium methoxide (0.007 M, 40 mL) was left at room temperature for 2 h and sodium hydroxide (95 mg) in water (2 mL) was added. The mixture was hydrogenated (atm. pressure, Pd/C, 10%, 100 mg) for 100 min, filtered, neutralised with *M* hydrochloric acid and the solvent was removed. The residue was acetylated (acetic anhydride-pyridine 1:1, 100 mL) for 17 h at room temperature and then co-concentrated with toluene. The residue was partitioned between dichloromethane (100 mL) and water (25 mL) and the organic phase was dried ($Na_2SO_4$) and concentrated. Column (5 × 18 cm) chromatography ($SiO_2$, ethyl acetate-isooctane 2:1) gave amorphous 19 (300 mg, 62%) with $[\alpha]_D^{21}$ +41° (*c* 1.0 chloroform). N.m.r. data: $^1H$ ($CDCl_3$, $Me_4Si$) δ 5.91 (d, 1H, *J* 3Hz, H—4″), 5.40 (dd, 1H, *J* 11 and 3Hz, H—3″), 5.21 (t, 1H, *J* 9Hz, H—3), 5.18 (dd, 1H, *J* 11 and 3.5Hz, H—2″), 5.11 (dd, 1H, *J* 11 and 8Hz, H—2′), 4.99 (d, 1H, *J* 3.5Hz, H—1″), 4.88 (dd, 1H, *J* 9 and 8Hz, H—2), 4.73 (dd, 1H, *J* 11 and 2.5Hz, H—3′), 4.56—4.38 (5H, *inter alia* H—1 and H—1′), 4.23—4.03 (4H), 4.02 (d, 1H, *J* 2.5Hz, H—4′), 3.98—3.70 (3H), 3.69—3.46 (2H), 2.14—1.98 (MeCO), 1.19 (t, 3H, *J* 7Hz, $CH_3$—$CH_2$). $^{13}C$ ($CDCl_3$, $Me_4Si$) δ 170.4, 170.3 (4C), 169.9, 169.6, 169.5, 169.4, 168.7, 100.9, 100.1 (2d, *J* 160 and 164Hz, C—1, C—1′), 99.4 (d, *J* 172Hz, C—1″), 76.8, 76.3, 73.0, 72.6, 72.3, 71.6 (2C), 68.8, 68.6, 67.7, 66.9, 66.9, 65.4 ($CH_2$), 62.1 ($CH_2$), 61.2 ($CH_2$), 60.1 ($CH_2$), 20.8, 20.7, 20.54 (3C), 20.5 (2C), 20.46, 20.4, 20.3, 14.9.

Ethyl 4-*O*-[4-*O*-(α-*D*-galactopyranosyl)-β-*D*-galactopyranosyl]-β-*D*-glucopyranoside (20)

A solution of 19 (440 mg, 0.46 mmol) in methanolic sodium methoxide (0.005 M, 40 mL) was left at room temperature for 18 h, then neutralised with Duolite C 26 ($H^+$) resin and the solvent was removed. The residue was dissolved in water and lyophilised to give 20 (245 mg, 100%) with $[\alpha]_D^{21}$ +71° (*c* 0.5, water). N.m.r. data: $^1H$ (DMSO-$d_6$, 50°, $D_2O$ added, $Me_4Si$) δ 4.81 (d, 1H, *J* 3.5Hz, H—1''), 4.28, 4.20 (d, each 1H, *J* 7 and 8Hz, H—1 and H—1'), 1.14 (t, 3H, *J* 7Hz, $CH_3$—$CH_2$). $^{13}C$ ($D_2O$, TSP) δ 106.1, 104.5, 103.2 (C—1, 1', 1''), 81.5, 80.2, 78.3, 77.7, 77.3, 75.8, 75.0, 73.7, 73.6, 72.0, 71.8, 71.4, 69.1 ($CH_2$), 63.3 ($CH_2$), 63.2 ($CH_2$), 62.9 ($CH_2$), 17.1.

Sugar[15] and methylation[16] analysis was in agreement with the proposed structure.

Neo-glycoprotein 21

A solution of 16 (44 mg, 0.07 mmol) and hydrazine hydrate (85%, 0.25 mL) in ethanol (2 mL) was left over-night, concentrated, dissolved in water and lyophilised. The resulting hydrazide was dissolved in dimethylsulfoxide (1 mL), then hydrogen chloride in dioxane (4 *M*, 105 μL) and t-butylnitrite (18 μL, 0.15 mmol) in dimethylsulfoxide (0.1 mL) were added. The mixture was stirred at room temperature for 30 min and sulfamic acid (10 mg, 0.11 mmol) in dimethylsulfoxide (0.1 mL) was added. After 15 min, the reaction mixture was added dropwise and with stirring to a solution of bovine serum albumin (BSA, 65 mg, 1 μmol) in sodium tetraborate-potassium hydrogencarbonate buffer (2.5 mL, 0.08 *M* $Na_2B_4O_7$ and 0.35 *M* $KHCO_3$). The pH was maintained at 9.0—9.3 by additions of sodium hydroxide solution (1 *M*). The mixture was stirred for 16 h at room temperature, dialysed ($H_2O$, 72 h) and lyophilised to give 21. The degree of binding (number of hapten molecules per molecule of protein) was 14 as determined by the phenol-sulfuric acid method[16]. With 0.35 mmol of 16 and 1 μmol of BSA, the degree of binding was 43.

Neo-glycoprotein 22

The above procedure was followed, using 16 (114 mg, 0.18 mmol) and the protein key-hole limpet haemocyanin (KLH, 30 mg, 0.034 μmol). This gave 22 with a degree of binding of 480, determined as above.

2,3,6 - Tri - *O* - acetyl - 6,6 - di - deuterio - 4 - *O* - (2,3,4,6 - tetra - *O* - acetyl - 6,6 - di - deuterio - α - *D* - galactopyranosyl) - α - *D* - galactopyranosyl - 1 - bromide (23) was prepared from 1,2,3,6 - tetra - *O* - acetyl - 6,6 - di - deuterio - 4 - *O* - (2,3,4,6 - tetra - *O* - acetyl - 6,6 - di - deuterio - α - *D* - galactopyranosyl) - α - *D* - galactopyranose [prepared from "digalacturonic acid" as described[5] for the protio compound except for the use of sodium borodeuteride in the reduction step. M.p. 153—155°, $[\alpha]_D^{21}$ +138° (*c* 0.9, chloroform). N.m.r. data were as expected.] by the same method as for 1 and used directly in the next step.

2 - Bromoethyl 2,3,6 - tri - *O* - benzyl - 4 - *O* - [2,3,6 - tri - *O* - acetyl - 6,6 - di - deuterio - 4 - *O* - (2,3,4,6 - tetra - *O* - acetyl - 6,6 - di - deuterio - α - *D* - galactopyranosyl] - β - *D* - galactopyranosyl] - β - *D* - glucopyranoside (24) was prepared from 23 and 6 as described for 9. 24 had m.p. 155—157°, $[\alpha]_D^{21}$ +58° (*c* 0.7, chloroform) and n.m.r. data as expected.

2 - Bromoethyl 4 - *O* - [2,3,6 - tri - *O* - acetyl - 6,6 - di - deuterio - 4 - *O* - (2,3,4,6 - tetra - *O* - acetyl - 6,6 - di - deuterio - α - *D* - galactopyranosyl) - β - *D* - galactopyranosyl) - β - *D* - glucopyranoside (25) was prepared from 24 as described for 10. 25 had m.p. 177—179°, $[\alpha]_D^{21}$ +78° (*c* 0.5, chloroform) and n.m.r. data as expected.

2 - Bromoethyl 2,3,6 - tri - *O* - acetyl - 4 - *O* - [2,3,6 - tri - *O* - acetyl - 6,6 - di - deuterio - 4 - *O* - (2,3,4,6 - tetra - *O* - acetyl - 6,6 - di - deuterio - α - *D* - galactopyranosyl) - β - *D* - galactopyranosyl] - β - *D* - glucopyranoside (26) was prepared from 25 as described for 11. 26 had m.p. 181—183°, $[\alpha]_D^{21}$ +44° (*c* 0.9, chloroform) and n.m.r. data as expected.

## Example 49

2-Bromoethyl glycosides · Synthesis from pullulan of spacer-arm, lipid and ethyl glycosides of a tetrasaccharide found in human urine; Preparation of neo-glycoproteions

Summary of the Example

Enzymic hydrolysis of pullulan, followed by acetylation and chromatography, gave α-*D*-Glc*p*-(1→6)-α-*D*-Glc*p*-(1→4)-α-*D*-Glc*p*-(1→4)-*D*-Glc*p* in fully acetylated form. Treatment with 2-bromoethanol and boron trifluoride etherate in dichloromethane gave the corresponding 2-bromoethyl glycoside which was converted to spacer-arm glycosides with methyl 3-mercaptopropionate and methyl 11-mercaptoundecanoate, a neo-glycolipid with octadecanethiol, and ethyl glycoside by hydrogenolysis of the bromine-carbon bond. The mercaptopropionate-derived spacer-arm glycoside was coupled to bovine serum albumin (BSA), and key-hole limpet hasemocyanine (KLH) thus furnishing the corresponding neo-glycoproteins.

Introduction

The tetrasaccharide α-*D*-Glc*p*-(1→6)-α-*D*-Glc*p*-(1→4)-α-*D*-Glc*p*-(1→4)-*D*-Glc$_9$ (1) is normally excreted in human urine[40]. Increased excretion is observed in patients with type II and type III glycogenosis[41], in Duchenne muscular dystrophy[42] and during normal pregnancy[43]. Rapid detection of 1 can be done by radioimmunoassay using antibodies raised against a neo-glycoprotein that was obtained by reductive

amination of 1 (isolated from urine[40]) with p-amino-phenylethylamine followed by coupling to key-hole limpet haemocyanine (KLH) and bovine serum albumine (BSA).

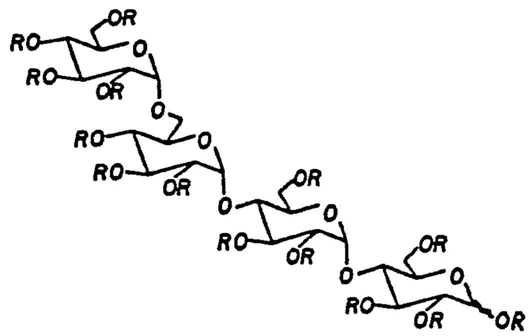

1: R = H

2: R = Ac

Since reductive amination transforms the reducing end of 1 into a glucitol derivative, it became of interest to prepare a spacer-arm glycoside with all four glucose units intact. We now report the preparation of 1 and its transformation, *via* the corresponding 2-bromoethyl glycoside 3, into different derivatives (4—12) of value for the preparation and evaluation of antibodies (Scheme 1 below).

Pullulan → 1 → 2 →

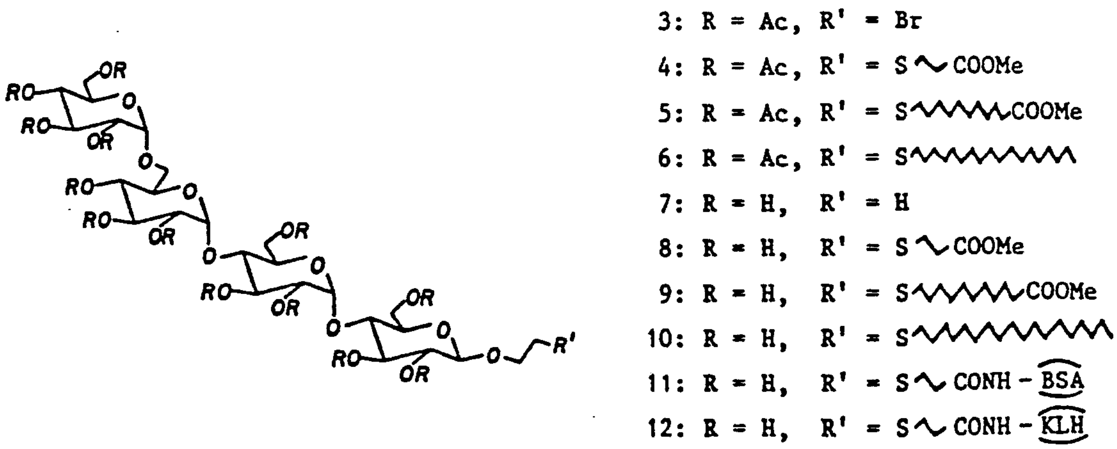


Scheme 1

Results and Discussion

Enzymatic hydrolysis[44] (β-amylase and pullulanase) of pullulan[45], followed by acetylation of the crude product and silica gel chromatography gave 2 in 12% over-all yield (αβ-ratio, ca 1:1). The structure of 1 was confirmed by methylation analysis and comparison of the m.s.-data with those of the authentic material[41]. Further structural support was obtained by n.m.r. analysis of 2—10 (see Experimental). This also confirmed the anomeric configurations in 1 as proposed by Hallgren et al[40].

The 2-bromoethyl glycoside 3 was prepared by boron-trifluoride etherate induced glycosidation[9] of the αβ-mixture 2 with 2-bromoethanol in dichloromethane. This gave the β-glycoside; the α-anomer could not be detected in the reaction mixture. Treatment of 3 with methyl 3-mercaptopropionate, methyl 11-mercaptoundecanoate (15) octadecylthiol and hydrogen-Pd/C gave spacer-arm glycosides 4 and 5, neoglycolipid 6 and ethyl glycoside 7 respectively. Deacetylation of 4, 5 and 6 gave 8, 9 and 10. These transformations are analogous to those described earlier [1,10].

The neo-glycoproteins 11 and 12 were prepared by coupling of 8 to BSA and KLH. A modification of the Inman[15]-Lemieux[14]-technique was used (see Experimental), where N,N-dimethylformamide was substituted by dimethylsulfoxide, due to the low solubility of 8 in the former solvent. The degree of binding (number of hapten molecules per molecule of protein) of 8 to BSA and KLH was 20 and 245 respectively.

Experimental

Pullulan was prepared as described[45] and purified by two precipitations from water by addition of isopropanol (200 g pullulan, 4 L water and 13 L isopropanol). Pullulanase and β-amylase are commercially available (Sigma Chemical Company). Optical rotations were obtained using a Perkin-Elmer 241 polarimeter. N.m.r. spectra were recorded on a Varian XL-200 spectrometer with tetramethylsilane (TMS) or sodium 3-(trimethylsilyl)-propionate-$d_4$ (TSP) as reference, N.m.r. assignments are based on double resonance and INEPT experiments.

4-*O*-{4-*O*-[6-*O*-(α-*D*-glucopyranosyl)-α-*D*-glucopyranosyl]-α-*D*-glucopyranosyl}-*D*-glucose (1)

Pullulane[45] (100 g) was dissolved in water (1 L) by heating and added to a solution of acetic acid (ca 15 mL) and sodium acetate (41 g) in water (5 L). The solution was adjusted to pH 5 and pullulanase and β-amylase (200 and 200000 units respectively) were added. The reaction mixture was stirred at 37° for 24 h (t.l.c.: $SiO_2$, acetone-isopropanol-0.1 M lactic acid; 2:2:1) and then heated at 120° for 20 min. (autoclave). The solution was concentrated (final volume: 15 L) and treated with ion-exchange resin (Duolite® C26H$^+$; 0.5 L). The resin was washed with water and the combined water solutions (ca 4 L) were ultrafiltered (Millipore PSAC 14205 NMWL 1000) which gave a colourless solution. Freeze-drying gave 1 (72 g) contaminated with glucose, maltose and maltotriose. Pure 1 was obtained by treating 2 (1.0 g) with methanolic sodium methoxide (50 mL; from ~1 mg of sodium) at room temperature while monitoring the reaction by t.l.c. ($SiO_2$, chloroform-methanol-water 65:35:10, lower phase). After 24 h, the reaction mixture was neutralised with dry Duolite® H$^+$ resin, filtered, concentrated and chromatographed (Waters Preppak-500/$C_{18}$:water) to give 1 (337 mg, 63% > 99.5% pure by HPLC). $[\alpha]_D^{25}$ + 165° (*c* 0.8, water). Lit.[2] $[\alpha]_D^{20}$ + 179° (*c* 1.14, water). N.m.r. data: $^1H$ ($D_2O$, TSP) δ 5.40, 5.38 (2d, each 1H, J3.8 and 3.5Hz, H—1′ and H—1″), 5.26 (d, ~ 0.3H, J3.7Hz, H—1), 4.99 (d, 1H, J3.7Hz, H—1‴), 4.68 (d, ~0.7H, J7.9Hz, H—1). $^{13}C$ ($D_2O$, TSP) δ 102.6, 102.2, 100.8 (C—1′, C—1″, C—1‴), 98.5, 94.7 (C—1), 79.9, 79.8, 79.6, 78.9, 77.3, 76.7, 76.0, 75.97, 75.8, 74.7, 74.6, 74.4, 74.3, 74.2, 74.1, 73.9, 72.7, 72.3, 72.1, 68.6, 63.4, 63.3, 63.2.

The structure of 1 was confirmed by methylation analysis[41].

1,2,3,6-tetra-*O*-acetyl-4-*O*-{2,3,6-tri-*O*-acetyl-4-*O*-[2,3,4-tri-*O*-acetyl-6-*O*-(2,3,4,6-tetra-*O*-acetyl-α-*D*-glucopyranosyl)-α-*D*-glucopyranosyl]-α-*D*-glucopyranosyl}-β-*D*-glucopyranose (2).

Compound 1 (63.7 g, *vide supra*) was dissolved in acetic anhydride-pyridine (1:2; 450 mL) and left at room temperature for 15 h. The mixture was co-concentrated with toluene and the acetylation procedure was repeated twice. The residue was dissolved in dichloromethane and washed with hydrochloric acid (1 M) and saturated, aqueous sodium hydrogencarbonate. Drying ($Na_2SO_4$) and evaporation gave a residue (112.5 g) which was chromatographed on silica gel (ethyl acetat-isooctane, 3:2) to give 2 (αβ-mixture 20.0 g, 12% from pullulan). β-2 (> 98% purity) had $[\alpha]_D$ + 114° (*c* 0.2, chloroform). N.m.r. data: $^1H$ ($CDCl_3$, $Me_4Si$) δ 6.25, 5.75 (2d, each ~ 0.5H, J3.6 and 8.0Hz, H—1). $^{13}C$ ($CDCl_3$, $Me_4Si$) δ 95.8, 95.8, 95.2 (C—1′, C—1″, C—1‴), 91.2 (C—1), 75.0, 73.5, 73.4, 73.0, 72.8, 72.6, 72.0, 71.7, 71.6, 70.9, 70.7, 70.4, 70.1, 69.9, 69.7, 69.4, 69.3, 69.0, 68.4, 67.4, 65.3, 62.8, 62.7, 62.7, 61.8, 20.9—20.3.

2-Bromoethyl 2,3,6-tri-*O*-acetyl-4-*O*-{2,3,6-tri-*O*-acetyl-4-*O*-[2,3,4-tri-*O*-acetyl-6-*O*-(2,3,4,6-tetra-*O*-acetyl-α-*D*-glucopyranosyl)-α-*D*-glucopyranosyl]-α-*D*-glucopyranosyl}-β-*D*-glucopyranoside (3).

Compound 2 (12.54 g, 9.85 mmol) and 2-bromoethanol (2.5 g, 1.4 mL, 20 mmol) were dissolved in dichloromethane (50 mL). Borontrifluoride etherate (7.1 g, 6.3 mL. 50 mmol) was added dropwise at room temperature and the mixture was stirred until the reaction was finished (48 h, t.l.c.: $SiO_2$, ethyl acetate-isooctane 3:1). The mixture was washed (water, saturated sodium hydrogen carbonate), dried ($Na_2SO_4$) and evaporated. The residue (12.3 g) was chromatographed ($SiO_2$ ethyl acetate-isooctane 3:2) to give 3 (4.95 g, 63% based on transformed 2) and unreacted 2 (5.05 g) 3 had $[\alpha]_D^{25}$ + 109° (*c* 1.3, chloroform). N.m.r. data: $^1H$ ($CDCl_3$, $Me_4Si$) δ 5.35, 5.28, 5.18 (3 d, each 1H, J3.8, 2.8 and 3.6Hz, H—1′, H—1″ and H—1‴), 4.60 (d, 1H, J7.9Hz, H—1), 3.42—3.49 (m, 2H, $CH_2$-Br). $^{13}C$ ($CDCl_3$, TMS) δ 170.6—169.3 (CO), 100.4 (C—1), 95.9, 95.7, 95.3 (C—1′, C—1″, C—1‴), 77.3, 75.0, 73.7, 72.7, 72.2, 71.9, 71.8, 70.7, 70.4, 70.1, 69.9, 69.8, 69.7, 69.4, 69.3, 68.9, 68.5, 68.4, 67.4, 63.0, 61.8, 29.9, 21.1—20.6.

2-(2-Methoxycarbonylethylthio)ethyl 2,3,6-tri-*O*-acetyl-4-*O*-{2,3,6-tri-*O*-acetyl-4-*O*-[2,3,4-tri-*O*-acetyl-6-*O*-(2,3,4,6-tetra-*O*-acetyl-α-*D*-glucopyranosyl)-α-*D*-glucopyranosyl]-α-*D*-glucopyranosyl}-β-*D*-glucopyranoside (4).

Compound 3 (4.00 g, 3.0 mmol), methyl 3-mercaptopropionate (0.546 g, 0.49 mL, 4.5 mmol), cesium carbonate (1.17 g, 3.6 mmol) and N,N-dimethylformamide (25 mL) were stirred at room temperature until 3 had been consumed (7 h; t.l.c. $SiO_2$, ethyl acetate-isooctane 4:1). The solvent was removed and the residue was chromatographed ($SiO_2$, ethyl acetate-isooctane 3:1) to give 4 (3.22 g, 78%). $[\alpha]_D^{25}$ + 102° (*c* 1.0, chloroform). N.m.r. data: $^1H$ ($CDCl_3$, $Me_4Si$) δ 5.36, 5.28, 5.19 (3 d, each 1H, J3.8, 4.1 and 3.7Hz, H—1′, H—1″, H—1‴), 4.57 (d, 1H, J7.9Hz, H—1), 3.71 (s, 3H, MeO), 2.81, 2.71, 2.61 (3t, each 2H, J6.7Hz, $CH_2$—S—$CH_2$ and $CH_2$—CO). $^{13}C$ ($CDCl_3$, $Me_4Si$) δ 100.2 (C—1), 95.8, 95.6, 95.2, (C—1′, C—1″, C—1‴), 75.1, 73.7, 72.6, 72.0, 71.9, 71.7, 70.7, 70.3, 70.0, 69.8, 69.6, 69.3, 69.2, 68.8, 68.4, 67.3, 65.2, 63.0, 62.9, 61.7, 60.3, 51.7, 34.6, 31.3, 27.2, 20.8—20.5.

2-(11-Methoxycarbonylundecylthio) ethyl 2,3,6-tri-*O*-acetyl-4-*O*-{2,3,6-tri-*O*-acetyl-4-*O*-[2,3,4-tri-*O*-acetyl-6-*O*-(2,3,4,6-tetra-*O*-acetyl-α-*D*-glucopyranosyl)-α-*D*-glucopyranosyl]-α-*D*-glucopyranosyl}-β-D-glucopyranoside (5)

Compound 3 (431 mg, 0.33 mmol), methyl 11-mercaptoundecanoate (15, 114 mg, 0.49 mmol), cesium carbonate (128 mg, 0.39 mmol) and N,N-dimethylformamide (3 mL) were stirred at room temperature for 15 h. The mixture was diluted with dichloromethane, washed with water (neutralisation with Duolite $H^+$ resin facilitated the phase separation), and dried ($Na_2SO_4$). The solvent was removed and the residue was chromatographed ($SiO_2$, ethyl acetate-isooctane 2:1) to give 5 (243 mg, 50%). $[\alpha]_D^{24}$ + 91° (*c* 4.4, chloroform). N.m.r. data: $^1H$ ($CDCl_3$, $Me_4Si$) δ 5.36, 5.28, 5.19 (3d, each 1H, J3.8, 4.1 and 3.6Hz, H—1', H—1'', H—1'''), 4.57 (d, 1H, J7.9Hz, H—1), 3.67 (s, 3H, MeO), 2.68, 2.52, 2.31 (3t, each 2H, J7Hz, $CH_2$—S—$CH_2$ and $CH_2CO$). $^{13}C$ ($CDCl_3$, $Me_4Si$) δ 174.1, 170.4—169.1, 100.1 (G—1), 95.6, 95.5, 95.1 (C—1', C—1'', C—1'''), 75.0, 73.6, 72.5, 71.9, 71.5, 70.5, 70.2, 69.9, 69.7, 69.6, 69.2, 69.1, 69.1, 69.1, 68.7, 68.3, 67.2, 65.1, 62.9, 62.8, 61.6, 51.2, 33.9, 32.3, 31.1, 29.5, 29.2, 29.1, 29.0, 29.0, 28.9, 28.6, 24.7, 20.7—20.4.

2-(Octadecylthio)ethyl 2,3,6-tri-*O*-acetyl-4-*O*-{2,3,6-tri-*O*-acetyl-4-*O*-[2,3,4-tri-*O*-acetyl-6-*O*-(2,3,4,6-tetra-*O*-acetyl-α-*D*-glucopyranosyl)-α-*D*-glucopyranosyl]-α-*D*-glucopyranosyl}-β-D-glucopyranoside (6).

Compound 3 (265 mg, 0.2 mmol), octadecanethiol (86 mg, 0.3 mmol), cesium carbonate (79 mg, 0.24 mmol) and N,N-dimethylformamide (10 mL) were stirred at room temperature until 3 had been consumed (48 h; t.l.c., $SiO_2$, ethyl acetate-isooctane 4:1). The solvent was removed and the residue was chromatographed ($SiO_2$/ethyl acetate-isooctane 1:1) to give 6 (142 mg, 44%). $[\alpha]_D^{25}$ + 93° (*c* 0.5, chloroform). N.m.r. data: $^1H$ ($CDCl_3$, $Me_4Si$) δ 5.30, 5.22, 5.12 (3d, each 1H, J4.0, 4.2 and 3.7Hz, H—1', H—1'' and H—1'''), 4.51 (d, 1H, J7.9Hz, H—1), 2.62, 2.46 (2t, each 2H, J7.0Hz, $CH_2$—S—$CH_2$). $^{13}C$ ($CDCl_3$, $Me_4Si$) δ 170.5—169.1 (CO), 100.2 (C—1), 95.7, 95.6, 95.2 (C—1', C—1'', C—1'''), 75.1, 73.7, 72.6, 72.0, 71.6, 70.6, 70.3, 70.0, 69.8, 69.7, 69.6, 69.3, 69.2, 68.8, 68.4, 68.3, 67.3, 65.2, 63.0, 62.9, 61.7, 32.4, 31.8, 31.2, 29.6, 29.5, 29.5, 29.4, 29.2, 29.1, 28.7, 22.6, 20.8—20.5, 14.0.

Ethyl 4-O-{4-O-[6-O-(α-*D*-glucopyranosyl)-α-*D*-glucopyranosyl]-α-*D*-glucopyranosyl}-β-*D*-glucopyranoside (7).

Compound 3 (122 mg) was dissolved in methanol (10 mL) and methanolic sodium methoxide (5 mL; from 1 mg of sodium) was added. Sodium hydroxide solution (0.1 *M*, 5 mL) was added and the reaction mixture was hydrogenated (72 mg Pd/C; 4 atm.) over night and then neutralised with Duolite® $H^+$ resin and filtered. One drop of conc. ammonium hydroxide in water was added and the solvent was removed. The residue (106 mg) was chromatographed ($SiO_2$, chloroform-methanol-water, 65:35:10, lower phase) to give 7 (24 mg, 40%). $[\alpha]_D^{24}$ + 108° (*c* 1.0, water) N.m.r. data: $^1H$ ($D_2O$, TSP) δ 5.40 (d, 2H, J3.8Hz, H—1' and H—1''), 4.96 (d, 1H, J3.7Hz, H—1'''), 4.49 (d, 1H, J8.0Hz, H—1), 1.23 (t, 3H, J7.1Hz, $CH_2CH_3$). $^{13}C$ ($D_2O$, TSP), δ 104.5 (C—1), 102.7, 102.2, 100.8 (C—1', C—1'', C—1'''), 79.8, 79.7, 79.0, 77.3, 76.0, 75.8, 75.8, 74.6, 74.5, 74.4, 74.2, 74.1, 73.9, 72.3, 72.2, 69.0, 68.6, 63.5, 63.2, 17.0.

2-(2-Methoxycarbonylethylthio)ethyl 4-*O*-{4-*O*-[6-*O*-(α-*D*-glucopyranosyl)-α-*D*-glucopyranosyl]-α-*D*-glucopyranosyl}-β-*D*-glucopyranoside (8).

Compound 4 (3.0 g, 2.2 mmol) was dissolved in methanol (25 mL) and added to a solution of sodium (1 mg) in methanol (75 ml). The reaction mixture was monitored by t.l.c. (chloroform-methanol-water 65:35:10, lower phase). After 9 h at room temperature and 30 min. at 40°', the solvent was removed and the residue was chromatographed ($SiO_2$, chloroform-methanol-water, 65:35:10, lower phase) to give 8 (1.50 g, 84%). $[\alpha]_D^{25}$ + 113° (*c* 0.7, water). N.m.r. data: $^1H$ ($D_2O$, TSP) δ 5.41 (d, 2H, J3.5Hz, H—1' and H—1''), 4.98 (d, 1H, J3.6Hz, H—1'''), 4.53 (d, 1H, J7.9Hz, H—1), 3.75 (s, 3H, MeO). $^{13}C$ ($D_2O$, TSP) δ 177.4 (CO), 104.3 (C—1), 102.0, 101.6, 100.2, (C—1', C—1'', C—1'''). 79.3, 79.1, 78.3, 76.7, 75.4, 75.2, 75.1, 73.9, 73.8, 73.6, 73.5, 73.3, 71.6, 71.5, 71.1, 68.0, 62.8, 62.7, 62.6, 62.6, 54.5, 36.3, 32.9, 28.6.

2-(11-Methoxycarbonylundecylthio)ethyl 4-*O*-[6-*O*-(α-*D*-glucopyranosyl)-α-*D*-glucopyranosyl]-α-*D*-glucopyranosyl}-β-*D*-glucopyranoside (9).

Compound 5 (218 mg, 0.148 mmol) was deacetylated and purified essentially as above and freeze-dried to give 9 (100 mg, 73%). $[\alpha]_D^{25}$ + 94° (*c* 0.8, water). N.m.r. data: $^1H$ ($D_2O$, TSP, 70°) δ 5.31 (m, 2H, H—1' and H—1''), 4.93 (d, 1H, J3.6Hz, H—1'''), 4.41 (d, 1H, J7.9Hz, H—1), 3.64 (s, 3H, MeO), 2.76, 2.56 (2t, 2H each, *J*7.0Hz, $CH_2$—S—$CH_2$). 2.28 (t, 2H, *J*7.3Hz, $CH_2$—CO). $^{13}C$ ($D_2O$, TSP, 65°) δ 178.0 (CO), 105.9, 103.6, 103.4, 101.6 (C—1, C—1', C—1'', C—1'''), 81.5, 81.4, 79.5, 78.2, 76.6, 76.4, 75.3, 75.0, 74.9, 74.8, 73.2, 72.9, 72.4, 69.5, 64.2, 54.8, 37.2, 35.4, 34.6, 32.9, 32.6, 32.5, 32.34, 32.3, 32.2, 31.9, 28.0.

2-(Octadecylthio)ethyl 4-*O*-{4-*O*-[6-*O*-(α-*D*-glucopyranosyl)-α-*D*-glucopyranosyl]-α-*D*-glucopyranosyl}-β-*D*-glucopyranoside (10).

Compound 6 (120 mg, 0.079 mmol) was deacetylated essentially as above and freeze dried to give 10 (73 mg, 95%). The reaction was monitored by t.l.c. ($SiO_2$, acetic acid-methanol-water 2:1:1). $[\alpha]_D^{24}$ + 77° (*c* 0.6, chloroform-methanol 1:1). N.m.r. data: $^1H$ ($D_2O$, TSP) δ 5.35, 5.33 (2d, each 1H, J3.9Hz, H—1' and H—1''), 4.96 (d, 1H, J3.5Hz, H—1'''), 4.44 (d, 1H, J7.9Hz, H—1), 2.80, 2.58 (2t, each 2H, J7.0Hz, $CH_2$—S—$CH_2$). $^{13}C$ ($CDCl_3$, $(CD_3)_2SO$ $Me_4Si$) δ 102.8, 101.7, 100.8, 98.8 (C—1, C—1', C—1'', C—1'''), 81.5, 81.4, 80.1, 76.1,

73.3, 73.1, 72.8, 72.3, 72.2, 72.1. 72.0, 71.7, 70.2, 70.1, 68.7, 60.8, 31.7, 31.4, 30.8, 29.3, 29.1, 28.8, 28.3, 22.2, 14.0.

*Neoglycoprotein* 11 was prepared using 8 (56 mg, 0.07 mmol) and bovine serum albumine (BSA, 65 mg, 1 μmol). The degree of binding was 8. With 0.07 mmol of BSA, the degree of binding was 20.

*Neoglycoprotein* 12 was prepared, using 8 (112 mg, 0.14 mmol) and key-hole limpet haemocyanin (KLH, 30 mg, 0.034 mmol; assumed molecular weight, 870 000; supplier, Schwarts/Mann, Spring Valley, New York). The degree of binding was 65. With 0.7 mmol of 8 and 0.034 mmol of KLH, the degree of binding was 245.

*Methyl 11-bromoundecanoate* (13). 11-Bromoundecanoic acid (commercial material, 25.0 g, 94 mmol), Duolite® $H^+$ resin (10 g), calcium chloride (26.2 g, 94 mmol) and methanol (700 mL) were stirred for 15 h. The methanol was removed and dichloromethane was added. The mixture was washed with water, dried ($Na_2SO_4$) and concentrated. Distillation gave pure 13 (22.0 g, 84%) with b.p. 103—104° (2,7 Pa). $^1$H—N.m.r. data: ($CDCl_3$, $Me_4Si$) δ 3.67 (s, 3H, MeO), 3.41 (t, 2H, J6.8Hz, Br—$CH_2$), 2.31 (t, 2H, J7.3Hz, $CH_2CO$).

*Methyl 11-thioacetylundecanoate* (14). Compound 13 (17.8 g, 64 mmol), thioacetic acid (9.7 g, 9.0 mL, 128 mmol), methyltrioctylammonium chloride (0.5 g), cesium carbonate (25 g, 77 mmol), dichloromethane (95 mL) and water 95 mL were stirred at room temperature for 15 h. The reaction was monitored by t.l.c. ($SiO_2$, ethyl acetate-isooctane, 1:5). The mixture was extracted with dichloromethane and the combined extracts were dried ($Na_2SO_4$) and concentrated. Distillation gave pure 14 (15.6 g, 89%) with b.p. 137—139° (13,3 Pa) and m.p. < 25° (from isooctane). N.m.r. data: $^1$H ($CDCl_3$, $Me_4Si$) δ 3.67 (s, 3H, MeO), 2.86 (t, 2H, J7.1Hz, $CH_2CO$). $^{13}$C ($CDCl_3$, $Me_4Si$) δ 195.9, 174.1 (CO), 51.3, 34.0, 30.5, 29.4, 29.3, 29.2, 29.1, 29.0, 28.9, 28.7, 24.8.

Anal. Calc. for $C_{14}H_{26}O_3S$: C, 61.27; H, 9.50. Found: C, 60.80; H, 9.79.

*Methyl 11-mercaptoundecanoate* (15). Compound 14 (15.6 g, 56.9 mmol) in methanol (50 mL) was added to a solution of hydrogen chloride in methanol (HCl was bubbled into 600 mL of MeOH for 10 min.). The reaction was monitored by t.l.c. ($SiO_2$, ethyl acetate-isooctane 1:5). After 48 h, an excess of sodium hydrogencarbonate was added and the mixture was diluted with dichloromethane (150 mL), filtered and concentrated. Distillation gave pure 15 (9.0 g, 68%) with b.p. 93—94° (7—$10^{-2}$ torr). N.m.r. data: $^1$H ($CDCl_3$, $Me_4Si$) δ 3.68 (s, 3H, MeO), 2.53 (q, 2H, J~7Hz, H—S—$CH_2$), 2.31 (t, 2H, J7.5Hz, $CH_2CO$). $^{14}$C ($CDCl_3$, $Me_4Si$) δ 174.2 (CO), 51.3, 34.0, 33.9, 29.3, 29.2, 29.1, 29.0, 28.9, 28.3, 24.8, 24.5.

## Example 50

### 2-Bromoethyl glycosides · synthesis of spacer-arm, lipid and ethyl glycosides of the terminal trisaccharide portion of the blood-group $P_1$ antigen. Preparation of neo-glycoproteins.

Summary of the Example

The title compounds were prepared *via* the correspondiong 2-bromoethyl glycoside 5 by displacement of bromide ion with methyl 3-mercaptopropionate, octadecylthiol and hydrogen, respectively. Silver triflate promoted glycosylation of 2-bromoethyl 3,6-di-*O*-benzyl-2-deoxy-2-phthalimido β-*D*-glucopyranoside (2) with 2,3,6-tri-*O*-acetyl-4-*O*-(2,3,4,6-tetra-*O*-acetyl-α-*D*-galactopyranosyl)-α-*D*-galactopyranosyl bromide (1) gave 5. The spacer-arm glycoside 9 was coupled to bovine serum albumin and key-hole limpet haemocyanin, thus furnishing the corresponding neo-glycoproteins.

Introduction

Carbohydrates of the P blood-group systems[48] have been suggested to be specific receptors for uropathogenic E. coli bacteria[24,26]. Synthetic studies on these and related compounds have lead to the preparation of glycosides of the oligosaccharides α-*D*-Gal-(1→4)-β-*D*-Gal[49], α-*D*-Gal-(1→4)-β-*D*-Gal-(1→4)-β-*D*-Glc[50], α-*D*-Gal-(1→4)-β-*D*-Gal-(1→4)-β-*D*-GlcNAc[51] and β-*D*-GalNAc-(1→3)-α-*D*-Gal-(1→4)-β-*D*-Gal-(1→4)-β-*D*-Glc[52].

We now report a new synthesis of the terminal trisaccharide portion of the $P_1$ antigen in the form of its 2-bromoethyl glycoside (5) followed by transformations into spacer-arm, lipid and ethyl glycosides[53] as well as into neo-glycoproteins. The synthesis is based on the acetobromo sugar 1 which is readily available from pectin *via* an enzymatic hydrolysis-borohydride reduction sequence[49g].

Results and Discussion

The over-all reaction sequence is depicted in Fig. 8. The partly protected 2-bromoethyl glycoside 2 was glycosylated with acetobromo sugar 1,[49]g, using silver triflate and tetramethyl urea in dichloromethane, to give the trisaccharide glycoside 3 (41%). The benzyl protecting groups of 3 were removed by hydrogenolysis in acetic acid[53], to give 4 (92%). Acetylation of 4 gave the key intermediate 5 (72%), which was used for the preparation of 6, 7 and 14. Treatment of 5 with methyl 3-mercaptopropionate or octadecanethiol in N,N-dimethylformamide, using cesium carbonate as promoter[53], gave spacer-arm glycoside 6 (94%) and neoglycolipid 7 (91%) respectively. Hydrogenation of 5 under basic reaction conditions[53], followed by N-deprotection and reacetylation gave the ethyl glycoside 14 (28%). It should be noted that hydrogenation under acidic reaction conditions cleaves benzyl groups while leaving the 2-bromoethyl group intact (e.g. 3→4) whereas basic reaction conditions cleaves the carbon-bromine bond without affecting benzyl groups (e.g. 3→12).

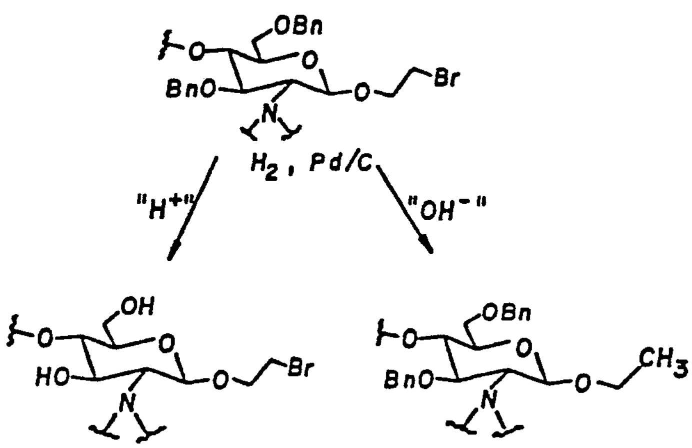


N-deprotection and reacetylation of 6 and 7 gave 8 (50%) and 10 (52%) respectively. O-deacetylation of 8, 10, and 14 gave spacer-arm glycoside 9 (90%), neo-glycolipid 11 (87%) and ethyl glycoside 15 (60%) respectively. The latter was also prepared from 3 *via* 12 and 13.

The spacer-arm glycoside 9 was coupled to the proteins bovine serum albumin (BSA) and key-hole limpet haemocyanin (KLH), which gave the neo-glycoproteins 16 and 17. The degree of binding [53a] was 17 and 420 respectively.

Experimental

General methods were as reported[53c]. Tetramethylsilane ($Me_4Si$) or sodium 3-(trimethylsilyl)-propionate-$d_4$ (TSP) were used as n.m.r. references.

2-bromoethyl 3,6-di-*O*-benzyl-2-deoxy-2-phthalimido-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (3).

Compound 2 (4.56 g, 7.65 mmol), tetramethylurea (1.74 g, 15 mmol) and silver trifluoromethanesulfonate (3.5 g, 13.62 mmol) were dissolved in dichloromethane (30 mL) and cooled (0°). 1 (8.25 g, 11.8 mmol) in dichloromethane (13 mL) was added with stirring. The mixture was protected from light, stirred at 0° for 2h and at room temperature for 22h. Solids were removed by filtration through. Celite and the filtrate was diluted with dichloromethane, washed with *M* hydrochloric acid and aqueous sodium hydrogencarbonate, dried ($Na_2SO_4$) and concentrated. The residue (12 g) was chromatographed ($SiO_2$-column, 5 × 18 cm, toluene-ether 1:2) to give 3 (3.8 g, 41%). Crystallisation from methanol gave material (2.81 g) with m.p. 98—101°, $[\alpha]_D^{21}$ +65° (*c* 0.7, chloroform). N.m.r. data: $^1H$ ($CDCl_3$, $Me_4Si$) δ *inter alia* 7.85—7.60 (bs, 4H), 7.50—7.30 (5H), 6.94—6.79 (3H), 5.54 (bd, 1H, *J*3Hz, H—4''), 5.26 (dd, 1H, *J*11 and 3Hz, H—3''), 5.17—5.15 (3H, H—1, H—2' and H—2''), 4.94 (d, 1H, *J*3Hz, H—1''), 4.90 (d, 1H, *J*12.5Hz, $PhCH_2$), 4.80 (d, 1H, *J*12Hz, $PhCH_2$), 4.67 (dd, 1H, *J*10.5 and 3Hz, H—3'), 4.64 (d, 1H, *J*8Hz, H—1'), 4.52 (d, 1H, *J*12Hz, $PhCH_2$), 4.44 (d, 1H, *J*12.5Hz, $PhCH_2$), 3.29 (bt, 2H, *J*~6Hz, $CH_2Br$), 2.10, 2.09, 2.05, 2.02, 2.01, 1.91 and 1.82 (6s, each 3H, MeCO). $^{13}C$ ($CDCl_3$, $Me_4Si$) δ 170.5, 170.4, 170.3, 170.2, 170.0, 169.3, 168.8, 168.0, 128.5—123.1, 100.5 (d, *J*164Hz), 99.3 (d, *J*172Hz, C—1''), 98.4 (d, *J*163Hz), 78.3, 77.1, 76.9, 74.7, 74.5 ($CH_2$), 73.5 ($CH_2$), 72.7, 71.6, 69.2 (2C, $CH_2$), 68.2, 67.7, 67.4 ($CH_2$), 67.1, 66.9, 61.2 ($CH_2$), 60.2 ($CH_2$), 55.4 (C—2), 29.9 ($CH_2Br$), 20.9, 20.6 (3C), 20.5 (2C), 20.3.

Anal. Calc. for $C_{56}H_{65}BrNO_{24}$: C, 55.31; H, 5.39. Found: C, 55.22; H, 5.41.

2-Bromoethyl-2-deoxy-2-phthalimido-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (4).

Compound 3 (5.0 g, 4.1 mmol) was hydrogenated (10% Pd/C, 1.0 g, 2 atm.) in acetic acid (50 mL) for 17h and the mixture was filtered, concentrated and dried (oilpump) to give 4 (3.9 g, 92%) as a colourless, amorphous solid with $[\alpha]_D^{21}$ +49° (*c* 1.5, chloroform). N.m.r. data: $^1H$ ($CDCl_3$, $Me_4Si$) δ *inter alia* 7.96—7.81 (2H), 7.80—7.68 (2H), 5.58 (dd, 1H, *J*3 and 1Hz, H—4''), 5.39 (d, 1H, *J*8.5Hz, H—1), 5.34 (dd, 1H, *J* 11 and 3Hz, H—3''), 5.25 (dd, 1H, *J*11 and 8Hz, H—2'), 5.19 (dd, 1H, *J*11 and 3.5Hz, H—2''), 4.93 (d, 1H, *J*3.5Hz, H—1''), 4.79 (dd, 1H, *J*11 and 2.5Hz, H—3'), 4.68 (d, 1H, *J*8Hz, H—1'), 3.34 (t, 2H, *J*6Hz, $CH_2Br$), 2.13 (6H), 2.10, 2.08, 2.05, 1.97 and 1.86. $^{13}C$ ($CDCl_3$, $Me_4Si$) δ 170.6, 170.5 (2C), 170.4, 170.1, 169.8, 169.1, 168.0 (broad, 2C), 134.0 (2C), 131.7 (2C), 123.3 (2C), 101.9 (d, *J*~167Hz, C—1 or C—1'), 99.8 (d, *J*174Hz, C—1''), 98.4 (d, *J*~163Hz, C—1 or C—1'), 82.2, 77.3, 74.2, 72.7, 72.5, 69.7, 69.6, 68.43, 68.38, 67.7, 67.4, 67.2, 62.5 ($CH_2$), 60.8 ($CH_2$), 60.4 ($CH_2$), 55.6 (C—2), 29.9 ($CH_2Br$), 20.9 (2C), 20.8, 20.6 (3C), 20.2.

2-Bromoethyl 3,6-di-*O*-acetyl-2-deoxy-2-phthalimido-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (5).

Compound 4 (3.76 g, 3.6 mmol) was acetylated (acetic anhydride-pyridine 1:1, 50 mL) at room temperature for 18 h and then co-evaporated with toluene. Crystallisation from ethanol gave 5 (3.64 g, 90%) with m.p. 227—229°, $[\alpha]_D^{21}$ + 50° (*c* 0.8, chloroform). N.m.r. data: $^1H$ ($CDCl^1$, $Me_4Si$) δ 7.92—7.81 (2H), 7.80—7.64 (2H), 5.76 (dd, 1H, *J*10.5 and 8Hz, H—3), 5.59 (bdd, 1H, *J*3 and 1Hz, H—4''), 5.44 (d, 1H, *J*8.5Hz, H—1), 5.38 (dd, 1H, *J*11 and 3Hz, H—3''), 5.17 (dd, 1H, *J*11 and 3.5Hz, H—2''), 5.13 (dd, 1H, *J*11 and 8Hz, H—2'), 4.98 (d, 1H, *J*3.5Hz, H—1''), 4.74 (dd, 1H, *J*11 and 2.5Hz, H—3'), 4.58 (d, 1H, *J*8Hz, H—1'), 4.64—3.70 (15H, *inter alia* H—1'), 3.34 (bt, 2H, *J*~6Hz, $CH_2Br$), 2.15, 2.12, 2.07 (9H), 2.06 (6H), 2.04, 1.96. $^{13}C$ ($CDCl_3$, $Me_4Si$) δ 170.7, 170.52, 170.48 (2C), 170.4, 170.1, 169.7, 169.5, 168.9, 167.9 (broad, 2C), 134.2 (2C), 131.6, 131.5, 123.5 (2C), 101.0 (d, *J*160Hz, C—1'), 99.6 (d, *J*171Hz, C—1''), 98.1 (d, *J*166Hz, C—1), 76.9, 76.8, 72.7, 72.6, 71.6, 71.4, 69.8 ($CH_2CH_2Br$), 69.0, 68.8, 67.9, 67.1 (2C), 62.2 ($CH_2$), 61.1 ($CH_2$), 60.2 ($CH_2$), 54.7 (C—2), 29.8 ($CH_2Br$), 21.0, 20.9, 20.8, 20.7 (3C), 20.6, 20.5, 20.4.

Anal. Calc. for $C_{46}H_{56}BrNO_{26}$: C, 49.38; H, 5.05. Found: C, 49.50; H, 5.08.

2-(2-Methoxycarbonylethylthio)ethyl 3,6-di-*O*-acetyl-2-deoxy-2-phthalimido-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*glucopyranoside (6).

Compound 5 (3.91 g, 3.5 mmol), methyl 3-mercaptopropionate[54] (0.84 g, 7.0 mmol), cesium carbonate (1.30 g, 4.0 mmol) and N,N-dimethylformamide (15 mL) were stirred at room temperature for 2.5h. The reaction was monitored by t.l.c. ($SiO_2$, ethyl acetate-isooctane, 3:1). Dichloromethane (100 mL) was added and the mixture was washed with water (50 mL). The aqueous phase was extracted with dichloromethane (25 mL) and the combined organic phases were washed with water (20 mL), dried ($Na_2SO_4$) and concentrated. The residue was crystallised from methanol to give 6 (3.80 g, 94%) with m.p. 151—153° (phase transition starting at 118°), $[\alpha]_D^{21}$ +52° (c 0.7, chloroform). N.m.r. data: $^1H$ ($CDCl_3$, $Me_4Si$) δ *inter alia* 7.96—7.74 (4H), 5.74 (m, 1H, H—3), 5.58 (dd, 1H, *J* 3 and 1Hz, H—4''), 5.42 (d, 1H, *J*8.5Hz, H—1), 5.38 (dd, 1H, *J*11 and 3.5Hz, H—3''), 5.17 (dd, 1H, *J*11 and 3.5Hz, H—2''), 5.13 (dd, 1H, *J*11 and 8Hz, H—2'), 4.98 (d, 1H, *J*3.5Hz, H—1''), 4.74 (dd, 1H, *J*11 and 2.5Hz, H—3'), 4.59 (d, 1H, *J*8Hz, H—1'), 3.67 (s, 3H, $CH_30$), 2.70—2.56 (4H, $CH_2S$), 2.42 (bt, 2H, *J*~7Hz, $CH_2S$), 2.15, 2.12, 2.07 (6H), 2.064, 2.059, 2.04, 1.96 and 1.95. $^{13}C$ ($CDCl_3$ $Me_4Si$): δ 172.1, 170.7, 170.5 (2C), 170.4, 170.0, 169.7, 169.5, 168.9, 167.8 (2C), 134.2 (2C), 134.15 (2C), 123.5 (2C), 101.0 (C—1' or C—1), 99.6 (C—1''), 98.0 (C—1 or C—1'), 77.0, 76.8, 72.7, 72.6, 71.6, 71.5, 69.5 ($OCH_2CH_2$), 69.0, 68.8, 67.9, 67.0, 62.3 ($CH_2$), 61.1 ($CH_2$), 60.2 ($CH_2$), 54.8 (C—2), 51.8 ($OCH_3$), 34.5 ($CH_2$), 31.3 ($CH_2$), 27.1 ($CH_2$), 21.0, 20.9, 20.74, 20.69, 20.66, 20.64, 20.6, 20.5, 20.4.

Anal. Calc. for $C_{50}H_{63}NO_{28}S$: C, 51.05; H, 5.48. Found: C, 51.70; H, 5.41.

2-(Octadecylthio)ethyl 3,6-di-*O*-acetyl-2-deoxy-2-phthalimido-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (7).

Compound 5 (1.68 g, 1.5 mmol), octadecanethiol (0.81 g, 2.8 mmol), cesium carbonate (0.50 g, 1.53 mmol) and N,N-dimethylformamid (11 mL) were stirred at room temperature for 7.5 h and worked-up as above for 6. The resulting residue was chromatographed ($SiO_2$-column, 5×18 cm; ethyl acetate-isooctane 2:1 followed by 4:1 and finally ethyl acetate) to give 7 (1.80 g, 91%). Crystallisation from methanol gave an analytical sample with m.p. 150—152°, $[\alpha]_D^{21}$ +47° (C 0—6, chloroform). N.m.r. data: $^1H$ ($CDCl_3$, $Me_4Si$) δ 7.93—7.72 (4H), 5.75 (m, 1H, H—3), 5.58 (bd, 1H, *J*~3Hz, H—4''), 5.42 (d, 1H, *J*8Hz, H—1), 5.38 (dd, 1H, *J*11 and 3.5Hz, H—2''), 5.16 (dd, 1H, *J*11 och 3.5Hz, H—3''), 5.13 (dd, 1H, *J*11 and 8Hz, H—2'), 4.98 (d, 1H, *J*3.5Hz, H—1''), 4.74 (dd, 1H, *J*11 and 2.5Hz, H—3'), 4.63—4.34 (4H, *inter alia* H—1'), 4.58 (d, 1H, *J*8Hz, H—1'), 4.29—3.57 (11H), 2.55 (t, 2H, *J*7Hz, $CH_2S$), 2.31 (t, 2H, *J*7Hz, $CH_2S$), 2.15, 2.12, 2.07 (6H), 2.064, 2.058, 2.04, 1.96 and 1.95, 1.40—1.15 (bs), 0.88 (t, 3H, *J*6.5Hz, $CH_3$). $^{13}C$ ($CDCl_3$, $Me_4Si$) δ 170.7, 170.5 (3C), 170.4, 170.0, 169.7, 169.5, 168.9, 168.2 (broad, 2C), 134.2 (2C), 134.0 (2C), 123.5 (2C), 101.0 (d, *J*160Hz, C—1' or C—1), 99.6 (d, *J*173Hz, C—1''), 98.1 (d, *J*160Hz, C—1 or C—1'), 77.8, 72.7, 72.6, 71.62, 71.56, 69.7 ($OCH_2CH_2$), 69.0, 68.8, 67.9, 67.1 (2C), 62.4 ($CH_2$), 61.1 ($CH_2$), 60.2 ($CH_2$), 54.2 (C—2), 32.4—22.7 ($CH_2$), 21.0, 20.9, 20.74, 20.68 (2C), 20.64, 20.57, 20.51, 20.45, 14.14.

Anal. Calc. for $C_{64}H_{93}0_{26}S$: C, 58.03; H, 7.08. Found: C, 57.91; H, 7.02.

2-(Methoxycarbonylethylthio)ethyl 2-acetamido-2-deoxy-3,6-di-*O*-acetyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (8).

Compound 6 was treated as 7 in the preparation of 10. The crude 8 formed was chromatographed ($SiO_2$-column, ethyl acetate-isooctane 4:1 and ethyl acetate) to give pure 8 with $[\alpha]_D$ +33°. N.m.r. data: 1H ($CDCl_3$, $Me_4Si$) δ *inter alia* 5.86 (d, 1H, *J*9Hz, NH), 5.58 (dd, 1H, *J*3 and <1Hz, H—4''), 5.39 (dd, 1H, *J*11 and 3Hz, H—3''), 5.19 (dd, 1H, *J*11 and 3.5Hz, H—2''), 5.12 (dd, 1H, *J*11 and 8Hz, H—2'), 5.11 (t, 1H, *J*8.5Hz, H—3), 5.00 (d, 1H, *J*3.5Hz, H—1''), 4.76 (dd, 1H, *J*11 and 2.5Hz, H—3'), 4.60—4.38 (5H, *inter alia* H—1 and H—1'), 3.71 (S, $CH_30$), 2.82 (bt, 2H, *J*7Hz, $SCH_2CH_2C0$), 2.72 (t, 2H, *J*6.5Hz, $OCH_2CH_2$), 2.62 (bt, 2H, *J*7Hz, $SCH_2CH_2C0$), 2.13, 2.12, 2.11, 2.084, 2.075 (6H), 2.06, 2.05, 1.99 and 1.96 ($CH_3CO$). $^{13}C$ ($CDCl_3$ $Me_4Si$) δ 172.5—169.1, 101.0 (2C, C—1 and C—1'), 99.6 (C—1''), 77.0, 76.1, 72.9, 72.6, 71.9, 69.2 ($OCH_2CH_2$), 69.0, 68.7, 67.9, 67.1 (2C), 62.4 ($CH_2$), 61.4 ($CH_2$), 60.4 ($CH_2$), 53.4 (C—2), 51.9 ($CH_30$), 34.6 ($CH_2$), 31.5 ($CH_2$), 27.3 ($CH_2$), 23.2—20.5.

2-(2-Methoxycarbonylethylthio)ethyl 2-acetamido-2-deoxy-4-*O*-[4-*O*-(α-*D*-galactopyranosyl)-β-*D*-galactopyranosyl]-β-*D*-glucopyranoside (9).

Compound 8 (350 mg, 0.3 mmol) was dissolved in methanol (10 mL) by warming and then rapidly cooled. Methanolic sodium methoxide (0.2 *M*, 0.2 mL) was added and the mixture was stirred at room temperature for 18 h, neutralised (Duolite C—26 ($H^+$) resin), filtered and concentrated. The residue was dissolved in methanol (7 mL) and methanolic hydrazine hydrate (0.1 *M*, 3 mL) was added. The mixture was refluxed for 33 h while two additional portions (2×3 mL) were added (after 24 and 30 h). The reaction was monitored by t.l.c. ($SiO_2$, chloroform-methanol-water 65:35:10, lower phase). The solvent was removed and the residue was treated with a mixture of acetic anhydride (2.5 mL), ethanol (12 mL) and water (4 mL) at room temperature for 1 h. The ethanol was removed and the residue was partitioned between water (20 mL) and dichloromethane (5 mL). The aqueous phase was washed with dichloromethane (3×5 mL) and concentrated. The residue was chromatographed (Sephadex® G—10 column, 3×57 cm, water) to give 9 (157 mg, 75%) with $[\alpha]_D^{22}$ +34° (*c* 0.7, water) N.m.r. data: $^1H$ [$(CD_3)_2SO+D_2O$, 50°, $Me_4Si$] δ *inter alia* 4.80 (d, 1H, *J*3.5Hz, H—1''), 4.40 (d, 1H, *J*8Hz, H—1 or H—1'), 4.28 (d, 1H, *J*7Hz, H—1 or H—1'), 4.08 (dt, 1H, *J*6 and 1Hz), 3.61. (s, $CH_30$), 2.75—2.70 and 2.61—254 (m, each 2H, $SCH_2CH_2C0$), 2.64 (t, 2H, *J*7Hz, $OCH_2CH_2S$), 1.80 (s, 3H, MeCON). $^{13}C$ ($D_2O$, TSP) δ 178.0, 177.3, 106.1, 103.8, 103.1, 81.6, 80.1, 78.2, 77.7, 75.3, 75.0, 73.7, 73.6, 72.1 ($CH_2$), 72.0, 71.8, 71.4, 63.3 ($CH_2$), 63.2 ($CH_2$), 62.9 ($CH_2$), 58.0 (C—2), 55.2 ($CH_30$), 37.1 ($CH_2$), 33.8 ($CH_2$), 29.4 ($CH_2$), 23.2.

2-(Octadecylthio)ethyl 2-acetamido-2-deoxy-3,6-di-*O*-acetyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (10).

Compound 7 (1.46 g, 1.1 mmol) was dissolved in a mixture of methanol (75 mL) and tetrahydrofuran (10 mL) by warming. The clear solution was rapidly cooled to ~40°C and methanolic sodium methoxide (0.2 M, 5 mL) was added. The mixture was stirred at room temperature for 5 h, after which time the deacetylation was complete. (TLC; $SiO_2$, chloroform-methanol-water 65:35:10, lower phase), neutralised by addition of acidic ion-exchange resin (Duolite® C—26), filtered and evaporated. The residue was redissolved in refluxing methanol (50 mL) and hydrazine hydrate (0.5 g, 10 mmol) was added. The reflux was continued for 17½h, whereafter the solvent was evaporated. The residue was acetylated by stirring for 22 h at room temperature in a mixture of pyridine and acetic anhydride (1:1, 50mL). After evaporation, the residue was subjected to chromatography ($SiO_2$-column, 5x18 cm ethyl acetate-isooctane gradient: 2:1→4:1→ethyl acetate) which gave amorphous 10 (0.72 g, 52%) with $[\alpha]_D^{21}$ +32° (*c* 1.1, chloroform). N.m.r. data: $^1H$ ($CDCl_3$, $Me_4Si$) δ 5.67 (d, 1H, *J*9.5Hz, NH), 5.58 (bd, 1H, *J*3Hz, H—4''), 5.39 (dd, 1H, *J*11 and 3Hz, H—3''), 5.23—5.01 (3H, H—2', H—2'' and H—3), 4.99 (d, 1H, *J*3.5. H—1''), 4.75 (dd, 1H, *J*11 and 2.5Hz, H—3'), 4.58—4.37 (5H, *inter alia* H—1 and H—1'), 4.54 and 4.49 (2d, each 1H, *J*8Hz each, H—1 and H—1'), 4.23—3.90 (7H), 3.85—3.55 (4H), 2.69 (t, 2H, *J*7Hz, $OCH_2CH_2S$), 2.51 (t, 2H, *J*7Hz, $SCH_2$), 2.13, 2.12, 2.11 and 2.08 (4s, each 3H, MeC0), 2.075 and 2.05 (2s, each 6H, MeC0), 1.99 and 1.97 (2s, each 3H, MeC0), 1.66—1.20, 0.88 (t, 3H, *J*6.5Hz, $CH_3CH_2$). $^{13}C$ ($CDCl_3$) $Me_4Si$) δ 170.6 (2C), 170.4 (3C), 170.2, 170.1, 169.6, 169.0, 101.1 and 101.0 (d, *J* each 160Hz, C—1 and C—1'), 99.6 (d, *J*172Hz, C—1''), 76.9, 75.9, 72.7, 72.6 (2C), 71.8, 69.1 ($OCH_2CH_2$), 68.9, 68.7, 67.8, 67.1, 67.0, 62.4 ($CH_2$), 61.3 ($CH_2$), 60.2 ($CH_2$), 53.3 (C—2), 32.5—28.8, 23.3 ($CH_3CONH$), 22.7 ($CH_2$), 20.9—20.5, 14.1.

2-(Octadecylthio)ethyl 2-acetamido-2-deoxy-4-*O*-[4-*O*-(α-*D*-galactopyranosyl)-β-*D*-galactopyranosyl]-β-*D*-glucopyranoside (11).

Compound 10 (560 mg, 0.45 mmol) was dissolved in methanol (25 mL) and methanolic sodium methoxide (0.2 *M*, 1 mL) was added. The mixture was stirred at room temperature for 14.5 h, which gave an amorphous precipitate. This was dissolved by adding methanol (50 mL) and warming the mixture to ~40°. The solution was cooled to room temperature, neutralised with Duolite® C—26 ($H^+$) resin, filtered and concentrated. The residue was suspended in water (100 mL) and lyophilised to give amorphous 11 (340 mg, 87%) with $[\alpha]_D^{21}$ +35° (*c* 0.6, dimethyl sulfoxide). N.m.r. data: $^1H$ [$(CD_3)_2SO+D_20$, 50°, $Me_4Si$] δ *inter alia* 4.81 (d, 1H, *J*3.5Hz, H—1''), 4.40 and 4.29 (2d, each 1H, *J*7.5Hz, H—1 and H1'), 2.60 (t, 2H, *J*7Hz, $CH_2S$), 2.50 (t, 2H, *J*7Hz, $SCH_2$), 1.81 (s, 3H, $CH_3CON$), 0.86 (t, 3H, *J*6.5Hz, $CH_3CH_2$). $^{13}C$ ($(CD_3)_2SO$, $Me_4Si$) δ 168.7, 103.8, (d, *J*162Hz, C—1 or C—1'), 100.7 and 100.5 (2d, *J*~162 and 172Hz, C—1'' and C—1 or C—1'), 81.2, 77.1, 75.0 (2C), 72.8, 72.1, 71.0, 70.8, 69.1, 68.7, 68.5, 68.5 ($OCH_2CH_2$), 60.3 ($2CH_2$), 59.3 ($CH_2$), 54.5, 31.4—28.1 ($CH_2$), 22.9, 22.1 ($CH_2$), 13.9 ($CH_3CH_2$).

Ethyl-2-acetamido-2-deoxy-3,6-di-*O*-benzyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (12).

Compound 3 (310 mg, 0.26 mmol) was hydrogenated (10% Pd/C, 0.3 g, 39 psi) in methanolic sodium methoxide (0.02 *M*, 33 mL) at room temperature for 22 h, filtered and neutralised with Duolite C—26 ($H^+$) resin. The pH of the solution was adjusted to 7.5 by addition of solid sodium hydrogencarbonate and the mixture was filtered and concentrated (<1 torr). Methanol (25 mL) and hydrazine hydrate (0.8 g, 16 mmol) were added and the mixture was refluxed for 3 h. T.l.c. ($SiO_2$, chloroform-methanol-water 65:35:10, lower phase) showed complete conversion. The solvent was removed and the residue was acetylated (acetic anhydride-pyridine 1:1, 50 mL) at room temperature for 17 h. Co-evaporation with toluene gave a residue that was chromatographed ($SiO_2$-columm 1.5×38 cm, ethyl acetate-isooctane 3:1 and finally ethyl acetate)

to give crystalline 12 (151 mg, 55%). Recrystallisation from isopropanol-isooctane gave an analytical sample with m.p. 97—100°, $[\alpha]_D^{22}$ +40° (*c* 0.6, chloroform). N.m.r. data: $^1H$ ($CDCl_3$, $Me_4Si$) δ 7.42—7.20 (10H), 5.93 (d, 1H, *J*8Hz, NH), 5.55 (dd, 1H, *J*3 and 1Hz, H—4''), 5.32 (dd, 1H, *J*11 and 3Hz, H—3''), 5.19 (dd, 1H, *J*11 and 3.5Hz, H—2''), 5.14 (dd, 1H, *J*11 and 8Hz, H—2'), 4.99 (d, 1H, *J*3.5Hz, H—1''), 4.88—4.63 (5H), 4.58—4.44 (3H, *inter alia* H—1 or H—1' and Ph*CH*), 4.54 (d, 1H, *J*8Hz, H—1 or H—1'), 4.47 (d, 1H, *J*12Hz, PhCH), 4.31 (dd, 1H, *J*11 and 6.5Hz), 4.23—3.44, 2.13—1.92 (24H, MeCO), 1.18 (t, 3H, *J*7Hz, $CH_3CH_2$). $^{13}C$ ($CDCl_3$, $Me_4Si$) δ 170.5 (2C), 170.4, 170.3, 170.2, 170.1, 169.6, 169.5, 138.8, 138.0, 128.4—127.3, 100.0 (d, *J*165Hz, C—1 or C—1'), 99.7 (d, *J*163Hz, C—1 or C—1'), 99.3 (d, *J*172Hz, C—1''), 77.2, 76.8, 75.9, 74.3, 73.5 ($CH_2$), 73-1 ($CH_2$), 72.4, 71.9, 69.0, 68.8 ($CH_2$), 68.4, 67.7, 67.2, 67.0, 64.2 ($CH_2$), 61.4 ($CH_2$), 60.4 ($CH_2$), 53.1 (C—2), 23.3 ($CH_3CONH$), 20.9, 20.72, 20.69, 20.65 (2C), 20.6, 20.5, 15.0 ($CH_3CH_2$).

Anal. Calc. for $C_{50}H_{65}NO_{23}$: C, 57.30; H, 6.25. Found: C, 57.12; H 6.27.

Ethyl 2-acetamido-2-deoxy-4-*O*-[2,3,6-tri-*O*-acetyl-4-*O*-(2,3,4,6-tetra-*O*-acetyl-α-*D*-galactopyranosyl]-β-*D*-glucopyranoside (13).

Compound 12 (475 mg, 0.45 mmol) was hydrogenated (10% Pd/C, 200 mg, 1 atm.) in acetic acid (10 mL) for 5.5 h at room temperature. The mixture was filtered and the solvent was removed, which gave a crystalline residue. Recrystallisation from ethanol gave 13 (291 mg, 74%) with m.p. 260—262°, $[\alpha]_D^{22}$ +67° (*c* 0.9, chloroform). N.m.r. data: $^1H$ ($CDCl_3+D_2O$, $Me_4Si$) δ 5.59 (dd, 1H, *J*3 and 1Hz, H—4''), 5.37 (dd, 1H, *J*11 and 3Hz, H—3''), 5.23 (dd, 1H, *J*11 and 8Hz, H—2'), 5.21 (dd, 1H, *J*11 and 3.5Hz, H—2''), 4.96 (d, 1H, *J*3.5Hz, H—1''), 4.80 (dd, 1H, *J*11 and 3Hz, H—3'), 4.75 (d, 1H, *J*8.5Hz, H—1), 4.68 (d, 1H, *J*8Hz, H—1'), 4.51 (bt, 1H, *J*7Hz, H—5''), 4.44 (dd, 1H, *J*11 and 8.5Hz), 4.28—3.40 (13H), 2.142, 2.139, 2.10, 2.084, 2.080, 2.05, 2.01, 2.00 (8s, each 3H), 1.21 (t, 3H, *J*7Hz, $CH_3CH_2$). $^{13}C$ ($CDCl_3$, $Me_4Si$) δ 170.6, 170.5 (2C), 170.4, 170.3, 170.1, 169.9, 169.1, 101.8 (C—1 or C—1'), 100.2 (C—1 or C—1'), 99.8 (C—1''), 81.8, 77.3, 73.9, 72.7, 72.6, 71.9, 68.5, 68.4, 67.8, 67.4, 67.3, 65.3, 62.5, 61.0, 60.4, 56.6 (C—2), 23.6—20.5, 15.1.

Anal. Calc. for $C_{36}H_{53}NO_{23}$: C, 49.82; H, 6.16. Found: C, 50.0; H, 6.20.

Ethyl-2-acetamido-2-deoxy-3,6-di-*O*-acetyl-4-*O*-[2,3,6-tri-*O*-acetyl-4-*O*-(2,3,4,6-tetra-*O*-acetyl-α-*D*-galactopyranosyl)-β-*D*-galactopyranosyl]-β-*D*-glucopyranoside (14).

Compound 5 (0.65 g, 0.58 mmol) was hydrogenated (10% Pd/C, 0.31 g, 0.276 MPa) in methanolic sodium methoxide (0.02M, 56 mL) at room temperature for 20 h, filtered and neutralised with Duolite C—26 ($H^+$) resin. The mixture was filtered and concentrated and the residue was dissolved in methanol (20 mL) and hydrazine hydrate (1.7 g, 34 mmol), refluxed for 23 h and concentrated. The residue was acetylated (acetic anhydride-pyridine 1:1, 50 mL) at room temperature for 18 h. Co-concentration with toluene gave a residue that was dissolved in dichloromethane (75 mL) and washed with water (50 ml). The water phase was extracted with dichloromethane (25 mL) and the combined organic extracts were washed with water (10 mL), dried ($Na_2SO_4$), and concentrated. The residue was chromatographed ($SiO_2$-column, 1.5×46 cm, ethyl acetate) to give crystalline 14 (158 mg, 28%) with m.p. 130—133°, $[\alpha]_D^{22}$ +41° (*c* 0.7. chloroform). N.m.r. data: $^1H$ ($CDCl_3$, $Me_4Si$) δ 5.73 (d, 1H, *J*9.5Hz, NH), 5.58 (bd, 1H, *J*~3Hz, H—4''), 5.39 (dd, 1H, *J*11 and 3Hz, H—3''), 5.19 (dd, 1H, *J*11 and 3.5Hz, H—2''), 5.12 (dd, 1H, *J*11 and 8Hz, H—2'), 5.10 (dd, 1H, *J*9.5 and 8Hz, H—3), 5.00 (d, 1H, *J*3.5Hz, H—1''), 4.76 (dd, 1H, *J*11 and 2.5Hz, H—3'), 4.58—4.37 (5H, *inter alia* H—1, H—1'), 4.55 (d, 1H, *J*8Hz, H—1 or H—1'), 4.46 (d, 1H, *J*7.5Hz, H—1 or H—1'), 423—3.96 (6H), 3.95—3.73 (3H), 3.71—3.45 (2H), 2.14, 2.11, 2.106, 2.084, 2.079, 2.076, 2.06, 2.05, 1.99, 1.97, 1.19 (t, 3H, *J*7Hz, $CH_3CH_2$). $^{13}C$ ($CDCl_3$, $Me_4Si$) δ 170.6 (2C), 170.51, 170.46 (2C), 170.4, 170.3, 170.1, 169.7, 169.1, 101.1 (d, *J*164Hz, C—1 or C—1'), 100.7 (d, *J*160Hz, C—1 or C—1'), 99.6 (d, *J*174Hz, C—1''), 77.0, 76.1, 72.8, 72.7, 72.5, 71.9, 69.0, 68.7, 67.9, 67.1 (2C), 65.0 ($CH_2$), 62.5 ($CH_2$), 61.4 ($CH_2$), 60.3 ($CH_2$), 53.4 (C—2), 23.2—20.5, 15.1 ($CH_3CH_2$).

Anal. Calc. for $C_{40}H_{57}NO_{25}$: C, 50.47; H, 6.04. Found: C, 50.31; H, 6.02.

Ethyl 2-acetamido-2-deoxy-4-*O*-[4-*O*-(α-*D*-galactopyranosyl)-β-*D*-galactopyranosyl]-β-*D*-glucopyranoside (15).

*a:* Compound 14 (58 mg, 0.06 mmol) was O-deacetylated in methanolic sodium methoxide (0.036 *M*, 5.5 mL) at room temperature for 21 h. The mixture was neutralised with Duolite® C-26 ($H^+$) resin, filtered and concentrated. The residue was dissolved in water and lyophilised to give 15 (21 mg, 60%) with $[\alpha]_D^{22}$ +25° (*c* 0.7, water). N.m.r. data: $^1H$ [$(CD_3)_2SO+D_2O$, 50°, $Me_4Si$] δ *inter alia* 4.80 (d, 1H, *J*3.5Hz, H-1''), 4.37 (d, 1H, *J*8Hz, H—1 or H—1'), 4.29 (d, 1H, *J*7.5Hz, H—1 or H—1'), 4.07 (bt, 1H, *J*~6.5Hz), 1.81 (s, 3H, MeCON), 1.08 (t, 3H, *J*7Hz, $CH_3CH_2$).$^{13}C$ ($D_2O$, TSP), δ 177.4, 106.1, 103.4, 103.1, 81.6, 80.1, 78.2, 77.7, 75.4, 75.0, 73.7, 73.6, 72.0, 71.7, 71.4, 69.1 ($CH_2$), 63.3 ($CH_2$), 63.2 ($CH_2$), 62.9 ($CH_2$), 58.1, 25.0, 17.1. *b*: Compound 13 (15 mg, 0.017 mmol) was O-deacetylated and worked-up as above to give 15 (8.0 mg, 81%).

*Neoglycoproteins* 16 and 17. — Compound 9 (40 mg) was coupled to bovine serum albumin (BSA, 60 mg), to give 16. The degree of binding was 17. Another portion of 9 (120 mg) was coupled to key-hole limpet haemocyanin (KLH, 35 mg) using the corresponding method. The degree of binding was 420.

REFERENCES

1. A. F. Bochkov and G. E. Zaikov, "Chemistry of the O-Glycosidic Bond: Formation and Cleavage", Pergamon Press, 1979.

2. a Review: C. P. Stowell and Y. C. Lee, *Adv. Carboh. Chem. Biochem., 37,* 225 (1980).
   b Review: J. D. Alphin and J. C. Wriston, Jr., *CRC Critical Rev. Biochem.,* May 1981, pp 259—306.
3. a R. T. Lee and Y. C. Lee, *Carbohydr. Res., 37,* 193 (1974).
   b J. C. Jacquinet and H. Paulsen, *Tetrahedron Lett., 22,* 1387 (1981).
   c M. A. Bernstein and L. D. Hall, *Carbohydr. Res., 78,* Cl (1980).
4. N. Sharon and H. Lis, *Science, 177,* 949 (1972).
5. F. Marquez and A. Mesquida, *Anales de Quimica, 70,* 833 (1974).
6. G. Magnusson, G. Noori, J. Dahmén, T. Frejd and T. Lave, *Acta Chem. Scand., B 35,* 213 (1981).
7. B. Lindberg, *Acta Chem. Scand., 2,* 426—429 (1948).
8. R. J. Ferrier and R. H. Furneaux, *Methods Carbohydr. Chem., 8,* 251 (1980).
9. See ref. 1, p. 20.
10. N. Morishima, S. Koto, C. Kusuhara and S. Zen, *Chem., Lett.,* 427 (1981).
11. R. U. Lemieux, K. B. Hendriks, R. V. Stick and K. James, *J. Am. Chem. Soc., 97,* 4056 (1975).
12. Varian XL-200. See G. A. Morris and R. Freeman, *J. Am. Chem. Soc. 101,* 760 (1979).
13. P. A. S. Smith in "Molecular Rearrangements" part 1, p. 577, P. de Mayo, Ed., Interscience, New York, 1963.
14. R. U. Lemieux, D. R. Bundle and D. A. Baker, *J. Am. Chem. Soc., 97,* 4076 (1975).
15. J. K. Inman, B. Merchant, L. Claflin and S. E. Tacey, *Immunochemistry, 10,* 165 (1973).
16. M. Dubois, K. A. Gilles, J. K. Hamilton, P. A. Rebers and F. Smith, *Analyt. Chem., 28,* 350 (1956).
17. a C. R. McBroom, C. H. Samanen and I. J. Goldstein, *Methods Enzymol., 28,* 212 (1972).
    b D. F. Smith, D. A. Zopf and V. Ginsburg, *ibid., 50,* 169 (1978).
18. J. E. Hodge and B. T. Hofreiter, *Methods Carboh. Chem., I,* 389 (1962).
19. A. Shöberl and A. Wagner in Houben-Weyl "Methoden der Organischen Chemie", Georg Thieme Verlag, Stuttgart 1955, Vol. 9, pp. 55.
20. E. Fisher and F. Armstrong, *Ber., 35,* 3155 (1902).
21. R. U. Lemieux and R. M. Ratcliffe, *Can. J. Chem., 57,* 1244 (1979).
22. R. U. Lemieux, *Chem. Soc. Rev.,* 423 (1978).
23. A. R. Pray, *Inorg. Synth., 5,* 154 (1957).
24. G. Källenius, R. Möllby, S. B. Svensson, J. Winberg, A. Lundblad, S. Svensson and B. Cedergren, *FEMS Lett.,* 7 (1980) 297—302.
25. R. R. Race and R. Sanger, *Blood Groups in Man,* 6th edn., Blackwell, Oxford, 1975; M. Naiki and M. Kato, *Vox Sang.,* 37 (1979) 30—38.
26. H. Leffler and C. Svanborg-Edén, *FEMS Lett.,* 8 (1980) 127—134.
27. J. Dahmén, T. Frejd, T. Lave, F. Lindh, G. Magnusson, G. Noori, and K. Pålsson, *Carbohydr. Res.,* 113 (1983) 219—223.
28. D. D. Cox, E. K. Metzner, and E. J. Reist, *Carbohydr. Res.,* 63 (1978) 139—147; P. J. Garegg and H. Hultberg, ibid., 110 (1982) 261—266.
29. J. Dahmén, T. Frejd, G. Grönberg, T. Lave, G. Magnusson, and G. Noori, *Carbohydr. Res.,* (1983) UK 2538.
30. D. M. Hall, *Carbohydr. Res.,* 86 (1980) 158—160.
31. P. Garegg and H. Hultberg, *Carbohydr. Res.,* 93 (1981) C10—C11.
32. B. R. Baker, M. V. Querry, S. Bernstein, S. R. Safir, and Y. Subbarow, *J. Org. Chem.,* 12 (1947) 167—173.
33. J. K. Inman, B. Merchant, L. Claflin, and S. E. Tacey, *Immunochemistry,* 10 (1973) 165—174.
34. R. U. Lemieux, D. R. Bundle, and D. A. Baker, *J. Am. Chem. Soc.,* 97 (1975) 4076—4083.
35. J. Dahmén, T. Frejd, G. Magnusson, and G. Noori, *Carbohydr. Res.,* 111 (1982) C1—C4;
36. J. S. Sawardeker, J. H. Sloneker, and A. Jeanes, *Anal. Chem.,* 37 (1965) 1602—1604.
37. J. Björndal, C. G. Hellerqvist, B. Lindberg, and S. Svensson, *Angew. Chem. Int. Ed. Engl.,* 9 (1970) 610—619.
38. J. Dahmén, T. Frejd, G. Grönberg, T. Lave, G. Magnusson, and G. Noori, *Carbohydr. Res.,* (1983) UK 2562.
39. R. U. Lemieux and J. D. Stevens, *Can. J. Chem.,* 43 (1965) 2059—2070.
40. P. Hallgren, G. Hansson, K. G. Henriksson, A. Häger, A. Lundblad and S. Svensson, *Europ. J. Clin. Invest.,* 4 (1974) 429—433.
41. G. Lennartson, A. Lundblad, J. Lundsten, S. Svensson and A. Häger, Eur. J. Biochem., 83 (1978) 325—334.
42. A. Lundblad, S. Svensson, I. Yamashina and M. Ohta, *FEBS Lett.,* 97, (1979) 249—252.
43. P. Hallgren, B. S. Lindberg, A. Lundblad, *J. Biol. Chem.,* 252 (1977) 1034—1040.
44. B. V. McCleary, *Carbohydr. Res.,* 85 (1980) 160—163.
45. B. J. Catley, *J. General Microbiol.,* 78 (1973) 33—38.
46. G. Magnusson, G. Noori, J. Dahmén, T. Frejd and T. Lave, *Acta Chem. Scand.,* B 35 (1981) 213—216; J. Dahmén, T. Frejd, G. Magnusson and G. Noori, *Carbohydr. Res.,* 111 (1982) C1—C4; J. Dahmén, T. Frejd. G. Magnusson and G. Noori, *Carbohydr. Res.,* 114 (1983) 328—330; J. Dahmén, T. Frejd, G. Grönberg, T. Lave, G. Magnusson and G. Noori, *Carbohydr. Res.,* (UK 2538).
47. See G. A. Morris and R. Freeman, *J. Am. Chem. Soc.,* 101 (1979) 760—763.

48. R. R. Race and R. Sanger, *Blood Groups in Man,* 6th edn., Blackwell, Oxford, 1975; M. Naiki and M. Kato, *Vox Sang.,* 37 (1979) 30—38.
49. (a) G. O. Aspinall and R. S. Fanshawe, *J. Chem. Soc.,* (1961) 4215—4225; (b) M. E. Chacon-Fuertes and M. Martin-Lomas, *Carbohydr. Res.,* 43 (1975) 51—56; (c) P. A. Gent, R. Gigg, and A. A. E. Penglis, *J. Chem. Soc., Perkin Trans. 1,* (1976) 1395—1404; (d) D. D. Cox, E. K. Metzner, and E. J. Reist, *Carbohydr. Res.,* 62 (1978) 245—252; (e) P. J. Garegg and H. Hultberg, *ibid.,* 110 (1982) 261—262; (f) M—L. Milat, P. A. Zollo, and P. Sinäy, *ibid.* 100 (1982) 263—271; (g) J. Dahmén, T. Frejd, T. Lave, F. Lindh, G. Magnusson, G. Noori, and K. Pålsson, *ibid.* 113 (1983) 219—224.
50. (a) D. D. Cox, E. K. Metzner, and E. J. Reist, *Carbohydr. Res.,* 63 (1978) 139—147; (b) P. J. Garegg and H. Hultberg, *ibid.* 110 (1982) 261—266;
51. M. A. Nashed and L. Anderson, *Carbohydr. Res.,* 114 (1983) 43—52.
52. H. Paulsen and A. Bünsch, *Carbohydr. Res.,* 101 (1982), 21—30.
53. (a) J. Dahmén, T. Frejd, G. Magnusson, and G. Noori, *Carbohydr. Res.,* 111 (1982) C1—C4; (b) J. Dahmén, T. Frejd, G. Grönberg, T. Lave, G. Magnusson, and G. Noori, *ibid.,* (UK 2538); (c) *idem., ibid.,* (UK 2562).
54. B. R. Baker, M. V. Querry, S. Bernstein, S. R. Safir, and Y. Subbarow, *J. Org. Chem.,* 12 (1947) 167—173.
55. "Protective Groups in Organic Chemistry", J. F. W. McOmie, Ed. Plenum Press, London, 1973.
56. A. H. Haines, *Adv. Carbohydr. Chem. Biochem., 39,* 13 (1981).

**Claims**

1. An O-glycoside having the general formula:

$$(\text{sugar})_n\text{-O—}(CH_2)_m\text{—S—R—R}'$$

wherein n is an integer of from 1—10, inclusive, m is 2, R is selected from the group consisting of alkylene having at most 25 carbon atoms and arylene and R′ is selected from the group consisting of H, CHO, $CH(OR'')_2$, $NO_2$, $NH_2$, OH, SH, COOH, $COOCH_3$, $COOCH_2CH_3$, $CONHNH_2$ and $CON_3$, wherein R″ is $C_{1-4}$-alkyl.

2. Glycoside according to claim 1, wherein the sugar is an aldose selected from the group consisting of D-glucose, D-galactose, D-mannose, D-xylose, L-fucose, 2-acetamido-2-deoxy-D-glucose, 2-deoxy-2-phtalimido-glucose, 2-acetamido-2-deoxy-D-galactose, 2-azido-2-deoxy-D-glucose, 2-azido-2-deoxy-D-galactose, D-glucuronic acid and D-galacturonic acid, 2-deoxy-2-phtalimido glucose and 2-deoxy-2-phtalimido galactose.

3. An O-glycoside according to claim 1 including a spacer arm, wherein R is arylene and R′ is amino or nitro.

4. An O-glycoside according to claim 1 or 2, wherein R′ is an ester of a carboxylic acid.

5. An O-glycoside according to claim 1, wherein the sugar entity contains galactose and 2-acetamido-2-deoxy-D-galactose and n is from 2 to 5.

6. A process for preparing an O-glycoside having the general formula:

$$(\text{sugar})_n\text{-O-}(CH_2)_m\text{—S—R—R}'$$

wherein n is an integer of from 1—10, inclusive, m is an integer of from 2—20, inclusive, R is selected from the group consisting of alkylene having at most 25 carbon atoms and arylene and R′ is selected from the group consisting of H, CHO, $CH(OR'')_2$, $NO_2$, $NH_2$, OH, SH, COOH, $COOCH_3$, $COOCH_2CH_3$, $CONHNH_2$ and $CON_3$, wherein R″ is $C_{1-4}$-alkyl, comprising: reacting an Halo alkyl O-glycoside having the general formula:

$$(\text{sugar})_n\text{-O—}(CH_2)_m\text{-Hal}$$

wherein Hal is chlorine, bromine or iodine, m is an integer of 2—20 inclusive and n is an integer of 1 to 10, inclusive, with a thiol of the general formula:

$$\text{HS—R—R}'$$

wherein R and R′ have the above meaning.

7. A glycoconjugate having the general formula:

$$(\text{sugar})_n\text{-O—}(CH_2)_m\text{—S—R—R}''$$

wherein n is an integer of from 1—10, inclusive, m is 2, R is selected from the group consisting of alkylene having at most 25 carbon atoms and arylene and R″ constitutes a carrier.

8. A glycoconjugate according to claim 7 having the formula:

D-Galα1→4-D-Galβ$OCH_2CH_2$—S—$CH_2CH_2$CONH—R‴, and
L-Fucα1→2-D-Galβ$OCH_2CH_2SCH_2CH_2$CONH—R‴,

wherein R''' is selected from the group consisting of residues of proteins, polysaccharides, plastic materials and inorganic materials.

9. A bi-dentate O-glycoside having the general formula:

$$[(sugar)_n\text{-}O—(CH_2)_m—S]_2R$$

wherein m, n and R have the meaning given in claim 7.

## Patentansprüche

1. O-Glycosid der allgemeinen Formel

$$(Zucker)_n—O—(CH_2)_m—S—R—R'$$

worin n eine ganze Zahl von 1 bis 10 einschließlich ist, m 2 ist, R aus der Gruppe Alkylen mit höchstens 25 Kohlenstoffatomen und Arylen ausgewählt ist und R' aus der Gruppe H, CHO, $CH(OR'')_2$, $NO_2$, $NH_2$, OH, SH, COOH, $COOCH_3$, $COOCH_2CH_3$, $CONHNH_2$ und $CON_3$ ausgewählt ist, worin R'' $C_{1-4}$-Alkyl ist.

2. Glycosid nach Anspruch 1, worin der Zucker eine Aldose aus der Gruppe D-Glucose, D-Galactose, D-Mannose, D-Xylose, L-Fucose, 2-Acetamido-2-deoxy D-glucose, 2-Deoxy-2-phthalimidoglucose, 2-Acetamido-2-deoxy-D-galactose, 2-Azido-2-deoxy-D-glucose, 2-Azido-2-deoxy-D-galactose, D-Glucuronsäure und D-Galacturonsäure, 2-Deoxy-2-phthalimidoglucose und 2-Deoxy-2-phthalimidogalactose ist.

3. O-Glycosid nach Anspruch 1 mit einem Abstandshalterarm, worin R Arylen und R' Amino oder Nitro ist.

4. O-Glycosid nach Anspruch 1 oder 2, worin R' ein Ester einer Carbonsäure ist.

5. O-Glycosid nach Anspruch 1, worin der Zuckerrest Galactose und 2-Acetamido-2-deoxy-D-galactose enthält und n 2 bis 5 ist.

6. Verfahren zur Herstellung eines O-Glycosids der allgemeinen Formel

$$(Zucker)_n—O—(CH_2)_m—S—R—R'$$

worin n eine ganze Zahl von 1 bis 10 einschließlich ist, m 2 ist, R aus der Gruppe Alkylen mit höchstens 25 Kohlenstoffatomen und Arylen ausgewählt ist und R' aus der Gruppe H, CHO, $CH(OR'')_2$, $NO_2$, $NH_2$, OH, SH, COOH, $COOCH_3$, $COOCH_2CH_3$, $CONHNH_2$ und $CON_3$ ausgewählt ist, worin R'' $C_{1-4}$-Alkyl ist, bei dem man ein Halogenalkyl-O-glycosid der allgemeinen Formel

$$(Zucker)_n\text{-}O—(CH_2)_m\text{-}Hal$$

worin Hal Chlor, Brom oder Jod bedeutet, m eine ganze Zahl von 2 bis 20 einschließlich ist und n eine ganze Zahl von 1 bis 10 einschließlich ist, mit einem Thiol der allgemeinen Formel

$$HS—R—R'$$

worin R und R' die obige Bedeutung haben, umsetzt.

7. Glycokonjugat der allgemeinen Formel

$$(Zucker)_n\text{-}O—(CH_2)—S—R—R'',$$

worin n eine ganze Zahl von 1 bis 10 einschließlich ist, m 2 ist, R aus der Gruppe Alkylen mit höchstens 25 Kohlenstoffatomen und Arylen ausgewählt ist und R'' einen Träger darstellt.

8. Glycokonjugat nach Anspruch 7 der Formel

$$D\text{-}Gal\alpha 1\rightarrow 4\text{-}D\text{-}Gal\beta OCH_2CH_2—S—CH_2CH_2CONH—R'''$$

und

$$L\text{-}Fuc\alpha 1\rightarrow 2\text{-}D\text{-}Gal\beta OCH_2CH_2SCH_2CH_2CONH—R'''$$

worin R''' aus der Gruppe der Reste von Proteinen, Polysacchariden, Kunststoffmaterialien und anorganischen Materialien ausgewählt ist.

9. Bidentat-O-glycosid der allgemeinen Formel

$$[(Zucker)_n\text{-}O—(CH_2)_m—S]_2R,$$

worin m, n und R die in Anspruch 7 angegebene Bedeutung haben.

**Revendications**

1. O-glycoside de formule générale:

$$(sucre)_n\text{-}O—(CH_2)_m—S—R—R'$$

dans laquelle n est un nombre entier de 1 à 10, inclus, m est égal à 2, R est choisi dans le groupe constitué par un alkylène comportant au plus 25 atomes de carbone et un arylène, et R' est choisi dans le groupe constitué par H, CHO, $CH(OR'')_2$, $NO_2$, $NH_2$, OH, SH, COOH, $COOCH_3$, $COOCH_2CH_3$, $CONHNH_2$ et $CON_3$, où R'' est un alkyle en $C_{1-4}$.

2. Glycoside selon la revendication 1, dans lequel le sucre est un aldose choisi dans le groupe constitué par le D-glucose, le D-galactose, le D-mannose, le D-xylose, le L-fucose, le 2-acétamido-2-désoxy-D-glucose, le 2-désoxy-2-phtalimido-glucose, le 2-acétamido-2-désoxy-D-galactose, le 2-azido-2-désoxy-D-glucose, le 2-azido-2-désoxy-D-galactose, l'acide D-glucuronique et l'acide D-galacturonique, le 2-désoxy-2-phtalimidoglucose et le 2-désoxy-2-phtalimidogalactose.

3. O-glycoside selon la revendication 1, contenant un bras écarteur, dans lequel R est un arylène et R' est un amino ou un nitro.

4. O-glycoside selon la revendication 1 ou 2, dans lequel R' est un ester d'un acide carboxylique.

5. O-glycoside selon la revendication 1, dans lequel l'entité sucre contient du galactose et du 2-acétamido-2-désoxy-D-galactose et n a une valeur de 2 à 5.

6. Procédé de préparation d'un O-glycoside de formule générale:

$$(sucre)_n\text{-}O—(CH_2)_m—S—R—R'$$

dans laquelle n est un nombre entier de 1 à 10, inclus, m est un nombre entier de 2 à 20, inclus, R est choisi dans le groupe constitué par un alkylène comportant au plus 25 atomes de carbone et un arylène, et R' est choisi dans le groupe constitué par H, CHO, $CH(OR'')_2$, $NO_2$, $NH_2$, OH, SH, COOH, $COOCH_3$, $COOCH_2CH_3$, $CONHNH_2$ et $CON_3$, où R'' est un alkyle en $C_1—C_4$, comprenant:
la réaction d'un halogénoalkyl-O-glycoside de formule générale:

$$(sucre)_n\text{-}O—(CH_2)_m—Hal$$

dans laquelle Hal est un chlore, un brome ou un iode, m est un nombre entier de 2 à 20, inclus, et n est un nombre entier de 1 à 10, inclus, avec un thiol de formule générale:

$$HS—R—R'$$

dans laquelle R et R' ont la signification ci-dessus.

7. Glycoconjugué de formule générale:

$$(sucre)_n\text{-}O—(CH_2)_m—S—R—R''$$

dans laquelle n est un nombre entier de 1 à 10, inclus, m est égal à 2, R est choisi dans le groupe constitué par un alkylène comportant au plus 25 atomes de carbone et un arylène, et R'' constitue un véhicule.

8. Glycoconjuguée selon la revendication 7, de formule:

$$D\text{-}Gal\alpha1\rightarrow4\text{-}D\text{-}Gal\beta OCH_2CH_2—S—CH_2CH_2CONH—R''', \text{ et}$$

$$L\text{-}Fuc\alpha1\rightarrow2\text{-}D\text{-}Gal\beta OCH_2CH_2SCH_2CH_2CONH—R''',$$

où R''' est choisi dans le groupe constitué par des résidus de protéines, de polysaccharides, de matières plastiques et de matières minérales.

9. O-glycoside bidenté de formule générale:

$$[(sucre)_n\text{-}O—(CH_2)_m—S]_2R$$

dans laquelle m, n et R ont la signification donnée dans la revendication 7.

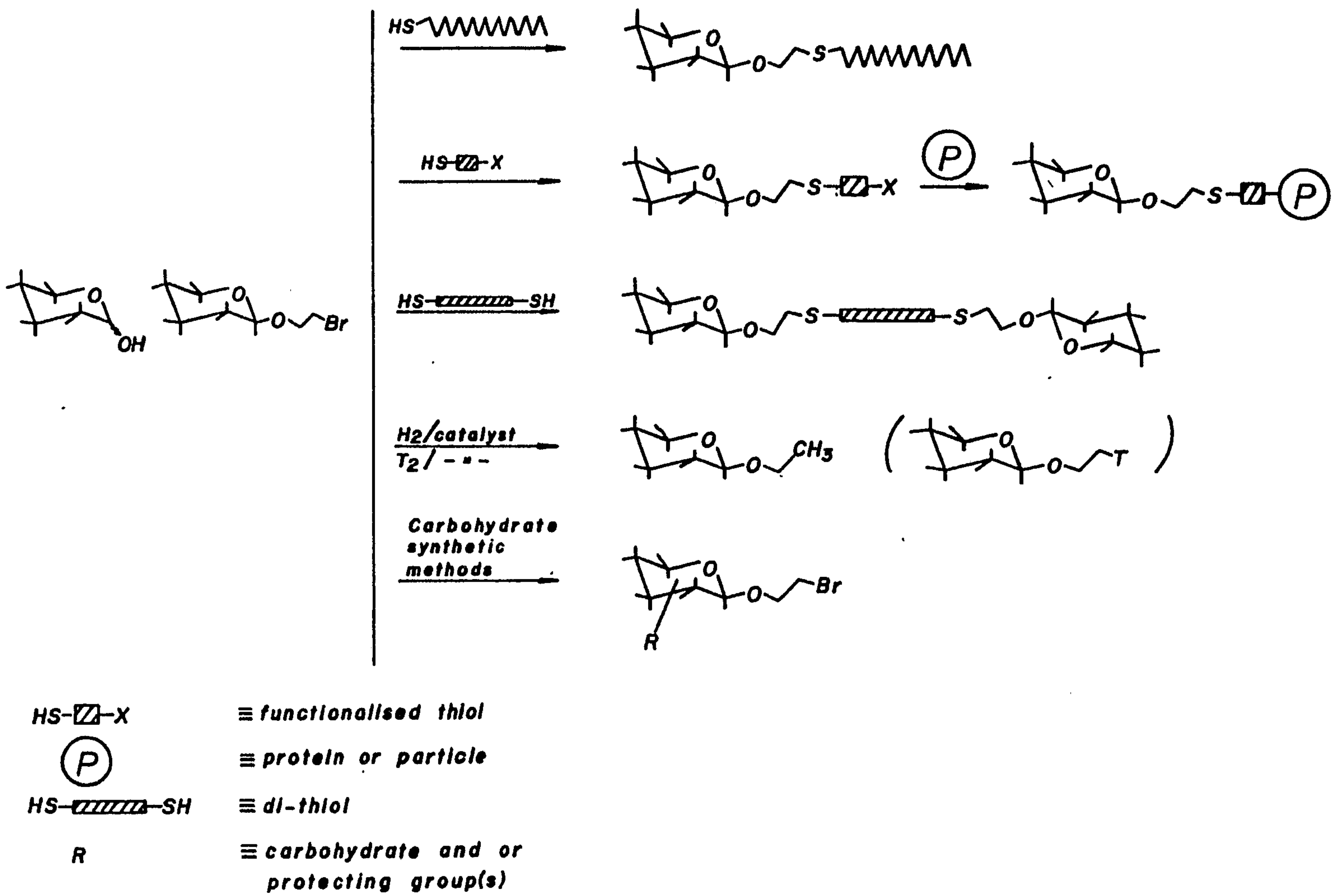


Figure 1. Principal applications of 2-bromoethyl glycosides

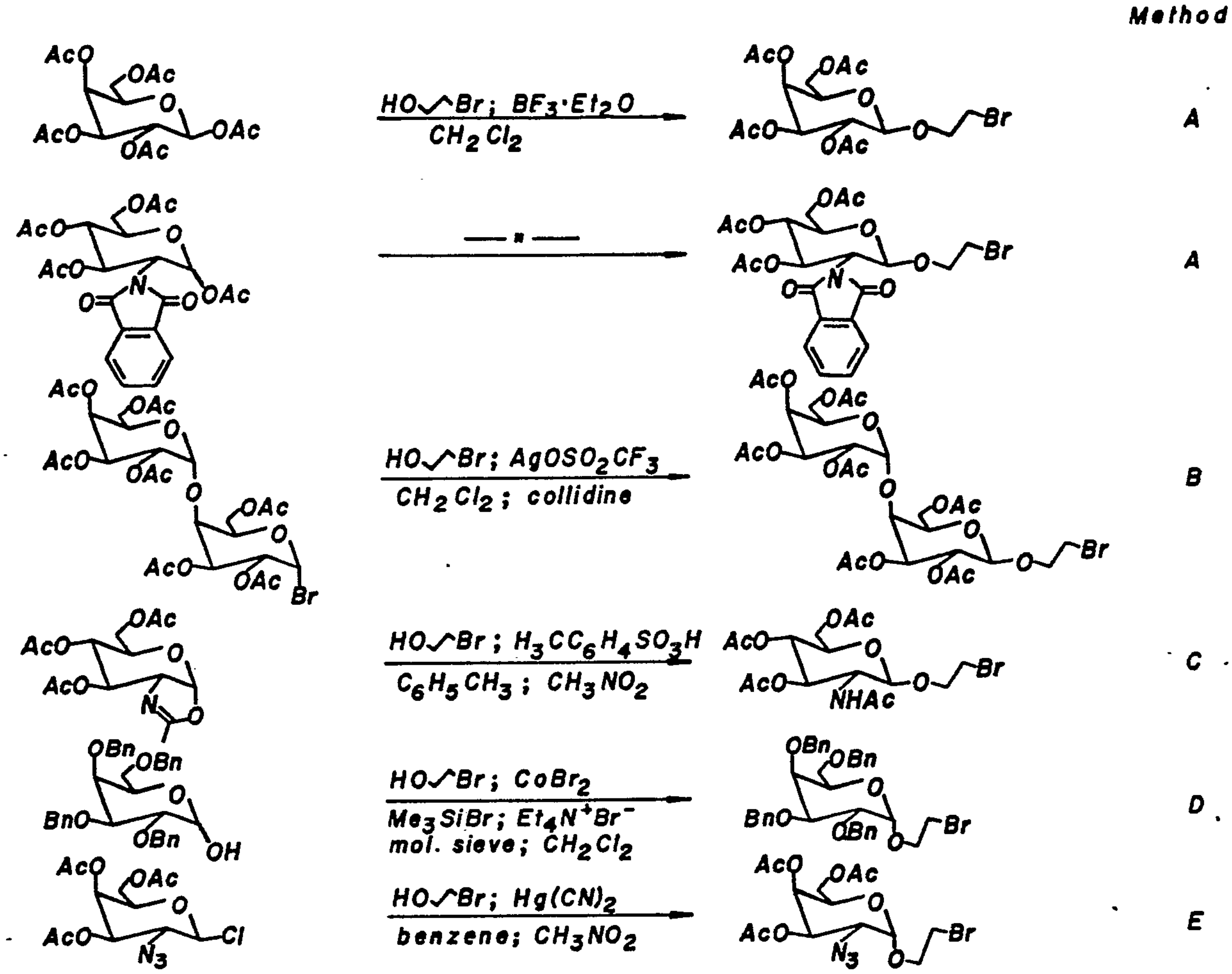


Figure 2, Examples of 2-bromoethyl glycoside synthesis.

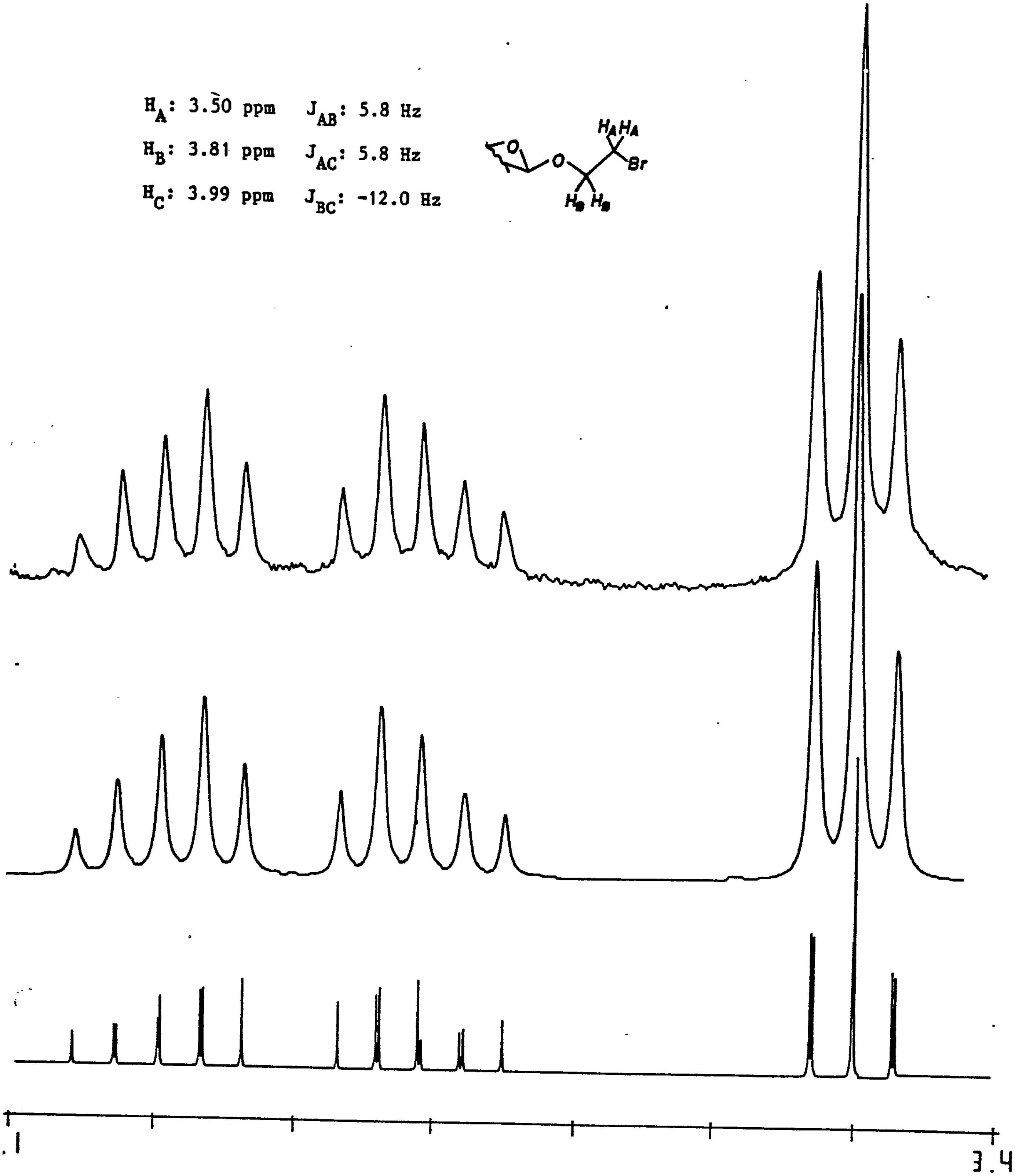


Figure 3. $^1$H-NMR spectrum of 2-bromoethyl 2,3,4-tri-O-acetyl- -L-fucopyranoside (7; 2-bromoethyl part). Top: Experimental spectrum; middle: simulated spectrum[26] with line-width 1.2 Hz; bottom; simulated spectrum with line-width 0.1 Hz.

15

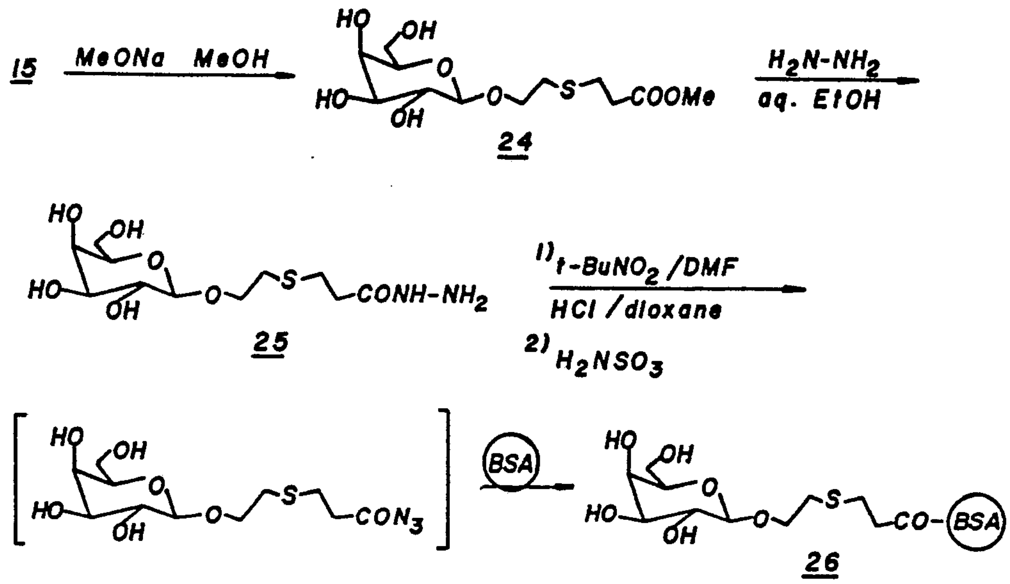


24

25

26

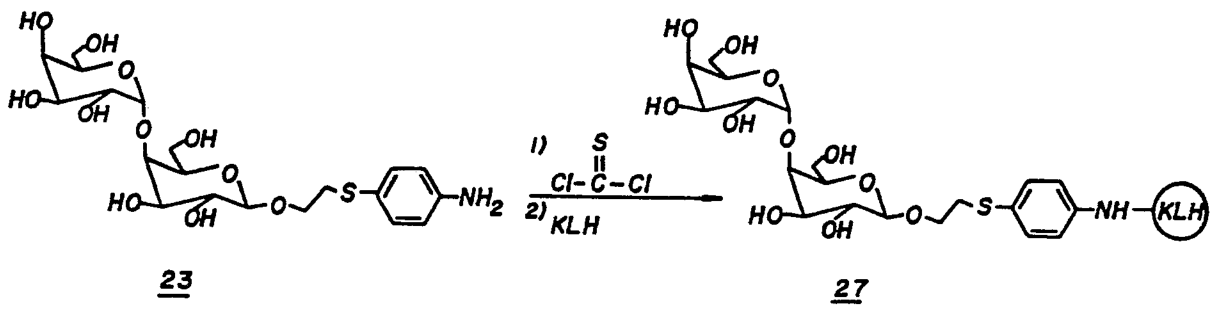


23

27

Figure 4. Model reactions for the coupling of spacer-arm glycosides to proteins. For a further example, see Figure 6.

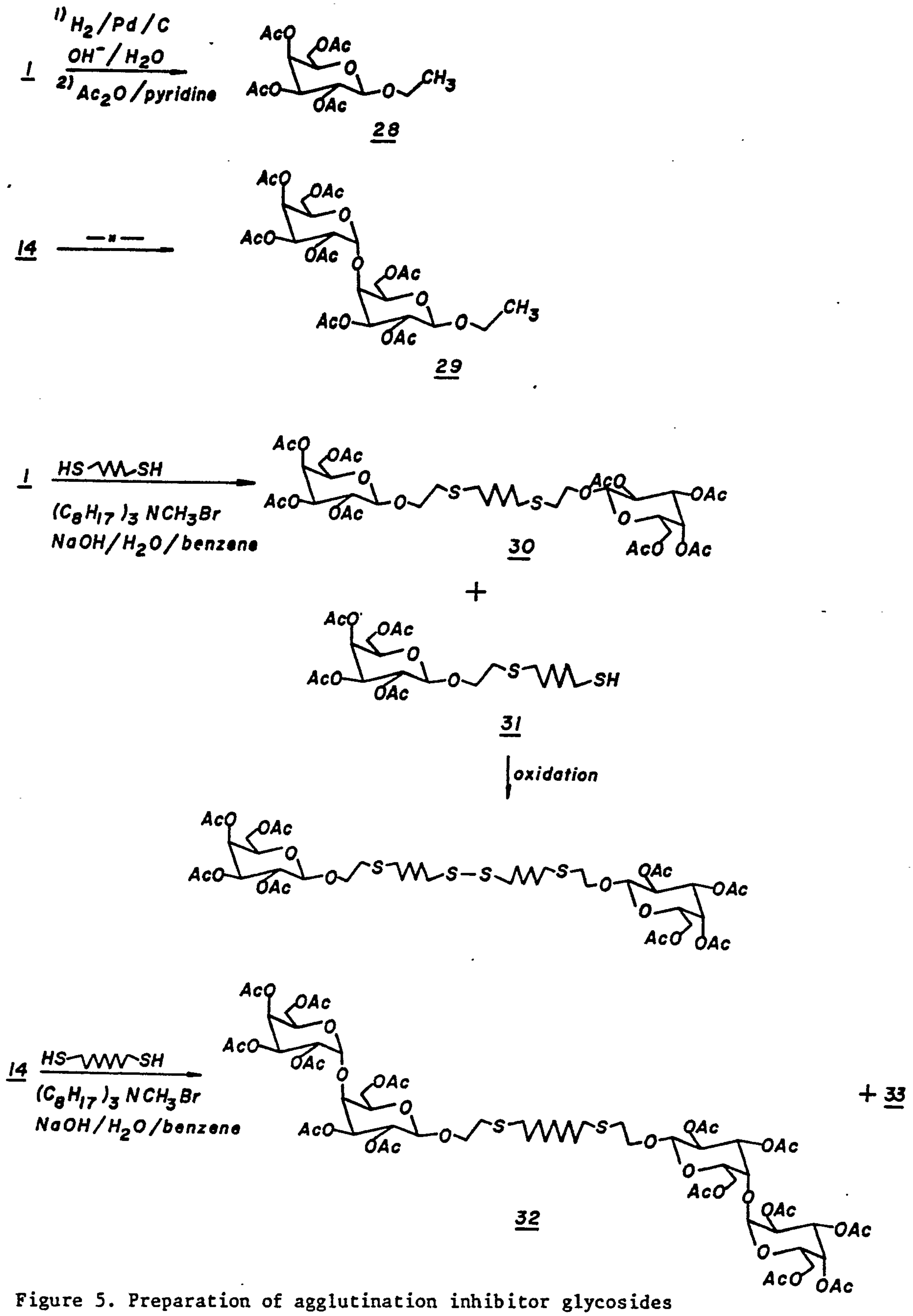


Figure 5. Preparation of agglutination inhibitor glycosides form 2-bromoethyl glycosides.

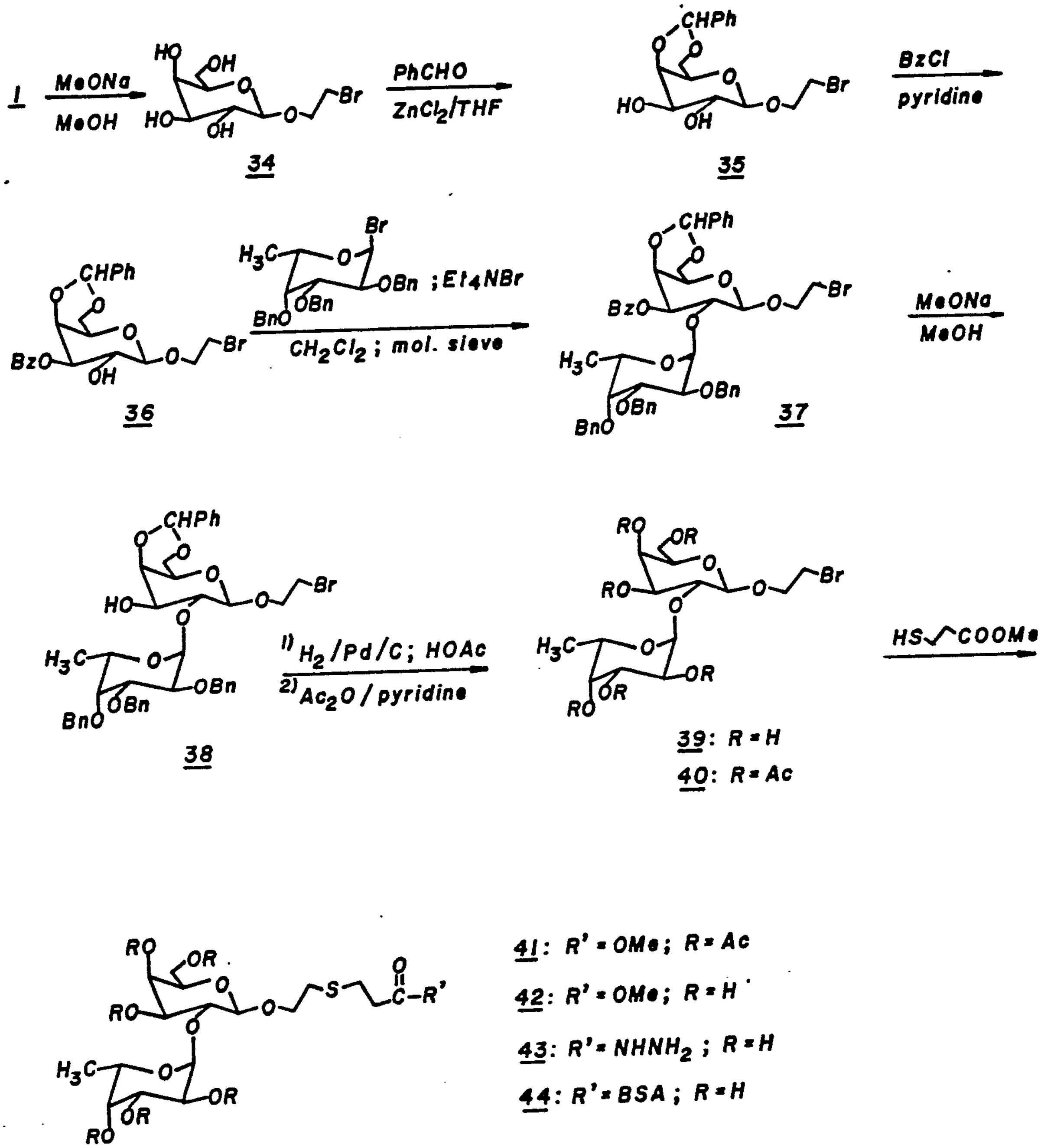


1 34 35 36 37 38

39: R = H
40: R = Ac

41: R' = OMe; R = Ac
42: R' = OMe; R = H
43: R' = $NHNH_2$ ; R = H
44: R' = BSA ; R = H

Figure 6. The 2-bromoethyl group in synthetic carbohydrate chemistry, exemplified by a total synthesis of the blood-group H specific glycoside.

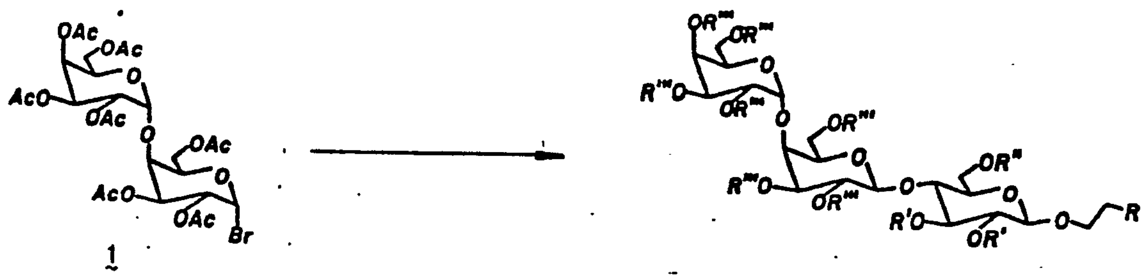

1

9 R=Br, R'=R"=Bn, R"'=Ac
10 R=Br, R'=R"=H, R"'=Ac
11 R=Br, R'=R"=R"'=Ac
12 R=Br, R'=Bz, R"=Bn, R"'=Ac
13 R=Br, R'=Bz, R"=H, R"'=Ac
15 R=S⌒COOMe, R'=R"=R"'=Ac
16 R=S⌒COOMe, R'=R"=R"'=H
17 R=S∧∧∧∧∧∧∧, R'=R"=R"'=Ac
18 R=S∧∧∧∧∧∧∧, R'=R"=R"'=H
19 R=H, R'=R"=R"'=Ac
20 R=H, R'=R"=R"'=H
21 R=S⌒ CONH-BSA, R'=R"=R"'=H
22 R=S⌒ CONH-KLH, R'=R"=R"'=H

+

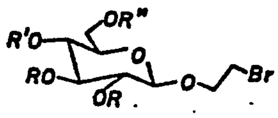

2 R=R'=R"=Ac
3 R=H, R'R"=CHPh
4 R=Bn, R'R"=CHPh
6 R=R"=Bn, R'=H
7 R=Bz, R'R"=CHPh
8 R=Bz, R'=H, R"=Bn

+

+

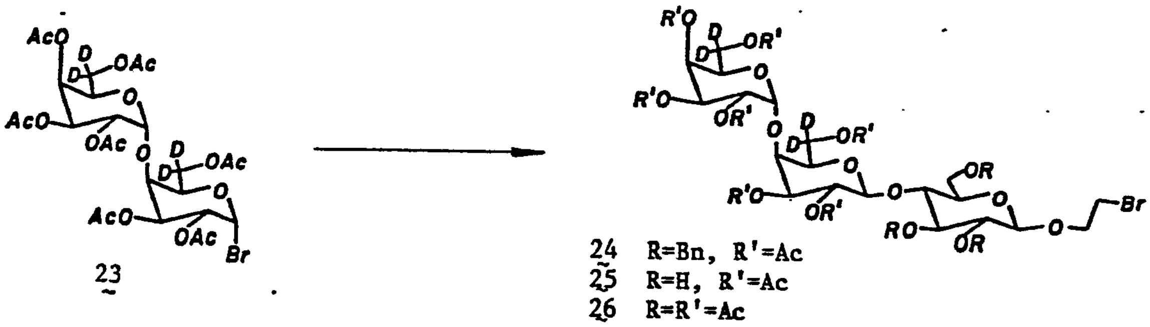

23

24 R=Bn, R'=Ac
25 R=H, R'=Ac
26 R=R'=Ac

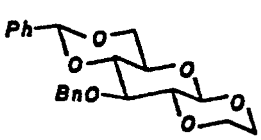

5

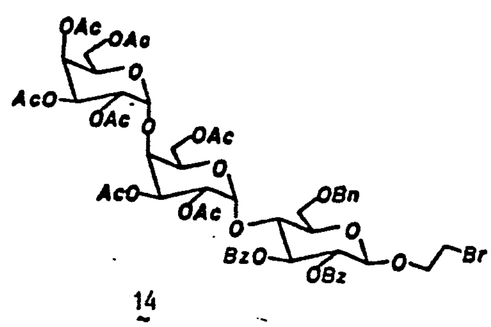

14

Figure 7

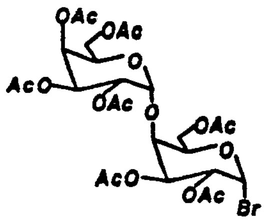

1

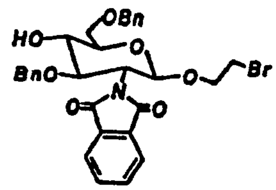

2

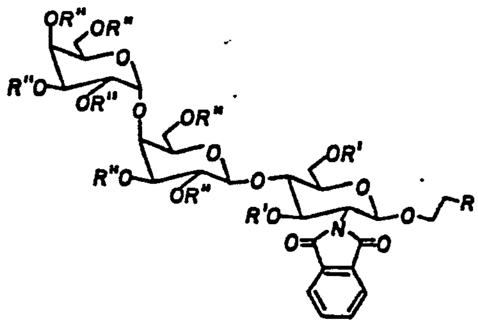

3 R=Br, R'=Bn, R"=Ac
4 R=Br, R'=H, R"=Ac
5 R=Br, R'=R"=Ac
6 R=S COOMe, R'=R"=Ac
7 R=S R'=R"=Ac

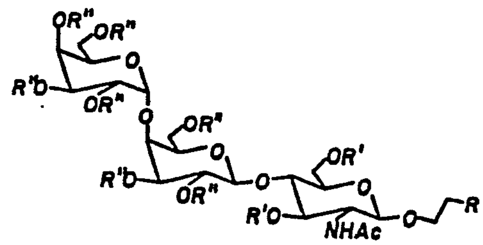

8 R=S COOMe, R'=R"=Ac
9 R=S COOMe, R'=R"=H
10 R=S, R'=R"=Ac
11 R=S, R'=R"=H
12 R=H, R'=Bn, R"=Ac
13 R=R'=H, R"=Ac
14 R=H, R'=R"=Ac
15 R=R'=R"=H
16 R=S CONH-BSA, R'=R"=H
17 R=S CONH-KLH, R'=R"=H

Figure 8